# EUROPEAN PATENT APPLICATION

(11) **EP 4 253 372 A1**
(43) Date of publication of application: **04.10.2023**
(21) Application number: 21910875.0
(22) Date of filing: 22.12.2021
(51) Int. Cl.: C07D 311/86, A61K 31/352, A61K 39/395, A61P 35/00, A61P 35/02, C07D 407/04

(54) **PHARMACEUTICAL COMPOSITION FOR AMELIORATING MALIGNANT DISEASES**

(30) Priority: 22.12.2020 JP 2020212015
(71) Applicant: Kinki University, Higashi-Osaka-shi Osaka 577-8502 (JP)
(72) Inventor: NISHIDA Shozo, Osaka 5778502 (JP); TSUBAKI Masanobu, Osaka 5778502 (JP); TAKEDA Tomoya, Osaka 5778502 (JP); TANABE Genzoh, Osaka 5778502 (JP); TAKASHIMA Katsuki, Osaka 5778502 (JP)
(74) Representative: Grünecker Patent- und Rechtsanwälte PartG mbB
(86) International application number: PCT/JP2021/047614
(87) International publication number: WO 2022/138735

(57) **Abstract**

Although compounds for treating or ameliorating malignant diseases have been proposed, patients had to endure a large burden since many of such compounds could not be orally ingested. Mangiferin has an effect of ameliorating malignant diseases through oral ingestion. However, such effect is minor and realistically it was not easy to ingest mangiferin. Further, in order to develop more effective pharmaceutical agents and the like for treating malignant diseases, it has been considered constantly necessary to obtain new or improved forms of an existing medical agent for inhibiting kinases such as NIK. Compounds represented by formula (1), formula (2), formula (3), formula (4), formula (5), formula (6), formula (7), formula (8), formula (9), formula (10), formula (11), and formula (12) have an effect of ameliorating malignant diseases through oral ingestion, and can exhibit said effect with an amount less than that for mangiferin.

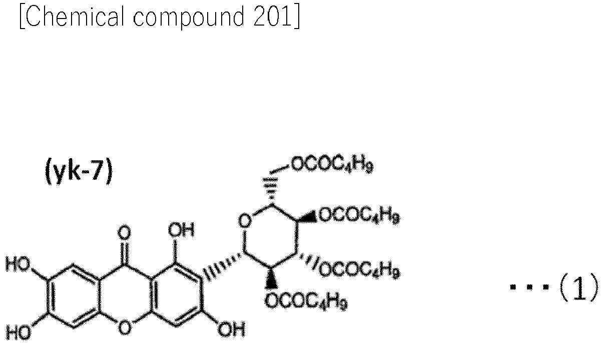

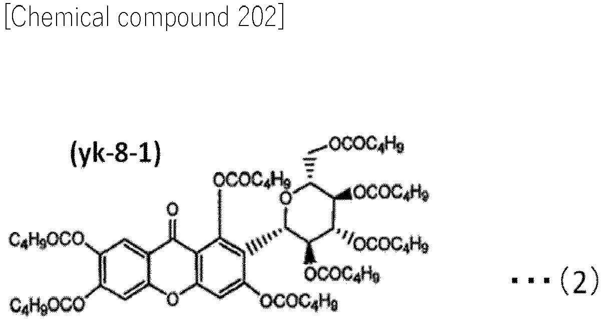

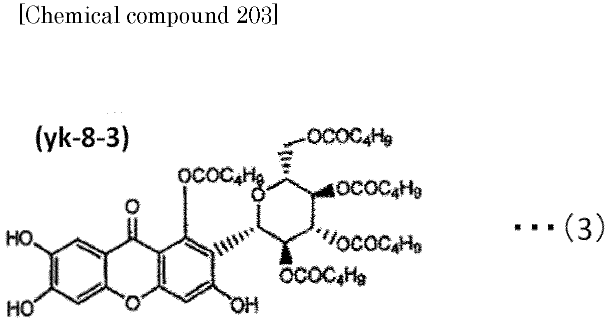

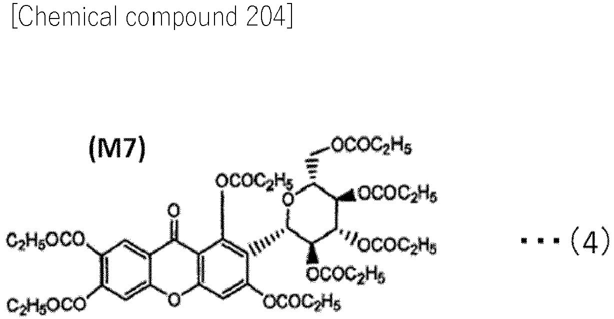

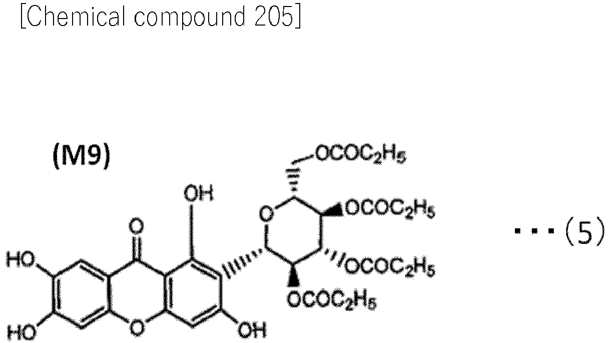

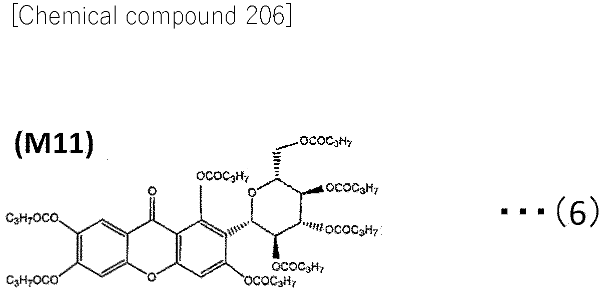

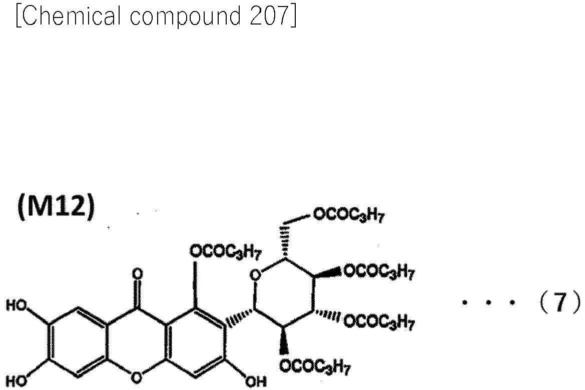

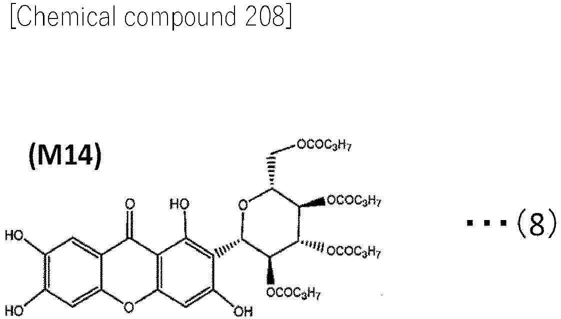

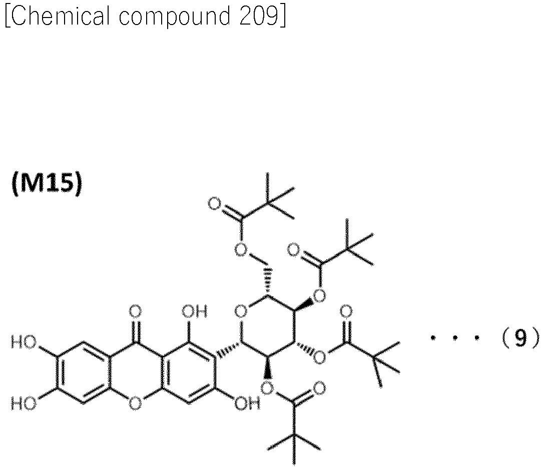

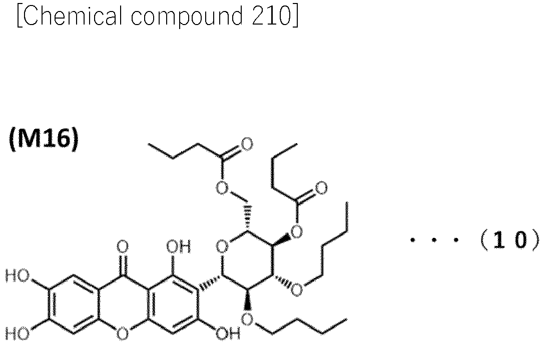

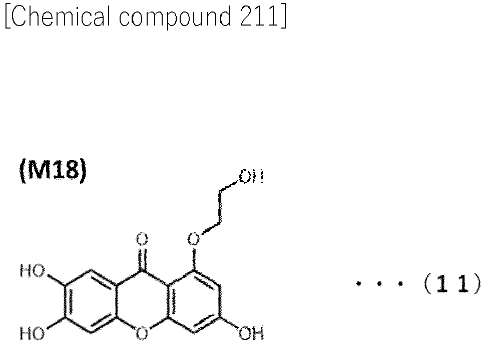

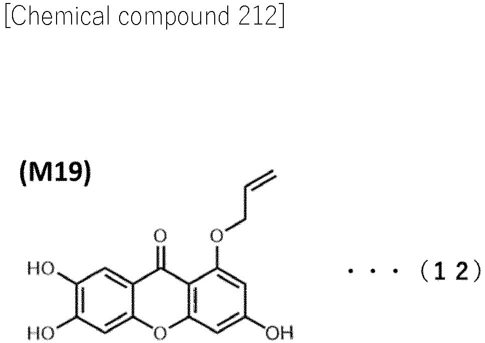

## Description

### [Technical Field]

The present invention relates to a compound that inhibit NF-xB-inducing kinase (also known as NIK-MAP3K14) useful for the treatment and/or prevention of malignant tumors such as malignant lymphoma and multiple myeloma. The present invention also relates to compositions, pharmaceutical compositions, and processed foods for the prevention and/or treatment of malignant tumors such as malignant lymphoma and multiple myeloma using said compounds.

### [Background Art]

NF-κB (Nuclear factor kappa B) is a transcription factor that regulates the expression of various genes involved in immune response, cell proliferation, apoptosis and carcinogenesis. The NF-κB is composed of five members: NF-κB p65 (p65), RelB, c-Rel, NF-κB1 (which are present in the form of both precursor p105 and cleavage type p50) and NF -kappaB2 (which are present in the form of both precursor p100 and cleavage type p52). Mainly, NF-κB1 (cleavage type p50; NF-κB p50) and p65 form heterodimers, and NE-κB2 (cleavage type p52; NF-κB p52) and RelB form heterodimers.

The activation of these NF-κB heterodimers is also performed by a signal transduction pathway that is strictly regulated by continuous events including phosphorylation reactions and proteolysis. These signaling pathway is classified into two pathways: the classical pathway (canonical pathway) and the non-classical pathway (non-canonical pathway).

NIK is a serine/threonine kinase and plays a role in both pathways; NIK is essential in non-classical signaling pathways and phosphorylates IKKα to partially degrade NF-κB p100 and release NF-κB p52.

NF-κB p52 forms a heterodimer with RelB, which then translocates into the nucleus and causes gene expression. In addition, the classical pathway forms a heterodimer of p65 and NF-κB p50 by activating the IKKα, IKKβ, and IKKγ complexes. This heterodimer translocation into the nucleus regulates gene expression.

NIK is activated by ligands such as B cell activating factor (BAFF), CD40 ligand, and tumor necrosis factor alpha (TNFα). It is known that NIK is important for the activation of signal transduction pathways by these ligands. Because of its important role, NIK expression is tightly regulated.

Under normal unstimulated conditions, the amount of NIK protein in cells is low because NIK is degraded by the interaction of NIK with the ubiquitin ligase, TNF receptor-associated factor (TRAF). When the non-classical pathway is stimulated by the ligand, the TRAF-NIK complex is thought to be dissociated by the activated receptor, thereby increasing NIK concentrations. (Non-Patent Literature 1).

BAFF is produced and secreted by T cells, monocytes/macrophages, dendritic cells, etc., and is known to regulate B cell differentiation, activation, survival, etc. via three types of receptors on B cells (Non-Patent Literature 2).

Known receptors for BAFF are BAFF-R (BAFF-Receptor), TACI (Transmenbrane activator and calcium modulator and cyclophilin ligand interactor) and BCMA (B cell maturation antigen) are known as receptors for BAFF.

BAFF-R and BCMA are expressed mainly on B cells, while TACI is expressed on B cells and activated T cells. The interaction of BAFF with BAFF-R activates the non-classical NF-κB signaling pathway through NIK activation.

It has been shown that the NF-κB pathway is constitutively activated in multiple myeloma (Non-Patent Literature 3 and Non-Patent Literature 4). It has also been shown that NIK gene amplification, deletion of the TRAF gene, and point mutations of the TRAF gene are observed in patients with multiple myeloma, and these genetic changes increase NIK protein expression and constitutively activate NIK, which is a factor that activates the NF-κB pathway. It has also been shown that inhibition of NIK with shRNA (NIK shRNA) suppresses NF-κB activation and induces cell death in multiple myeloma cell lines (Non-Patent Literature 3).

It has also been shown that serum BAFF levels are elevated in patients with multiple myeloma, and it has been reported that BAFF is secreted not only from monocytes and macrophages but also from multiple myeloma cells, and that the proliferation of multiple myeloma cells is enhanced by autocrine of BAFF (Non-Patent Literature 5 and Non-Patent Literature 6).

Similarly, point mutations in the TRAF gene and increased NIK protein expression have been shown in patients with hodgkin lymphoma, and cell death induction by NIK shRNA has been reported in hodgkin lymphoma cells as well (Non-Patent Literature 7).

Furthermore, NIK protein levels in the cytoplasm are also increased in adult T-cell leukemia cells, and NIK shRNA treatment has been shown to inhibit tumor growth in adult T-cell leukemia in vivo (Non-Patent Literature 8).

It is also shown that API 2-MAT1 fusion proteins produced in chromosome translocation (t (11; 18) (q21; q21)) in Mucosa associated lymphoid tissue (MALT) lymphoma induce constitutively activation of NIK by performing protein cleavage at the position of the 325-th arginine of NIK. This constitutively activation of NIK activates the non-classical NF-κB pathway, which is involved in cell adhesion and apoptosis resistance (Non-Patent Literature 9).

In diffuse large B-cell lymphoma (DLBCL) cells, NIK is highly expressed in the cytoplasm by BAFF stimulation. Activation of NIK by this high expression is an important signaling mechanism involved in lymphoma growth, and NIK shRNA has been shown to suppress the growth of DLBCL cell lines by inhibiting NIK-induced NF-κB activation in vitro (Non-Patent Literature 10).

BAFF expression has also been found in B lymphoma cells from patients with chronic B lymphoma, which has been shown to reduce drug-induced apoptosis (Non-Patent Literature 11). BAFF overexpressing mice have also been reported to induce the development of B lymphoma (Non-Patent Literature 5).

In addition, BAFF expression has been found in the serum and lymphoma cells of patients with MALT lymphoma, DLBCL, mantle cell lymphoma, hodgkin lymphoma, and burkitt lymphoma, which induces lymphoma cell proliferation and apoptosis resistance, and BAFF-high expressing lymphoma patients have a poorer prognosis compared to patients with low BAFF expression (Non-Patent Literature 12, Non-Patent Literature 13, and Non-Patent Literature 14).

In T lymphomas from patients with peripheral T lymphoma, NIK expression is higher than in T cells from healthy donors, activates the downstream non-classical NF-κB pathway, and patients with high nuclear NF-κB expression have a poorer prognosis compared to those with low expression. It has also been shown that NIK siRNA treatment can induce cell death in peripheral T lymphoma cells (Non-Patent Literature 15).

It has been reported that BAFF expression in serum is high in patients with acute lymphocytic leukemia and that there is a correlation between BAFF expression and an increase in acute lymphocytic leukemia cells (Non-Patent Literature 16 and Non-Patent Literature 17). It has also been shown that tumor cell proliferation is increased by non-classical pathway activation via NIK activation by BAFF in acute lymphocytic leukemia cells (Non-Patent Literature 18 and Non-Patent Literature 19).

The role of NIK in tumor cell growth is not limited to hematopoietic tumors; it has been shown that NIK is highly expressed in certain pancreatic cancer cell lines and that their cell proliferation is inhibited by NIK siRNA treatment (Non-Patent Literature 20).

It has also been shown that BAFF concentrations are higher in the serum of pancreatic cancer patients than in healthy individuals, and that BAFF concentrations correlate with tumor growth and stage progression. It has also been reported that BAFF-R is expressed in pancreatic cancer tissues, that NF-κB p52 and RelB are highly expressed, and that B lymphocytes surrounding pancreatic cancer tissues produce BAFF (Non-Patent Literature 21).

It has been reported that in basal-like breast cancer cell lines, high NIK expression induces constitutively activation of NF-xB (Non-Patent Literature 22). BAFF expression has also been found in tumor tissues of breast cancer patients, indicating that BAFF enhances breast cancer cell migration (Non-Patent Literature 23 and Non-Patent Literature 24).

In malignant melanoma, tissue microarray analysis has shown that NIK expression is significantly higher than in benign tissues, and NIK shRNA has been shown to inhibit tumor growth, induce apoptosis, and arrest the cell cycle in vivo (Non-Patent Literature 25).

Furthermore, NF-κB has been shown to be activated in non-small cell lung cancer tissues and cell lines, and NIK siRNA treatment has been shown to induce apoptosis and inhibit anchorage-independent cell proliferation (Non-Patent Literature 26).

NIK has also been shown to be highly expressed in hepatocarcinoma patients and hepatocarcinoma cell lines, and NIK siRNA treatment and miR-520e, which reduces NIK expression, have been shown to inhibit cell proliferation in vitro and tumor growth in vivo (Non-Patent Literature 27). It has also been reported that serum BAFF levels are higher in hepatocarcinoma patients compared to healthy controls and that BAFF levels correlate with disease progression and patient prognosis (Non-Patent Literature 28).

Helicobacter pylori is known to be deeply involved in the development of gastric cancer, and it has been shown that NIK is permanently activated at the site of gastric infection and activates the non-classical NF-κB pathway in patients infected with this bacteria. It has also been reported that gastric cancer cells transfected with mutants that inhibit NIK activation suppress NF-κB activation by Helicobacter pylori (Non-Patent Literature 29 and Non-Patent Literature 30). Furthermore, it has been shown that BAFF concentrations in feces are elevated in gastric cancer patients compared to healthy controls (Non-Patent Literature 31).

Activation of the non-classical NF-κB pathway is associated with the development of colitis and colon cancer, and in mice lacking NLRP12, which negatively regulates NIK, has been shown to activate the non-classical NF-κB pathway via NIK activation, inducing colitis and colon cancer. It has also been reported that the expression of OLFM1, which negatively regulates NIK in colon cancer patients, is higher in non-cancerous areas compared to cancerous areas, and that NIK siRNA treatment in colon cancer cells inhibits cell proliferation and migration (Non-Patent Literature 32 and Non-Patent Literature 33). Furthermore, it has been shown that higher serum BAFF concentrations are associated with shorter progression-free survival and overall survival in patients with colorectal cancer (Non-Patent Literature 34).

NIK and RelB are highly expressed in head and neck tumor cells, and siRNA treatment of NIK and RelB has been shown to inhibit tumor cell migration and invasion (Non-Patent Literature 35).

Overexpression of NIK and RelB has been found in renal cancer patients and has been shown to reduce 10-year survival compared to patients with low expression, and the status of NIK expression has been reported to be a prognostic factor (Non-Patent Literature 36). It has also been reported that BAFF expression is higher in renal cancer patients compared to healthy controls and that BAFF expression correlates with stage progression and patient prognosis (Non-Patent Literature 37).

It has been shown that overexpression of NIK in glioma cells promotes tumorigenesis and that activation of the non-classical NF-κB pathway by NIK activation enhances glioma cell migration and invasion (Non-Patent Literature 38).

NIK mRNA expression is higher in ovarian cancer patient tissues compared to normal ovarian tissues, and the NIK/NF-κB p52 (non-classical) pathway is activated in ovarian cancer cells, and NIK shRNA treatment inhibits anchorage-dependent and anchorage-independent cell proliferation. Furthermore, it has been shown that tumor growth in vivo is inhibited by NIK shRNA (Non-Patent Literature 39).

In patients with cervical cancer, it has been shown that cancer tissue is less differentiated and that NIK activation is higher as the disease stage progresses, and it has been reported that NIK activation inhibits apoptosis of cancer cells (Non-Patent Literature 40).

As described above, in malignant tumors such as multiple myeloma, malignant lymphoma (MALT lymphoma, DLBCL, mantle cell lymphoma, burkitt lymphoma, Hodgkin lymphoma, adult T cell leukemia, peripheral T lymphoma, etc.), pancreatic cancer, breast cancer, malignant melanoma, lung cancer, liver cancer, gastric cancer, colon cancer, head and neck tumor, glioma, renal cancer, ovarian cancer and cervical cancer, it is already common scientific knowledge that overexpression of BAFF and NIK occurs in malignant tumors, and that the activation of NIK activates NF-κB, which is the cause of these malignant tumors.

Therefore, pharmaceuticals and the like that can inhibit NIK activation by BAFF and NIK overexpression and suppress non-classical NF-κB signaling pathways have a therapeutic effect on BAFF overexpression, NIK and non-classical NF-κB signaling have a therapeutic effect on malignant tumors (including at least the diseases listed above) in which excessive activation of BAFF overexpression, NIK and non-classical NF-κB signaling is observed.

Multiple myeloma is also known to be associated with a characteristic concomitant condition called CRAB (hypercalcemia, renal impairment, anemia, and bone lesions).

The development of CRAB is associated with multiple myeloma cell-expressed monoclonal immunoglobulin (M protein), immunoglobulin free light chain, and osteoclast activating factors such as interleukin 6 (IL-6) and macrophage inflammatory protein 1α (MIP - 1α), have been reported to be involved (Non-Patent Literature 41, Non-Patent Literature 42, and Non-Patent Literature 43).

M protein and immunoglobulin free light chains caused by multiple myeloma are known to deposit in the kidneys, causing renal damage. They also deposit in systemic tissues, causing amyloidosis in various tissues and developing various symptoms such as neurological disorders and arrhythmias. Furthermore, in blood, they cause hyperviscosity syndrome.

IL-6 secretion in multiple myeloma has been shown to increase differentiation into osteoclasts and activate osteoclasts, which has been reported to progress bone lesions (Non-Patent Literature 42). MIP-la secreted by multiple myeloma has also been shown to enhance differentiation and activation of osteoclasts, thereby increasing bone lesions (Non-Patent Literature 43 and Non-Patent Literature 44). It has also been reported that the progression of bone lesions caused by these factors leads to hypercalcemia (Non-Patent Literature 45). In other words, osteoclast activating factors cause bone lesions, hypercalcemia associated with bone lesions, pathological fractures, spinal cord compression fractures, spinal cord compression symptoms associated with spinal cord compression fractures, and neurological symptoms associated with spinal cord compression symptoms.

It has been reported that myeloma develops in NF-xB2 mutant mice and the expression of M protein is increased (Non-Patent Literature 46). Furthermore, it has been reported that activation of the NF-κB pathway enhances the expression of osteoclast activating factors such as IL-6 and MIP-la (Non-Patent Literature 47).

IL-6 has also been shown to increase in patient serum with the progression of multiple myeloma stage and correlate with tumor progression. Furthermore, IL-6 secreted by multiple myeloma has been reported to promote proliferation and survival of multiple myeloma cells by autocrine (Non-Patent Literature 49).

It has been reported that serum MIP-la levels increase in patients with multiple myeloma as the disease progresses, which has been shown to correlate with prognosis. It has also been shown that MIP-la antisense inhibits tumor growth in vivo by blocking MIP-la autocrine (Non-Patent Literature 50).

As described above, it is already a scientific common sense that bone lesions caused by M protein expression and osteoclast activating factors develop in multiple myeloma due to activation of the NF-κB pathway. Furthermore, it is already common knowledge that increased expression of IL-6 and MIP-la induced by NF-κB pathway activation promotes multiple myeloma growth and survival by autocrine mechanism.

Therefore, pharmaceuticals and other products that can inhibit NIK activation, NIK activation via overexpression of NIK, and non-classical NF-κB signaling pathway, have therapeutic effect against M protein- and immunoglobulin free light chains-induced renal damage, amyloidosis, hyperviscosity syndrome, osteoclast activating factors-induced bone lesions (bone destruction), hypercalcemia associated with bone lesions, pathological fractures, spinal cord compression fractures, spinal cord compression symptoms associated with spinal cord compression fractures, and neurological symptoms associated with spinal cord compression symptoms in multiple myeloma. In other words, it has a therapeutic effect on CRAB. In addition, pharmaceuticals and other products that can inhibit NIK activation, NIK activation via overexpression of NIK, and non-classical NF-κB signaling pathway have a therapeutic effect on IL-6 and MIP-la autocrine-induced cell growth and survival of multiple myeloma.

Patent Literature 1 (JP 2016-531858) discloses 3-(1H-pyrazol-4-yl)-1H-pyrrolo[2,3-c]pyridine derivatives as NIK inhibitors. Here, the compound is shown to be an inhibitor of NIK, also known as NIK-MAP3K14, and the patent is granted for the compound to be used for the prevention or treatment of cancer.

In the Patent Literature 1, the said compound is only mentioned as an example of EC50 against three types of cancer cells, JJN-3, L-363, and LP-1, all of which are multiple myeloma cell lines. From the above, it is recognized as technical common knowledge that NIK inhibitors have a therapeutic effect on malignant tumors widely.

In addition, Patent Literature 2 (JP-A-H7-082263) discloses that xanthone compounds having the structure of formula (14) have anticancer activity.

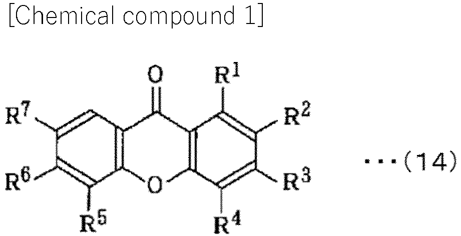

Furthermore, (R¹-R^{7:} H, -OH, C1-6 alkyl, C1-6 alkoxy, epoxypropoxy), and benzophenone compounds.

In addition, Patent Literature 3 (JP 2017-031146) describes that mangiferin inhibits NIK and is effective in multiple myeloma and malignant melanoma. As described above, compounds with xanthone skeleton were found to have anticancer activity.

### [Citation List]

### [Patent Literatures]

[Patent Literature 1] JP 2016-531858
[Patent Literature 2] JP-A-H7-082263
[Patent Literature 3] JP 2017-031146

### [Non-Patent Literature]

[Non-Patent Literature 1] Thu YM, Richmond A.: Cytokine Growth Factor Rev. 2010, 21, 213-226.
[Non-Patent Literature 2] Moore PA, Belvedere O, Orr A, Pieri K, LaFleur DW, Feng P, Soppet D, Charters M, Gentz R, Parmelee D, Li Y, Galperina O, Giri J, Roschke V, Nardelli B, Carrell J, Sosnovtseva S, Greenfield W Ruben SM, Olsen HS, Fikes J, Hilbert DM.: Science. 1999, 285, 260-263.
[Non-Patent Literature 3] Annunziata CM, Davis RE, Demchenko Y, Bellamy W, Gabrea A, Zhan F, Lenz G, Hanamura I, Wright G, Xiao W, Dave S, Hurt EM, Tan B, Zhao H, Stephens O, Santra M, Williams DR, Dang L, Barlogie B, Shaughnessy JD Jr, Kuehl WM, Staudt LM.: Cancer Cell. 2007, 12, 115-130.
[Non-Patent Literature 4] Demchenko YN, Glebov OK, Zingone A, Keats JJ, Bergsagel PL, Kuehl WM.: Blood. 2010, 115, 3541-3552.
[Non-Patent Literature 5] Sutherland AP, Mackay F, Mackay CR.: Pharmacol Ther. 2006, 112, 774-786.
[Non-Patent Literature 6] Novak AJ, Darce JR, Arendt BK, Harder B, Henderson K, Kindsvogel W, Gross JA, Greipp PR, Jelinek DF.: Blood. 2004, 103, 689-694.
[Non-Patent Literature 7] Ranuncolo SM, Pittaluga S, Evbuomwan MO, Jaffe ES, Lewis BA.: Blood. 2012, 120, 3756-3763.
[Non-Patent Literature 8] Saitoh Y, Yamamoto N, Dewan MZ, Sugimoto H, Martinez Bruyn VJ, Iwasaki Y, Matsubara K, Qi X, Saitoh T, Imoto I, Inazawa J, Utsunomiya A, Watanabe T. Masuda T, Yamamoto N, Yamaoka S.: Blood. 2008, 111, 5118-5129.
[Non-Patent Literature 9] Rosebeck S, Madden L, Jin X, Gu S, Apel IJ, Appert A, Hamoudi RA, Noels H, Sagaert X, Van Loo P, Baens M, Du MQ, Lucas PC, McAllister-Lucas LM.: Science. 2011, 331, 468-472.
[Non-Patent Literature 10] Pham LV, Fu L, Tamayo AT, Bueso-Ramos C, Drakos E, Vega F, Medeiros LJ, Ford RJ.: Blood. 2011, 117, 200-210.
[Non-Patent Literature 11] Kern C, Cornuel JF, Billard C, Tang R, Rouillard D, Stenou V, Defrance T, Ajchenbaum-Cymbalista F, Simonin PY, Feldblum S, Kolb JP.: Blood. 2004, 103, 679-688.
[Non-Patent Literature 12] He B, Chadburn A, Jou E, Schattner EJ, Knowles DM, Cerutti A.: J Immunol. 2004, 172, 3268-3279.
[Non-Patent Literature 13] Novak AJ, Grote DM, Stenson M, Ziesmer SC, Witzig TE, Habermann TM, Harder B, Ristow KM, Bram RJ, Jelinek DF, Gross JA, Ansell SM.: Blood. 2004, 10. 4, 2247-2253.
[Non-Patent Literature 14] Oki Y, Georgakis GV, Migone TS, Kwak LW, Younes A.: Haematologica. 2007, 92, 269-270.
[Non-Patent Literature 15] Odqvist L, Sánchez-Beato M, Montes-Moreno S, Martin-Sánchez E, Pajares R, Sánchez-Verde L, Ortiz-Romero PL, Rodriguez J, Rodriguez-Pinilla SM, Iniesta-Martinez F, Solera-Arroyo JC, Ramos-Asensio R, Flores T, Palanca JM, Bragado FG, Franjo PD, Piris MA.: Clin Cancer Res. 2013, 19, 2319-2330.
[Non-Patent Literature 16] Sun B, Li L, Xu M, Wang X, Wang F, Ni H.: J. Pediatr. Hematol. Oncol. 2016, 38: 167-172.
[Non-Patent Literature 17] Jablonska E, Dakowicz L, Ratajczak-Wrona W, Garley M, Sawicka-Powierza J, Krawczuk-Rybak M.: Clin Lab. 2014, 60, 1757-1764.
[Non-Patent Literature 18] Maia S, Pelletier M, Ding J, Hsu YM, Sallan SE, Rao SP, Nadler LM, Cardoso AA.: PLoS One 2011, 6, e20787.
[Non-Patent Literature 19] Rihacek M, Bienertova-Vasku J, Valik D, Sterba J, Pilatova K, Zdrazilova-Dubska L.: Biomed. Res. Int. 2015, 2015, 792187.
[Non-Patent Literature 20] Nishina T, Yamaguchi N, Gohda J, Semba K, Inoue J.: Biochem Biophys Res Commun. 2009, 388, 96-101.
[Non-Patent Literature 21] Koizumi M, Hiasa Y, Kumagi T, Yamanishi H, Azemoto N, Kobata T, Matsuura B, Abe M, Onji M.: PLoS One. 2013, 8, e71367.
[Non-Patent Literature 22] Yamamoto M, Ito T, Shimizu T, Ishida T, Semba K, Watanabe S, Yamaguchi N, Inoue J.: Cancer Sci. 2010, 101, 2391-2397.
[Non-Patent Literature 23] Zhu J, Sun L, Lin S, Zhao R, Zhou L, Fang D, Chen L, Liu J, Shi W, Zhang L, Yuan S.: J. Exp. Clin. Cancer Res. 2012, 31, 31.
[Non-Patent Literature 24] Pelekanou V, Notas G, Athanasouli P, Alexakis K, Kiagiadaki F, Peroulis N, Kalyvianaki K, Kampouri E, Polioudaki H, Theodoropoulos P, Tsapis A, Castanas E, Kampa M.: Front. Oncol. 2018, 8, 301.
[Non-Patent Literature 25] Thu YM, Su Y, Yang J, Splittgerber R, Na S, Boyd A, Mosse C, Simons C, Richmond A.: Oncogene. 2012, 31, 2580-2592.
[Non-Patent Literature 26] Saitoh Y, Martinez Bruyn VJ, Uota S, Hasegawa A, Yamamoto N, Imoto I, Inazawa J, Yamaoka S.: Lung Cancer. 2010, 70, 263-270.
[Non-Patent Literature 27] Zhang S, Shan C, Kong G, Du Y, Ye L, Zhang X.: Oncogene. 2012, 31, 3607-3620.
[Non-Patent Literature 28] Khlaiphuengsin A, Chuaypen N, Pinjaroen N, Sirichindakul B, Hirankarn N, Tangkijvanich P.: Plasma B-cell activating factor levels and polymorphisms in hepatitis B-related hepatocellular carcinoma: clinical correlation and prognosis. Asian Pac J Allergy Immunol. 2020. In press.
[Non-Patent Literature 29] Feige MH, Vieth M, Sokolova O, Täger C, Naumann M.: Biochim Biophys Acta Mol Cell Res. 2018, 1865, 545-550.
[Non-Patent Literature 30] Maeda S, Yoshida H, Ogura K, Mitsuno Y, Hirata Y, Yamaji Y, Akanuma M, Shiratori Y, Omata M.: Gastroenterology. 2000, 119, 97-108.
[Non-Patent Literature 31] Xie C, Quan R, Wang L, Chen C, Yan W, Fu Y.: Clin. Exp. Immunol. 2019, 198, 131-140.
[Non-Patent Literature 32] Allen IC, Wilson JE, Schneider M, Lich JD, Roberts RA, Arthur JC, Woodford RM, Davis BK, Uronis JM, Herfarth HH, Jobin C, Rogers AB, Ting JP.: Immunity. 2012, 36, 742-754.
[Non-Patent Literature 33] Shi W, Ye Z, Zhuang L, Li Y, Shuai W, Zuo Z, Mao X, Liu R, Wu J, Chen S, Huang W.: J Pathol. 2016, 240, 352-365.
[Non-Patent Literature 34] Chereches G, Barbos O, Buiga R, Balacescu O, Iancu D, Todor N, Balacescu L, Miron N, Bejinariu N, Ciuleanu TE.: J. BUON 2017, 22, 658 -666.
[Non-Patent Literature 35] Das R, Coupar J, Clavijo PE, Saleh A, Cheng TF, Yang X, Chen J, Van Waes C, Chen Z.: Mol Carcinog. 2019, 58, 411-425.
[Non-Patent Literature 36] Lua J, Qayyum T, Edwards J, Roseweir AK.: Urol Int. 2018, 101, 190-196.
[Non-Patent Literature 37] Jiang M, Lin J, Xing H, An J, Yang J, Wang B, Yu M, Zhu Y.: Peer J 2020, 8, e9453.
[Non-Patent Literature 38] Cherry EM, Lee DW, Jung JU, Sitcheran R.: Mol Cancer. 2015 Jan 27;14:9.
[Non-Patent Literature 39] Uno M, Saitoh Y, Mochida K, Tsuruyama E, Kiyono T, Imoto I, Inazawa J, Yuasa Y, Kubota T, Yamaoka S.: PLoS One. 2014, 9, e88347.
[Non-Patent Literature 40] Zhou X, Li H, Chai Y, Liu Z.: Int J Gynecol Cancer. 2015, 25, 770-778.
[Non-Patent Literature 41] Rajkumar SV, Dimopoulos MA, Palumbo A, Blade J, Merlini G, Mateos MV, Kumar S, Hillengass J, Kastritis E, Richardson P, Landgren O, Paiva B, Disp enzieri A, Weiss B, LeLeu X, Zweegman S, Lonial S, Rosinol L, Zamagni E, Jagannath S, Sezer O, Kristinsson SY, Caers J, Usmani SZ, Lahuerta JJ, Johnsen HE, Beksac M, Cavo M, Goldschmidt H, Terpos E, Kyle RA, Anderson KC, Durie BG, Miguel JF.: Lancet Oncol. 2014, 15, e538-48.
[Non-Patent Literature 42] Harmer D, Falank C, Reagan MR.: Front. Endocrinol. 2019, 9, 788.
[Non-Patent Literature 43] Roussou M, Tasidou A, Dimopoulos MA, Kastritis E, Migkou M, Christoulas D, Gavriatopoulou M, Zagouri F, Matsouka C, Anagnostou D, Terpos E.: Leukemia 2009, 23, 2177-2181.
[Non-Patent Literature 44] Tsubaki M, Kato C, Isono A, Kaneko J, Isozaki M, Satou T, Itoh T, Kidera Y, Tanimori Y, Yanae M, Nishida S.: J. Cell. Biochem. 2010, 111, 1661 - 1672.
[Non-Patent Literature 45] Lee OL, Horvath N, Lee C, joshua D, Ho J, Szer J, Quach H, Spencer A, Harrison S, Mollee P, Roberts AW, Talaulikar D, Brown R, Augustson B, Ling S, Jaksic W, Gibson J, Kalff A, Johnston A, Kalro A, Ward C, Prince HM, Zannettino A.: Intern. Med. J. 2017, 47, 938-951.
[Non-Patent Literature 46] McCarthy BA, Yang L, Ding J, Ren M, King W, ElSalanty M, Zakhary I, Sharawy M, Cui H, Ding HF.: BMC Cancer. 2012, 12, 203.
[Non-Patent Literature 47] Vrabel D, Pour L, Sevcikov S.: Blood Rev. 2019, 34, 56-66.
[Non-Patent Literature 48] Hu L, Wu W, Chai X, Luo J, Wu Q.: Bioorg. Med. Chem. Lett. 2011, 21, 4013-4015.
[Non-Patent Literature 49] Frassanito MA, Cusmai A, Iodic e G, Dammacco F. Autocrine interleukin -6 production and highly malignant multiple myeloma: relation with resistance to drug-induced apoptosis. Blood. 2001.97. 483-489.
[Non-Patent Literature 50] Terpos E, Politou M, Viniou N, Rahemtulla A. Significance of Leuk Lymphoma. 2005. 46. 1699-1707.

### [Summary of Invention]

### [Technical Problem]

As shown in Patent Literature 1, chemical agents are used to inhibit NIK, but with the exception of mangiferin in Patent Literature 3, the medication route is often intravenous administration, which places a heavy burden on the patient. Therefore, the purpose of this invention is to provide therapeutic agents and ameliorating compositions that inhibit NIK and ameliorate malignant tumors such as malignant lymphoma, lymphoid leukemia, and multiple myeloma, even when administered orally.

Although the mangiferin of Patent Literature 3 is effective by oral administration, the required intake amount is large, and even if oral, it could not be easily taken. Therefore, a more effective or active therapeutic agent and an improvement composition were sought.

Furthermore, new compounds or improved compounds of existing drugs that inhibit kinases such as NIK are always needed to develop more effective pharmaceuticals and other products for treat and prevent malignant tumors.

### [Solution to Problem]

The inventors investigated compounds that inhibit NIK to solve the above problem, and found a compound with a xanthone skeleton that has a skeleton not found before and has NIK inhibitory activity, and confirmed that it actually induces cell death in malignant lymphoma, lymphocytic leukemia, multiple myeloma, bortezomib-resistant multiple myeloma, and rituximab-resistant malignant lymphoma. The compounds were also found to markedly inhibit tumor growth in malignant lymphoma and multiple myeloma in vivo.

Furthermore, these compounds were found to induce transformation from plasma cells to B cell-like cells by inducing decreased expression of CD138, a marker of multiple myeloma malignancy, and increased expression of CD20, a B cell marker, and were confirmed to be capable of treating multiple myeloma when used in combination with anti-CD20 monoclonal antibodies.

In addition, we confirmed that these compounds inhibit monoclonal immunoglobulin production, immunoglobulin free light chain production, and bone destruction inducing factor production in multiple myeloma, thereby reducing the symptoms of CRAB that occur in patients with multiple myeloma. Furthermore, we confirmed that these compounds inhibit IL-6 and MIP-la secretion, which are involved with autocrine-induced cell proliferation and survival in multiple myeloma cells, thereby inhibiting cell proliferation and survival in multiple myeloma.

In other words, the new compounds of the present invention are compounds of the following formulas (1) to (12).

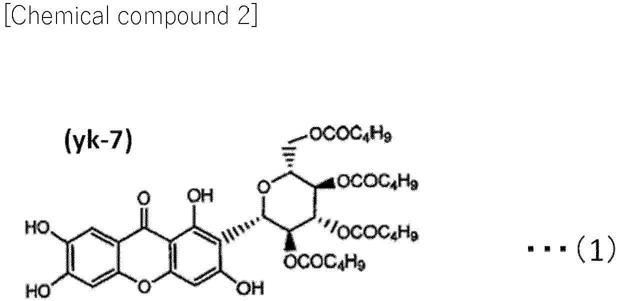

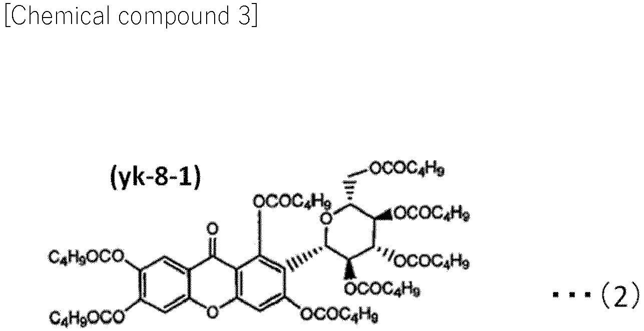

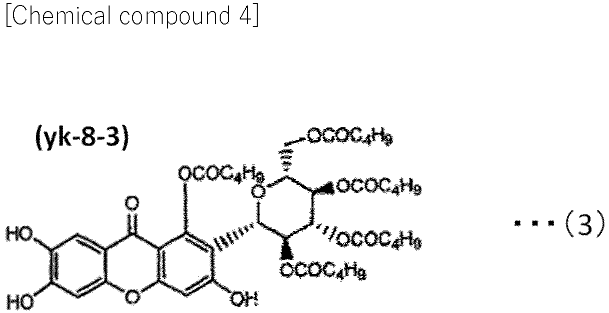

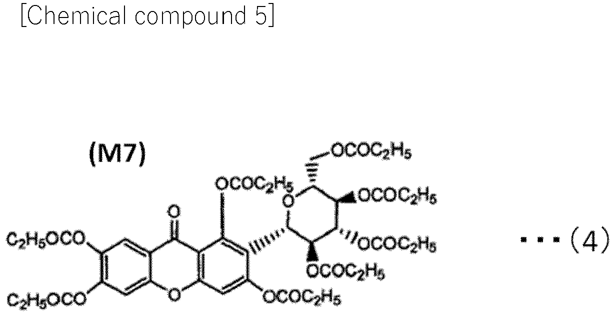

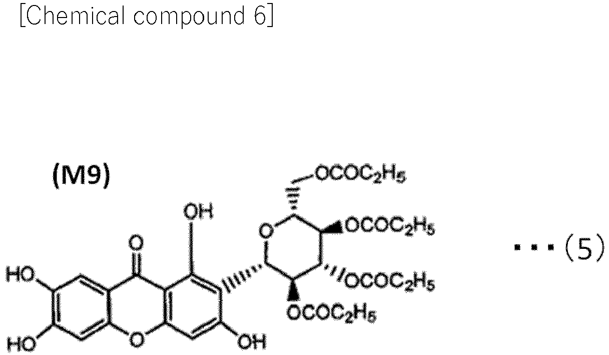

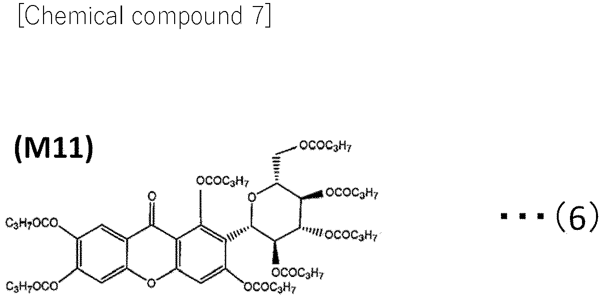

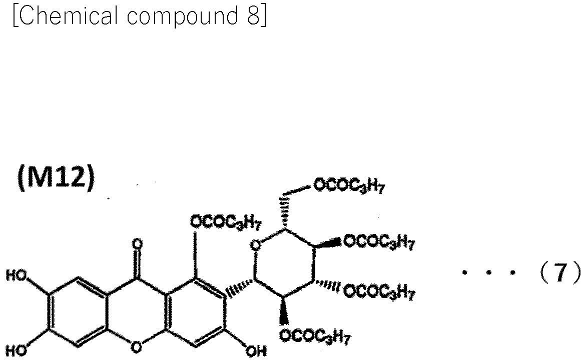

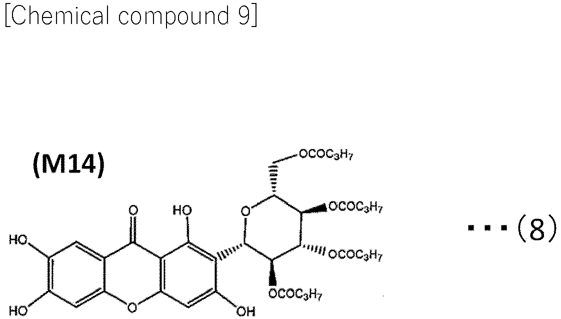

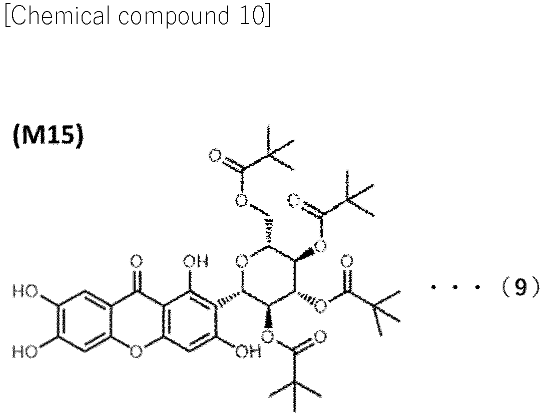

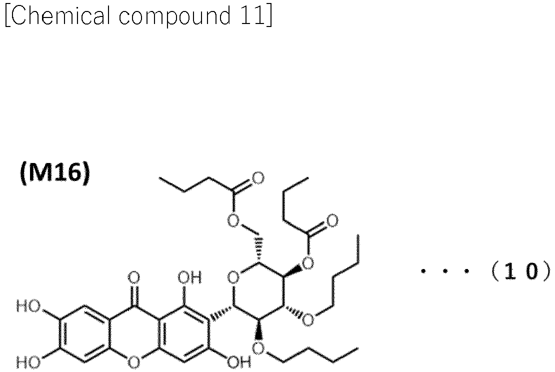

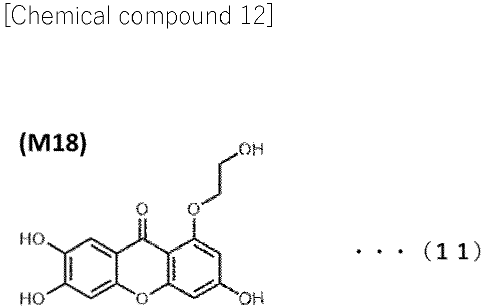

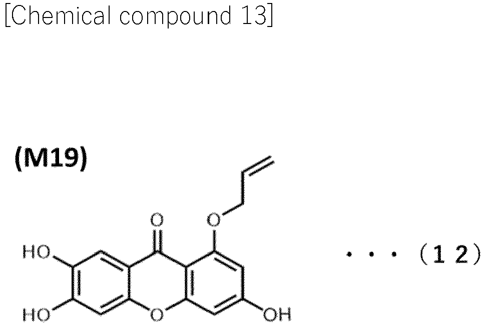

The compositions for improving malignant tumor diseases are characterized in that they contain at least one of the compounds of formulas (1) to (12) above as an active ingredients.

### [Advantageous Effects of Invention]

The pharmaceutical compounds of the present invention can improve not only malignant lymphoma, lymphocytic leukemia, and multiple myeloma, but also malignant tumors that have acquired drug resistance, such as bortezomib-resistant multiple myeloma and rituximab-resistant malignant lymphoma. For example, it can effectively induce cell death in malignant tumor cells such as malignant lymphoma, lymphocytic leukemia, multiple myeloma, bortezomib-resistant multiple myeloma, and rituximab-resistant malignant lymphoma. In addition, it does not affect normal cells at concentrations that induce cell death in malignant tumor cells.

The compounds can also decrease CD138, a known marker for myeloma, and increase CD20, a known marker for lymphoma. In other words, they can return myeloma into B cell-like states. This is called "transformation to B-cell-like". Anti-human CD20 monoclonal antibody drugs such as rituximab, obinutuzumab, ofatumumab, and ibritumomab tiuxetan are known as specific agents for lymphomas of CD20-expressing cells.

In other words, combining these compounds with anti-human CD20 monoclonal antibody drugs such as rituximab, obinutuzumab, ofatumumab, ibritumomab tiuxetan, which are known as specific drugs for lymphoma, can improve myeloma (plasmacytoma).

In addition, the malignant tumors mentioned above are thought to enhance the development, proliferation and survival of malignant tumor cells by overexpression of NIK protein. Therefore, the compounds for improving malignant lymphoma, lymphocytic leukemia, and multiple myeloma, etc. is considered to have an improving effect not only on the malignant tumors shown in the examples, but also on other malignant tumors.

Furthermore, the compounds can inhibit monoclonal immunoglobulin production, immunoglobulin free light chain production, and bone destruction-inducing factor production associated with multiple myeloma. Thus, they can ameliorate the so-called CRAB associated with these productions, such as hypercalcemia, renal disorders, anemia, and bone lesions.

The compounds of this invention can also inhibit renal damage due to increased expression of M protein, immunoglobulin free light chain, IL-6 and MIP-la, etc., amyloidosis, hyperviscosity syndrome, bone lesions (bone destruction) due to increased expression of osteoclast activating factor, hypercalcemia associated with bone lesions, pathological fractures, spinal cord compression fractures, spinal cord compression symptoms associated with spinal cord compression fractures, and neurological symptoms associated with spinal cord compression symptoms. In other words, they can inhibit the development of CRAB. Furthermore, the compounds can inhibit IL-6 and MIP-la production in multiple myeloma. Therefore, they can inhibit cell proliferation and survival by IL-6 and MIP-la autocrine in multiple myeloma.

### [Brief Description of Drawing]

[Figure 1] The graph shows the cell death inducing effect of each compound in KMS-28 cells, as examined by trypan blue staining.
[Figure 2] The graph shows the cell death inducing effect of each compound in L363 cells, as examined by trypan blue staining.
[Figure 3] The graph shows the cell death inducing effect of each compound in RPMI8226 cells, as examined by trypan blue staining.
[Figure 4] The graph shows the cell death inducing effect of each compound in bortezomib-resistant RPMI8226 cells, as examined by trypan blue staining.
[Figure 5] The graph shows the cell death inducing effect of each compound in Rec-1 cells, as examined by trypan blue staining.
[Figure 6] The graph shows the cell death inducing effect of each compound in Raji cells, as examined by trypan blue staining.
[Figure 7] The graph shows the cell death-inducing effect of rituximab in Raji and RR1 cells, as examined by trypan blue staining.
[Figure 8] The graph shows the cell death inducing effect of each compound in RR1 cells, as examined by trypan blue staining.
[Figure 9] The graph shows the cell death inducing effect of each compound in SU-DHL-4 cells, as examined by trypan blue staining.
[Figure 10] The graph shows the cell death inducing effect of each compound in CCRF-SB cells, as examined by trypan blue staining.
[Figure 11] The graph shows the cell death inducing effect of M9, M11, M12, M14, M15, M16, M18, and M19 in MEC-1 cells, as examined by trypan blue staining.
[Figure 12] The graph shows the cell death inducing effect of M9, M11, M12, M14, M15, M16, M18, and M19 in HUT-78 cells, as examined by trypan blue staining.
[Figure 13] The graph shows the cell death inducing effect of M9, M11, M12, M14, M15, M16, M18, and M19 in ATN-1 cells, as examined by trypan blue staining.
[Figure 14] The graph shows the cell death inducing effect of each compound in RPMI1788 cells, as examined by trypan blue staining.
[Figure 15] The image shows the results of western blotting of phospho-NIK, NIK, phospho-IKK, IKK, β-actin, NF-κB p52 nuclear, NF-κB p65 nuclear, and Lamin expression in KMS-28BM cells treated with mangiferin 8a, norathyriol, M7, M8, M9, yk-7, yk-8-1 and yk-8-3.
[Figure 16] The image shows the results of western blotting of phospho-NIK, NIK, phospho-IKK, IKK, β-actin, NF-κB p52 nuclear, NF-κB p65 nuclear, and Lamin expression in KMS-28BM cells treated with M11, M12, M14, M15, M16, M18, and M19.
[Figure 17] This graph shows the expression status of CD138 in KMS-28BM cells for each compound of M7, M9, and yk-7.
[Figure 18] This graph shows the expression status of CD138 in KMS-28BM cells for each compound of yk-8-3 and yk-8-1.
[Figure 19] This graph shows the expression status of CD138 in KMS-28BM cells for each compound of M11, M12, M14, and M15.
[Figure 20] This graph shows the expression status of CD138 in KMS-28BM cells for each compound of M16, M18, and M19.
[Figure 21] This graph shows the expression status of CD20 in KMS-28BM cells for each compound of M7, M9, and yk-7.
[Figure 22] This graph shows the expression status of CD20 in KMS-28BM cells for each compound of yk-8-3 and yk-8-1.
[Figure 23] This graph shows the expression status of CD20 in KMS-28BM cells for each compound of M11, M12, M14, and M15.
[Figure 24] This graph shows the expression status of CD20 in KMS-28BM cells for each compound of M16, M18, and M19.
[Figure 25] This graph shows the expression status of CD138 in L363 cells for each compound of M7, M9, and yk-7.
[Figure 26] This graph shows the expression status of CD138 in L363 cells for each compound of yk-8-3 and yk-8-1.
[Figure 27] This graph shows the expression status of CD138 in L363 cells for each compound of M11, M12, M14, and M15.
[Figure 28] This graph shows the expression status of CD138 in L363 cells for each compound of M16, M18, and M19.
[Figure 29] This graph shows the expression status of CD20 in L363 cells for each compound of M7, M9, and yk-7.
[Figure 30] This graph shows the expression status of CD20 in L363 cells for each compound of yk-8-3 and yk-8-1.
[Figure 31] This graph shows the expression status of CD20 in L363 cells for each compound of M11, M12, M14, and M15.
[Figure 32] This graph shows the expression status of CD20 in L363 cells for each compound of M16, M18, and M19.
[Figure 33] The graph shows the results of the cell death induction effect of M9, M14, M15, M16, M18, and M19 combined with rituximab in KMS-28BM cells, as examined by trypan blue staining.
[Figure 34] The graph shows the results of the cell death induction effect of M9, M14, M15, M16, M18, and M19 combined with rituximab in L363 cells, as examined by trypan blue staining.
[Figure 35] The graph shows the inhibitory effect of each compound on the production of IgG in KMS-28BM cells.
[Figure 36] The graph shows the inhibitory effect of each compound on the production of λ light chains in KMS-28BM cells.
[Figure 37] The graph shows the inhibitory effect of each compound on the production of IL-6 in KMS-28BM cells.
[Figure 38] The graph shows the inhibitory effect of each compound on the production of IgG in L363 cells.
[Figure 39] The graph shows the inhibitory effect of each compound on the production of λ light chains in L363 cells.
[Figure 40] The graph shows the inhibitory effect of each compound on the production of MIP-la in L363 cells.
[Figure 41] A graph showing the inhibitory effect of M9 on tumor growth after transplantation of L363 cells into mice.
[Figure 42] These are the photograph of mice in which L363 cells were transplanted into mice and the tumor growth inhibitory effect of M9 was examined.
[Figure 43] A graph showing the inhibitory effect of M9 and M14 on tumor growth after transplantation of Raji cells into mice.
[Figure 44] These are the photograph of mice in which Raji cells were transplanted into mice and the tumor growth inhibitory effect of M9 and M14 was examined.

### [Description of Embodiments]

The following is a description of the new compounds related to the present invention and pharmaceutical compositions and the like containing the new compounds as active ingredients to improve malignant tumor diseases such as malignant lymphoma, lymphocytic leukemia, multiple myeloma, bortezomib-resistant multiple myeloma, and rituximab-resistant malignant lymphoma. The following description is an example of an embodiment and an example of the invention, and the invention is not limited to the following description. The following embodiments may be modified to the extent that they do not depart from the purpose of the invention.

In this description, "improvement" may include not only treatment of the target disease or symptom, but also reduction and suppression of the symptom. The term "inhibition of cell proliferation" may include not only slowing down the rate of proliferation of the target cell, but may also mean that the cell prevents proliferation, that the cell dies, or that the cell loses its function.

The new compounds pertaining to the present invention are represented by formulas (1) to (12) with a xanthone skeleton. Hereinafter, "compound regarding the present invention" may refer to at least one of the following 12 compounds.

Formula (1) is 2',3',4',6'-tetra-O-valerylmangiferin (hereafter referred to as "yk-7").

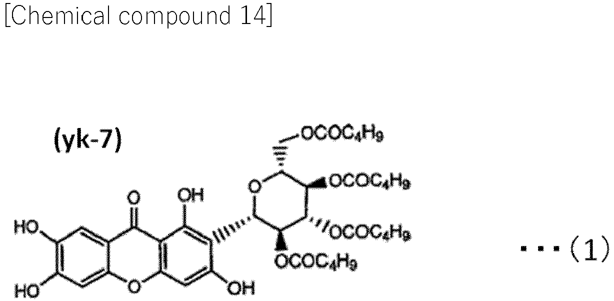

Formula (2) is 1,3,2',3',4',6,6',7-octa-O-valerylmangiferin (hereafter referred to as "yk-8-1").

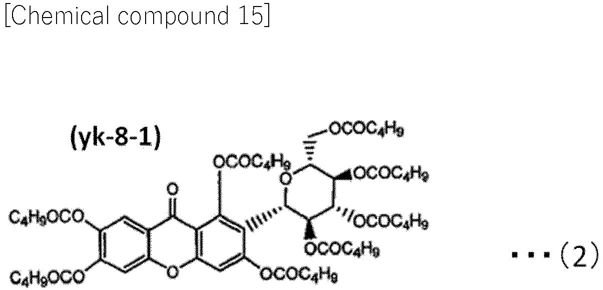

Formula (3) is 1,2',3',4',6-penta-0-valerylmangiferin (hereafter referred to as "yk-8-3").

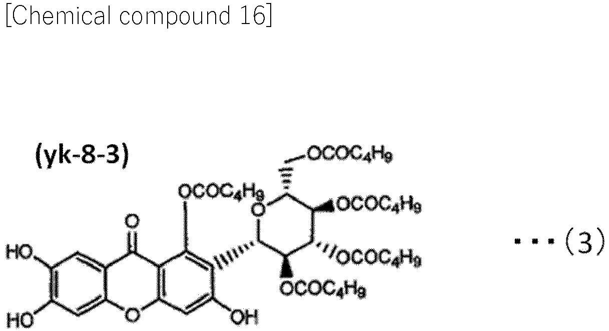

Formula (4) is 1,3,2',3',4',6,6',7-octa-O-propionylmangiferin (hereafter referred to as "M7").

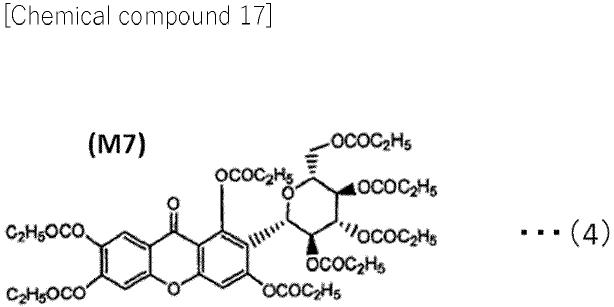

Formula (5) is 2',3',4',6'-tetra-O-propionylmangiferin (hereafter referred to as "M9").

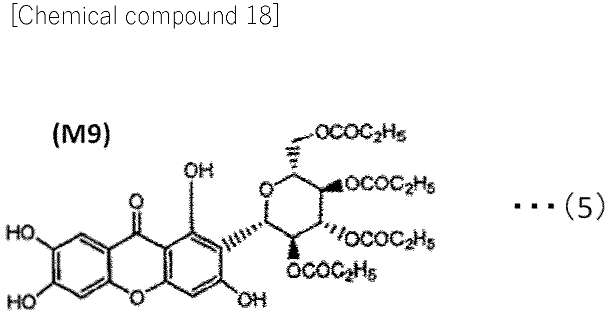

Formula (6) is 1,3,2',3',4',6,6',7-octa-O-butyrylmangiferin (hereafter referred to as "M11").

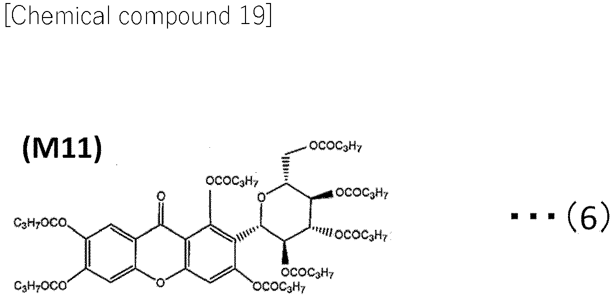

Formula (7) is 1,2',3',4',6-penta-O-butyrylmangiferin (hereafter referred to as "M12").

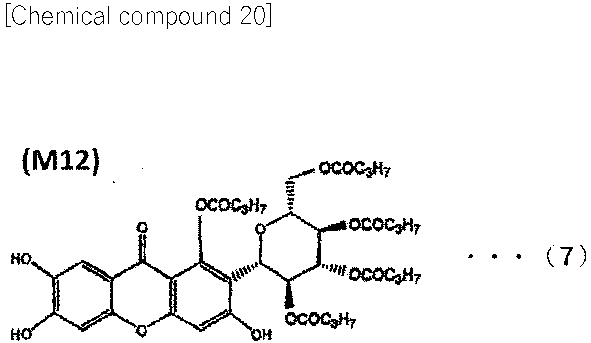

Formula (8) is 2',3',4',6'-tetra-O-butyrylmangiferin (hereafter referred to as "M14").

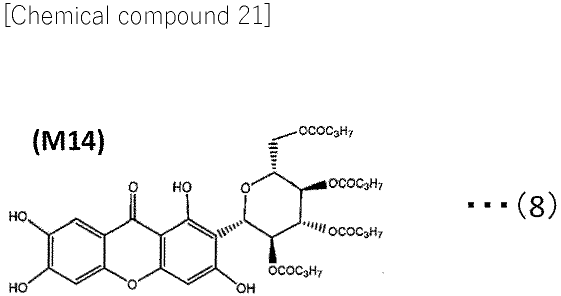

Formula (9) is 2',3',4',6'-tetra-O-pivaloylmangiferin (hereafter referred to as "M15").

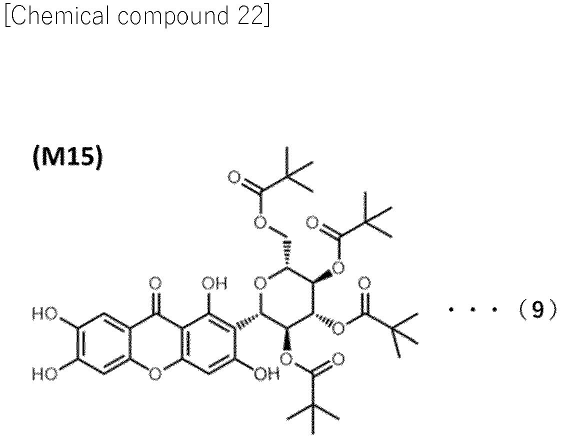

Formula (10) is 2',3'-di-O-butyl-4',6'-di-O-butyrylmangiferin (hereafter referred to as "M16").

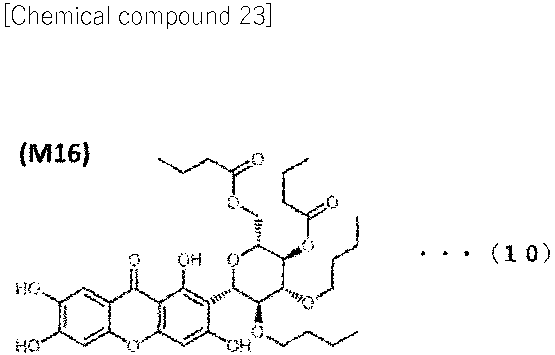

Formula (11) is 3,6,7-trihydroxy-1-(2-hydroxyethoxy)-9H-xanthen-9-one (hereafter "M18").

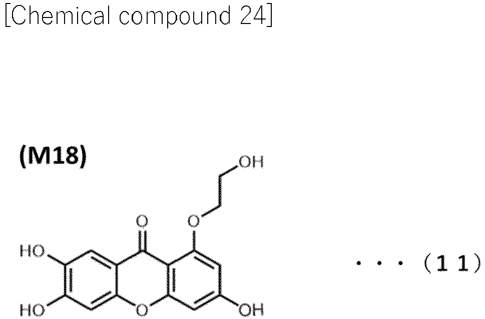

Formula (12) is 1-allyloxy-3,6,7-trihydroxy-9H-xanthen-9-one (hereafter referred to as "M19").

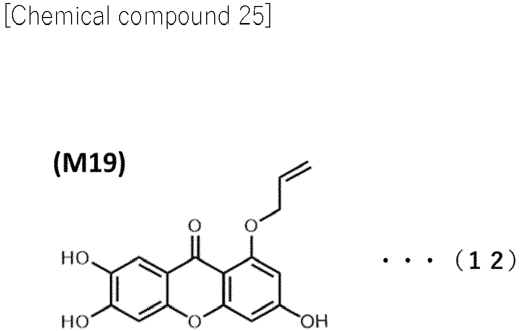

The substitution position numbers were numbered as shown in formula (13).

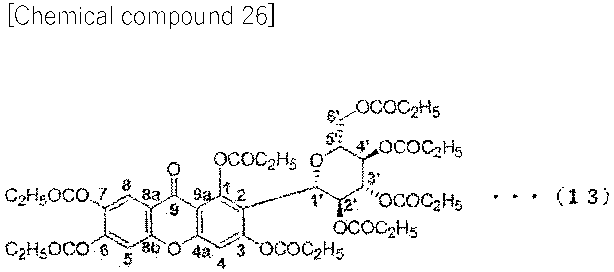

These compounds of the present invention can be made into compositions for growth inhibition of malignant tumor cells such as malignant lymphoma, lymphocytic leukemia, multiple myeloma, bortezomib-resistant multiple myeloma, and rituximab-resistant malignant lymphoma. Its mechanism of action is to inhibit NIK in the intracellular signaling pathway. Therefore, the compounds for growth inhibition of malignant tumor cells can inhibit the growth of cancer cells such as pancreatic cancer, breast cancer, malignant melanoma, lung cancer, liver cancer, gastric cancer, colon cancer, head and neck tumor, glioma, renal cancer, ovarian cancer, and uterine cancer as well as the above mentioned ones.

In other words, it can be said that the compositions for inhibiting the growth of malignant tumor cells contains at least one of the compounds of the present invention as an active ingredients.

Like mangiferin, the compounds is a relatively low molecular weight compounds that dissolves well in water, can be taken orally, and has no effect on normal cells, such as inhibition of cell growth. Therefore, when applied to the human body, it is expected to work as an ameliorative composition with minimal side effects. Therefore, the compounds can be used as a pharmaceutical composition to improve malignant tumor diseases.

The pharmaceutical composition for the improvement of malignant tumor disease of the present invention contains at least any one of the above 12 compounds of the present invention as an active ingredients. It may also contain other ingredients acceptable as drugs.

The pharmaceutical compositions of the present invention can be effective when administered orally. Therefore, they can be provided as an internal drug. For example, the compositions for improving malignant tumor disease in powder form can be formulated into capsules, granules, dispersions, tablets, etc. For oral dosage forms, additives such as binders, lubricants, disintegrants, coloring agents, flavoring substances, preservatives, antioxidants, and stabilizers are added, and capsules, granules, dispersions, and tablets can be manufactured according to conventional methods.

The pharmaceutical compositions of the present invention can be administered by intravenous, subcutaneous, or intramuscular injection. Furthermore, the pharmaceutical compositions of the present invention may be formulated into topical formulations such as liquids, ointments, creams, gels, patchs, and aerosols for parenteral administration. When made into topical formulations, water, lower alcohols, solubilizing agents, surfactants, emulsion stabilizers, gelling agents, adhesives, and other necessary base ingredients can be added. Additives such as vasodilators, adrenal corticosteroids, keratolytic agents, moisturizers, disinfectants, antioxidants, coolants, fragrances, and dyes may also be added as needed.

Furthermore, since the compounds of the invention can inhibit NIK signaling, the pharmaceutical compositions of the present invention can improve malignant tumor diseases such as multiple myeloma, lymphoid leukemia, malignant lymphoma (MALT lymphoma, DLBCL, burkitt lymphoma, hodgkin lymphoma, adult T cell leukemia, peripheral T lymphoma, etc.), bortezomib-resistant multiple myeloma, rituximab-resistant malignant lymphoma, pancreatic cancer, breast cancer, malignant melanoma, lung cancer, liver cancer, gastric cancer, colon cancer, head and neck tumor, glioma, renal cancer, ovarian cancer, and cervical cancer.

In addition, because the compounds of the present invention inhibit the production of monoclonal immunoglobulins such as IgG, immunoglobulin free light chains, and osteoclast activating factors such as IL-6 and MIP-la from myeloma cells, the pharmaceutical compositions of the present invention are also pharmaceutical compositions for the amelioration of symptoms such as CRAB (hypercalcemia, renal disorders, anemia, and bone lesions), which are known as concomitant symptoms of multiple myeloma.

Furthermore, the compounds also inhibit the production of monoclonal immunoglobulins such as IgG and immunoglobulin free light chains by suppressing myeloma cells. Amyloidosis and hyperviscosity syndromes caused by excessive production of these are not included in CRAB. However, they can be said to be concomitant symptoms of multiple myeloma. Therefore, in this description, amyloidosis and hyperviscosity syndrome are added to CRAB and referred to as "CRAB, etc.". The pharmaceutical compositions of the present invention are also pharmaceutical compositions for improving CRAB, etc.

The compounds can also decrease CD138, a known marker for multiple myeloma, and increase CD20, a known marker for lymphoma. In other words, these compounds can return the myeloma back to a B-cell-like state. This is called "transformation to B-cell-like". Anti-human CD20 monoclonal antibody drugs such as rituximab, obinutuzumab, ofatumumab, and ibritumomab tiuxetan are known as specific agents for cancer of cells expressing CD20 (malignant lymphomas).

In other words, the mixture of the compounds and anti-human CD20 monoclonal antibody drugs such as rituximab, obinutuzumab, ofatumumab, ibritumomab tiuxetan, which are known as specific drugs for lymphoma, can be a pharmaceutical composition to improve multiple myeloma (plasmacytoma).

The compounds of the present invention can also be used as active ingredients to provide transformation inducing agents that transform multiple myeloma cells to B cell-like cells.

Since the compounds of the present invention inhibit the production of IL-6 and MIP-la, which are involved in multiple myeloma autocrine-induced growth promotion, they can be pharmaceutical compositions that inhibit the growth of multiple myeloma. The compounds can also be used as an inhibitor of IL-6 or MIP-la because it inhibits IL-6 and MIP-la.

It is also possible to provide the compounds of the present invention as processed foods or agents. In other words, the compounds of the present invention, when ingested as processed foods or agents, have the same effect as the pharmaceutical compositions for improvement of the present invention. The agent includes supplements and additives.

Processed foods or agents include not only general processed foods such as candy, gum, jelly, biscuits, cookies, rice crackers, bread, noodles, fish and meat paste products, tea, soft drinks, coffee drinks, milk beverages, lactic acid bacteria beverages, yogurt, ice cream, pudding, and so on, but also foods with health claims, such as food for specified health uses and food with nutrient function claims, as defined by the Ministry of Health, Labour and Welfare's Food with Health Claims System, are included. In addition, nutritional supplements, feed, food additives, etc. are also included in processed foods or agents.

These processed foods or agents can be prepared by adding the compounds for improving malignant tumor disease to the ingredients of the processed foods or agents. The addition of the compounds to the processed foods or agents may be said to be the addition of the compounds as an active ingredients.

The processed foods or agents may also be said to be a processed foods or agents with a label stating that it is for the amelioration of malignant tumors such as multiple myeloma, lymphocytic leukemia, malignant lymphoma (MALT lymphoma, DLBCL, mantle cell lymphoma, burkitt lymphoma, hodgkin lymphoma, adult T-cell leukemia, peripheral T lymphoma, etc.), bortezomib-resistant multiple myeloma, and rituximab-resistant malignant lymphoma, pancreatic cancer, breast cancer, malignant melanoma, lung cancer, liver cancer, stomach cancer, colon cancer, head and neck tumor, glioma, renal cancer, ovarian cancer, and cervical cancer.

In addition, the processed foods or agents may be said to be a processed foods or agents with a label stating that it is for the improvement of CRAB (kidney failure, amyloidosis, hyperviscosity syndrome, bone lesions (bone destruction), hypercalcemia associated with bone lesions, pathological fractures, spinal cord compression fractures, spinal cord compression symptoms associated with spinal cord compression fractures, neurological symptoms associated with spinal cord compression symptoms.

The compounds of the invention may also be used as compositions for cell growth inhibition, since they inhibit cell growth of malignant tumors such as multiple myeloma, lymphoid leukemia, and malignant lymphomas (MALT lymphoma, DLBCL, mantle cell lymphoma, burkitt lymphoma, hodgkin lymphoma, adult T-cell leukemia, peripheral T lymphoma, etc.), bortezomib-resistant multiple myeloma, rituximab-resistant malignant lymphoma, pancreatic cancer, breast cancer, malignant melanoma, lung cancer, liver cancer, gastric cancer, colon cancer, head and neck tumor, gliomas, renal cancer, ovarian cancer and cervical cancer.

### [Example]

The synthesis of the compounds shown in formulas (1) through (12) is described below. The explanation of the synthesis is in the following order: (4) Formula (M7), (6) Formula (M11), (2) Formula (yk-8-1), (7) Formula (M12), (3) Formula (yk-8-3), (5) Formula (M9), (8) Formula (M14), (9) Formula (M15), (10) Formula (M15), (11) Formula (M14), (12) Formula (M14), (13) Formula (M14), (14) Formula (M14)), (1) formula (yk-7), (9) formula (M15), (10) formula (M16), (11) formula (M18), and (12) formula (M19).

### < Synthesis of (4) formula (M7) >

Mangiferin (4.21 g, 10.0 mmol), propionic anhydride (19.2 mL, 149 mmol), DMAP (122 mg, 1 mmol) and dry pyridine (60 mL) were heated at 100 °C for 24 h. The reaction solution was poured into ice water (600 mL) and extracted with ethyl acetate. The ethyl acetate layer was washed sequentially with ice-cold 10% sulfuric acid, saturated sodium bicarbonate solution, and saturated brine, dried over anhydrous sodium sulfate, filtered, and concentrated. The residue was purified using column chromatography (n-hexane/ethyl acetate = 1/1) to afford the title compound (7.19 g, 83%) as a colorless solid.

The NMR spectrum is shown below.

IR (KBr): 1774, 1755, 1667, 1620, 1458, 1354, 1273, 1157, 1114, 1083 cm⁻¹. ¹H NMR (800 MHz, DMSO-d₆, 25 °C) δ: 0.71- 0.77/0.93-0.97 (each 3H, m, COCH₂CH₃), 0.98-1.02/1.12-1.16/1.19-1.28 (each 6H, m, COCH₂CH₃), 1.92-2.01 (2H, m, COCH₂CH₃), 2.17-2.32 (6H, m, COCH₂CH₃), 2.60-2.94 (8H, m, COCH₂CH₃), 3.86-3.92 (1H, m, H-6'a), 4.20-4.30 (2H, m, H-5' and H-6'b), 501-5.06 (1H, m, H-4'), 5.10-5.20 (1H, m, H-1'), 5.45-5.53 (2H, m, H-2' and H-3'), 7.51/7.53 (1H, each s, H-4), 7.68/7.69 (1H, each s, H-5), 7.97/7.98 (1H, each s, H-8). ¹³C NMR (200 MHz, DMSO-d₆ 25 °C) δ: 8.64/8.75/8.77/8.91/8.94/8.96/9.00/9.13/9.16 (COCH₂CH₃), 26.58/26.63/26.7/26.8/26.89/26.94/27 0/27.1/27.32/27.34 (COCH₂CH₃), 62.1/62.2 (C6'), 68.18/68.20 (C4'), 69.6/70.0 (C2'), 70.8/71.3 (C1'), 73.5/73.6 (C3'), 75.0/75.1 (C5'), 110.2/111.8 (C4), 111.6/112.7 (C9a), 113.2/113.3 (C5), 118.8/118.9 (C2), 119.6/119.7 (C8a), 120.3/120.4 (C8), 139.5/139.6 (C7), 147.9 (C6), 149.1/150.9 (C1), 152.47/152.51 (C8b), 153.4/154.8 (C4a), 156.5/156.8 (C3), 170.9/171.04/171.06/171.09/171.67/171.72/171.9/172.4/172.6/172.7/173.18/173.22/173.51 /173.54 (COCH₂CH₃), 173.1 (C9). HRMS (ESI) m/z: [M+Na]⁺ Calcd for C₄₃H₅₀O₁₉Na 893.2839; Found 893.2853.

### < Synthesis of (6) formula (M11) >

M11 was synthesized according to the method for M7, except that propionic anhydride was replaced with butyric anhydride. The yield was 81%.

The NMR spectrum is shown below.

Colorless solid: IR (KBr): 1775, 1751, 1665, 1618, 1458, 1157, 1092 cm⁻¹. ¹H NMR (800 MHz, DMSO-d₆, 25 °C) δ: 0.52-1.11 (24H, m, COCH₂CH₂CH₃), 1.20-1.97 (16H, m, COCH₂CH₂CH₃), 2.14-2.88 (16H, m, COCH₂CH₂CH₃), 3.88-3.94 (1H, m, H -6'a), 4.16-4.25 (2H, m, H-5' and H-6'b), 5.02-5.06 (1H, m, H-4'), 5.06-5.12 (1H, m, H-1'), 5.45-5.52 (1H, m, H-3'), 5.54-5.65 (1H, m, H-2'), 7.50/7.53 (1H, each s, H-4), 7.68/7.69 (1H, each s, H-5), 7.95/7.96 (1H, each s, H-8). ¹³C NMR (200 MHz, DMSO-d₆, 25 °C) δ: 13.1/13.41/13.45/13.48/13.51/13.58/13.64/13.8 (COCH₂CH₂CH₃), 17.5/17.62/17.66/17.72/17.76/17.83/17.88/17.90 (COCH₂CH₂CH₃), 34.9/35.09/35.15/35.17/35.19/35.31/35.33/35.4/35.68/35.73 (COCH₂CH₂CH₃), 62.0/62.2 (C6'), 68.2/68.3 (C4'), 69.3/69.8 (C2'), 70.9/71.3 (C1'), 73.36/73.43 (C3'), 75.1/75.2 (C5'), 110.2/111.6 (C4), 111.6/112.6 (C9a), 113.3/113.4 (C5), 118.7/118.8 (C2), 119.6/119.7 (C8a), 120.35/120.40 (C8), 139.5 (C7), 147.82/147.85 (C6), 149.1/150.8 (C1), 152.46/152.49 (C8b), 153.4/154.7 (C4a), 156.6/156.8 (C3), 169.9/170.14/170.17/170.18/170.7/170.8/171.0/171.4/171.6/171.8/172.0/172.1/172.5/172.6 (COCH₂CH₃), 173.1 (C9).HRMS (ESI) m/z: [M+Na]⁺ Calcd for C₅₁H₆₇O₁₉Na 1005.4091; Found 1005.4092.

### < Synthesis of (2) formula (yk-8-1) >

yk-8-1 was synthesized according to the method for M7, except that propionic anhydride was replaced by herbaceous anhydride. The yield was 83%.

The NMR spectrum is shown below.

Colorless solids: IR (KBr): 1771, 1747, 1668, 1614, 1456, 1157, 1092 cm⁻¹. ¹H NMR (800 MHz, DMSO-d₆, 25 °C) δ: 0.52-1.82 (56H, m, COCH₂CH₂CH₂CH₃), 1.88-2.30/2.56-2.94 (16H, each m, COCH₂CH₂CH₂CH₃), 3.89-3.95 (1H, m, H-6 'a), 4.13-4.23 (2H, m, H-5' and H-6'b), 5.02-5.06 (1H, m, H-4'), 5.06-5.12 (1H, m, H-1'), 5.44-5.52 (1H, m, H-3'), 5.56-5.65 (1H, m, H-2'), 7.49/7.53 (1H, each s, H-4), 7.67/7.68 (1H, each s, H-5), 7.95/7.96 (1H, each s, H-8). ¹³C NMR (200 MHz, DMSO-d₆, 25 °C) δ: 13.4/13.59/13.64/13.7/13.77/13.80/13.9 (COCH₂CH₂CH₂CH₃), 21.4/21.66/21.68/21.72/21.74/21.86/21.88/22.0 (COCH₂CH₂CH₂CH₃), 26.1/26.25/26.29/26.36/26.45/26.47/26.51/26.52 (COCH₂CH₂CH₂CH₃), 32.8/33.0/33.09/33.14/33.21/33.23/33.7/33.8 (COCH₂CH₂CH₂CH₃), 62.1/62.3 (C6'), 68.29/68.32 (C4'), 69.3/69.8 (C2'), 70.8/71.3 (C1'), 73.4/73.5 (C3'), 75.1/75.2 (C5'), 110.2/111.6 (C4), 111.6/112.6 (C9a), 113.28/113.31 (C5), 118.7/118.8 (C2), 119.6/119.7 (C8a), 120.3/120.4 (C8), 139.5 (C7), 147.82/147.85 (C6), 149.1/150.9 (C1), 152.43/152.47 (C8b), 153.4/154.7 (C4a), 156.6/156.8 (C3), 169.9/170.1/170.3/170.86/170.94/171.1/171.5/171.7/171.9/172.12/172.14/172.66/172.69 (COCH₂CH₃), 173.1 (C9). HRMS (ESI) m/z: [M+Na]⁺ Calcd for C₅₉H₈₂O₁₉Na 1117.5343; Found 117.5344.

### < Synthesis of (7) formula (M12) >

A mixture of 1,3,2',3',4',6,6',7-octa-O-butyrylmangiferin (M11) (5.06 g, 5.68 mmol), ammonium acetate (6.7 g, 87.0 mmol), methanol (160 mL) and water (20 mL) was stirred at room temperature for 6 hours. Methanol was removed from the reaction solution by concentrating under reduced pressure, and the residue was diluted with ethyl acetate (100mL). The mixture was washed sequentially with water and saturated brine, dried over anhydrous sodium sulfate, filtered, and concentrated. The residue was purified by column chromatography [CHCl₃/CH₃OH (20:1)] to afford the title compound (3.89 g 93%) as a pale yellow solid.

The NMR spectrum is shown below.

IR (KBr): 3385, 1751, 1624, 1458, 1288, 1188, 1099 cm⁻¹. ¹H NMR (800 MHz, DMSO-d₆, 25 °C) δ: 0.48-1.09 (15H, m, COCH₂CH₂CH₃), 1.18-1.83 (10H, m, COCH₂CH₂CH₃), 1.89-2.86 (10H, m, COCH₂CH₂CH₃), 3.87 (0.5H, dd-like, J = ca. 12.6, 1.6, H-6a'), 4.02-4.11 (2H, br m, H-5, H-5', H-6a', H-6b'), 4.16 (0.5H, dd, J = 12.6, 4.8, H-6b'), 4.90 (0.5H, d, J = 9.6, H-1'), 5.01 (0.5H, dd, J = 9.6, 9.6, H-4'), 5.02 (0.5H, dd, J = 9.6, 9.6, H-4'), 5.15 (0.5H, d, J = 10.4, H-1'), 5.35 (0.5H, dd, J = 9.6, 9.6, H-3'), 5.37 (0.5H, br dd-like, J = ca. 9.6, 9.6, H-3'), 5.54 (0.5H, dd, J = 10.4, 9.6, H-2'), 5.88 (0.5H, br dd-like, J = ca. 9.6, 9.6, H-2'), 6.72/6.74 (each 0.5H, s, H-4), 6.797/6.803 (each 0.5H, s, H -5), 7.29/7.30 (each 0.5H, s, H-8), 9.67/10.5/11.2 (each 1H, br s, OH). ¹³C NMR (200 MHz, DMSO-d₆, 25 °C) δ: 13.1/13.4/13.50/13.54/13.6/13.7/13.9 (COCH₂CH₂CH₃), 17.7/17.90/17.93/17.96/18.02 (COCH₂CH₂CH₃), 35.1/35.2/35.3/35.37/35.41/35.46/35.50/35.54/35.9 (COCH₂CH₂CH₃), 62.0/62.4 (C-6'), 68.2/68.4 (C-4'), 68.5/70.3 (C-2'), 71.1/71.2 (C-1'), 73.7/74.0 (C-3'), 74.9/75.3 (C-5'), 99.7/100.8 (C-4), 102.52/102.54 (C-5), 106.6/107.8 (C-9a), 109.1 (C-8), 113.2/113.4 (C-2), 114.06/114.08 (C-8a), 143.9 (C-7), 149.8/149.9 (C-6, C-1), 151.3 (C-1), 153.3 (C-8b), 157.6/157.7 (C-4a), 160.9/162.3 (C-3), 170.3/171.1/171.2/171.5/171.9/172.2/172.6 (COCH₂CH₃), 172.7 (C-9).HRMS (ESI) m/z: [M-H]⁻ Calcd for C₃₉H₄₇O₁₆ 771.2859; Found 771.2858.

### < Synthesis of (3) formula (yk-8-3) >

In the synthetic method of M12, 1,3,2',3',4',6,6',7-octa-O-butyrylmangiferin (M11) was replaced by 1,3,2',3',4',6,6',7-octa-O-valerylmangiferin (yk-8-1) was used as the starting material, and the synthesis of yk-8-3 was carried out according to the synthetic method of M12. The yield was 90%.

The NMR spectrum is shown below.

Pale brown solid: IR (KBr): 3399, 1751, 1618, 1458, 1290, 1167, 1097 cm⁻¹. ¹H NMR (800 MHz, DMSO-d₆, 25 °C) δ: 0.56-1.82 (35H, m, COCH₂CH₂CH₂CH₃), 2.12-2.32 (6H, m, COCH₂CH₂CH₂CH₃), 2.58-2.87 (4H, m, COCH₂CH₂CH₂CH₃), 3.88 (0.5H, dd-like, J = ca. 12.6, 1.6, H-6a'), 4.02-4.10 (2H, br m, H-5, H-5', H -6a', H-6b'), 4.12 (0.5H, dd, J = 12.6, 5.0, H-6b'), 4.90 (0.5H, d, J = 9.9, H-1'), 5.00 (0.5H, dd, J = 9.6, 9.6, H-4'), 5.03 (0.5H, dd, J = 9.7, 9.7, H-4'), 5.15 (0.5H, d, J = 10.0, H-1'), 5.35 (0.5H, dd, J = 9.6, 9.6, H-3'), 5.37 (0.5H, br dd-like, J = ca. 9.7, 9.7, H-3'), 5.57 (0.5H, dd, J = 10.0, 9.6, H-2'), 5.87 (0.5H, br dd-like, J = ca. 9.9, 9.7, H-2'), 6.71/6.75 (each 0.5H, s, H-4), 6.796/6.804 (each 0.5H, s, H -5), 7.29/7.30 (each 0.5H, s, H-8), 9.61/10.5 (each 1H, br s, OH), 11.2/11.4 (each 0.5H, br s, OH). ¹³C NMR (200 MHz, DMSO-d₆, 25 °C) δ: 13.5/13.62/13.67/13.69/13.9 (COCH₂CH₂CH₂CH₃), 21.3/21.4/21.68/21.69/21.7/21.9/22.1 (COCH₂CH₂CH₂CH₃), 26.3/26.45/26.48/26.50/26.52/26.55/26.57 (COCH₂CH₂CH₂CH₃), 33.0/33.1/33.16/33.21/33.27/33.34/33.9 (COCH₂CH₂CH₂CH₃), 62.1/62.4 (C-6'), 68.3/68.4 (C-4'), 68.4/70.2 (C-2'), 71.0/71.2 (C-1'), 73.7/74.0 (C-3'), 74.9/75.2 (C-5'), 99.6/100.8 (C-4), 102.46/102.49 (C-5), 106.65/107.71 (C-9a), 109.1 (C-8), 113.1/113.2 (C-2), 114.0 (C-8a), 143.8 (C-7), 149.77/149.80 (C-6, C-1), 151.3 (C-1), 153.22/153.24 (C-8b), 157.6/157.7 (C-4a), 160.8/162.2 (C-3), 170.2/171.1/171.3/171.6/171.97/172.00/172.2/172.69/172.73 (COCH₂CH₃), 172.6 (C-9). HRMS (ESI) m/z: [M-H]⁻ Calcd for C₄₄H₅₇O₁₆ 841.3641; Found 841.3640.

### < Synthesis of (5) formula (M9) >

A mixture of 1,2',3',4',6-penta-O-propionylmangiferin (M8) (3.88 g, 5.52 mmol), N,N-dimethyltrimethylenediamine (3.47 mL, 27.6 mmol) and DMSO (40 mL) were stirred at room temperature for 1 hour. The reaction solution was poured into ice water (500 mL) and extracted with a mixture of diethyl ether and hexane (3/1). The organic layer was washed with saturated brine, dried over anhydrous sodium sulfate, filtered, and concentrated. The residue was purified by column chromatography [CHCl₃/CH₃OH (20:1)] to afford the title compound (3.08 g, 86%) as a pale yellow solid.

The NMR spectrum is shown below.

IR (KBr): 3399, 1751, 1618, 1475, 1292, 1190, 1084 cm⁻¹. ¹H NMR (800 MHz, DMSO-d₆, 60 °C) δ: 0.74/0.97/1.02/1.03 (each 3H, t, J= 7.6, COCH₂CH₃), 1.95/1.98 (each 1H, dq, J = 16.5, 7.6, COCH₂CH₃), 2.18 (2H, q, J = 7.6, COCH₂CH₃), 2.22-2.34 (4H, m, COCH₂CH₃), 3.99 (1H, ddd-like, J = 9.8, 4.5, 2.5, H-5'), 4.10 (1H, dd, J = 12.5, 2.5, H-6a'), 4.12 (1H, dd, J = 12.5, 4.5, H-6b'), 5.06 (1H, dd, J = 9.8, 9.8, H-4'), 5.10 (1H, d, J = 10.0, H-1'), 5.31 (1H, dd, J = 9.8, 9.5, H-3'), 5.85 (1H, br dd, J = 10.0, 9.5, H-2'), 6.35 (1H, s, H-4), 6.85 (1H, s, H-4), 7.34 (1H, s, H-8), 9.0-11.0 (3H, br, OH), 13.9 (1H, s, OH). ¹³C NMR (200 MHz, DMSO-d₆, 60 °C) δ: 8.71/8.79/8.82 (COCH₂CH₃), 26.72/26.73/26.77/26.83 (COCH₂CH₃), 62.0 (C-6'), 68.3 (C-4'), 69.1 (C-2'), 70.4 (C-1'), 74.2 (C-3'), 75.0 (C-5'), 93.3 (C-4), 101.0 (C-9a), 102.6 (C-5), 104.2 (C-2), 108.2 (C-8), 111.7 (C-8a), 143.8 (C-7), 150.8 (C-6), 154.2 (C-8b), 156.8 (C-4a), 161.9 (C-1), 163.6 (C-3), 171.9/172.5/172.7/173.1 (COCH₂CH₃), 179.0 (C-9). HRMS (ESI) m/z: [M-H]⁻ Calcd for C₃₁H₃₃O₁₅ 645.1814; Found 645.1817.

### < Synthesis of (8) formula (M14) >

In the synthetic method of M9, 1,3,2',3',4',6,6',7-octa-O-propionylmangiferin (M7) was replaced by 1,2',3',4',6-penta-O-butyrylmangiferin (M12) was used as the starting material, and the synthesis of M14 was carried out according to the synthetic method of M9. The yield was 90%.

The NMR spectrum is shown below.

Pale yellow solid: IR (KBr): 3399, 1751, 1618, 1474, 1292, 1188, 1085 cm⁻¹. ¹H NMR (800 MHz, DMSO-d₆, 60 °C) δ: 0.50 (3H, br, t-like, J = ca. 7.0, COCH₂CH₂CH₃), 0.83/0.871/0.872 (each 3H, t, J = 7.4, COCH₂CH₂CH₃), 1.18-1.30/1.44-1.49 (each 2H, m, COCH₂CH₂CH₃), 1.50-1.56 (6H, m, COCH₂CH₂CH₃), 1.93 (2H, t, J = 7.0, COCH₂CH₂CH₃), 2.15 (2H, t, J = 7.2, COCH₂CH₂CH₃), 2.22-2.31 (4H, m, COCH₂CH₂CH₃), 3.98 (1H, ddd-like, J = 9.8, 4.6, 2.5, H-5'), 4.09 (1H, dd, J = 12.5, 4.6, H-6a'), 4.11 (1H, dd, J = 12.5, 2.5, H-6b'), 5.06 (1H, dd, J = 9.8, 9.8, H-4'), 5.09 (1H, d, J = 10.0, H-1'), 5.31 (1H, dd, J = 9.8, 9.5, H-3'), 5.85 (1H, br dd, J = 10.0, 9.5, H-2'), 6.34 (1H, s, H-4), 6.85 (1H, s, H-4), 7.38 (1H, s, H-8), 8.9-11.5 (3H, br, OH), 13.9 (1H, s, OH). ¹³C NMR (200 MHz, DMSO-d₆, 60 °C) δ: 12.6/13.13/13.16/13.22 (COCH₂CH₂CH₃), 17.62/17.64/17.66 (COCH₂CH₂CH₃), 35.16/35.23/35.26/35.31 (COCH₂CH₂CH₃), 61.9 (C-6'), 68.3 (C-4'), 69.0 (C-2'), 70.4 (C-1'), 74.1 (C-9a), 102.6 (C-5), 104.2 (C-2), 108.2 (C-8), 111.7 (C-8a), 143.8 (C-7), 150.8 (C-6), 154.2 (C-8b), 156.8 (C-4a), 162.1 (C-1), 163.7 (C-3), 170.9/171.6/171.7/172.3 (COCH₂CH₃), 179.0 (C-9). HRMS (ESI) m/z: [M-H]⁻ Calcd for C₃₅H₄₁O₁₅ 701.2440; Found 701.2440.

### < Synthesis of (1) formula (yk-7) >

In the synthetic method of M9, 1,3,2',3',4',6,6',7-octa-O-propionylmangiferin (M7) was replaced by 1,2',3',4',6-penta-O-valerylmangiferin (yk-8-3) was used as the starting material, and the synthesis of yk-7 was carried out according to the synthetic method of M9. The yield was 82%.

The NMR spectrum is shown below.

Pale yellow solid: IR (KBr): 3393, 1751, 1618, 1474, 1292, 1184, 1091 cm⁻¹. ¹H NMR (800 MHz, DMSO-d₆, 60 °C) δ: 0.54 (3H, br s-like, COCH₂CH₂CH₂CH₃), 0.82/0.84/0.86 (each 3H, t, J = 7.4, COCH₂CH₂CH₂CH₃), 0.85-0.94 (2H, m, COCH₂CH₂CH₂CH₃), 1.10-1.52 (14H, m, C OCH₂CH₂CH₂CH₃), 1.95 (2H, t, J = 7.2, COCH₂CH₂CH₂CH₃), 2.16 (2H, t, J = 7.3, COCH₂CH₂CH₂CH₃), 2.21-2.32 (4H, m, COCH₂CH₂CH₂CH₃), 3.98 (1H, ddd-like, J= 9.8, 4.5, 2.5, H-5'), 4.08 (1H, dd, J = 12.5, 4.5, H-6a'), 4.10 (1H, dd, J = 12.5, 2.5, H-6b'), 5.05 (1H, dd, J= 9.8, 9.8, H-4'), 5.08 (1H, d, J= 10.0, H-1'), 5.31 (1H, dd, J = 9.8, 9.5, H-3'), 5.85 (1H, br dd, J = 10.0, 9.5, H-2'), 6.34 (1H, s, H-4), 6.85 (1H, s, H-4), 7.38 (1H, s, H-8), 9.0-11.5 (3H, br, OH), 13.9 (1H, s, OH). ¹³C NMR (200 MHz, DMSO-d₆, 60 °C) δ: 13.0/13.23/13.28/13.31 (COCH₂CH₂CH₂CH₃), 21.0/21.37/21.40 (COCH₂CH₂CH₂CH₃), 26.27/26.30/26.34 (COCH₂CH₂CH₂CH₃), 33.00/33.02/33.03/33.08 (COCH₂CH₂CH₂CH₃), 62.0 (C-6'), 68.4 (C-4'), 68.9(C-2'), 70.4 (C-1'), 74.1 (C-3'), 75.0 (C-5'), 93.3 (C-4), 101.0 (C-9a), 102.6 (C-5), 104.2 (C-2), 108.1 (C-8), 111.7 (C-8a), 143.8 (C-7), 150.8 (C-6), 154.1 (C-8b), 156.8 (C-4a), 161.7 (C-1), 163.7 (C-3), 171.0/171.7/171.9/172.4 (COCH₂CH₃), 179.0 (C-9). HRMS (ESI) m/z: [M-H]⁻ Calcd for C₃₉H₄₉O₁₅ 757.3066; Found 757.3060.

### < Synthesis of (9) formula (M15) >

Mangiferin (500 mg, 1.18 mmol), anhydrous pival acetic acid (4.79 mL, 23.7 mmol), 4-dimethylaminopyridine (15 mg, 0.12 mmol) and dry pyridine (10 mL) were heated at 100 °C for 2 weeks. The reaction solution was poured into ice water (50 mL) and extracted with ethyl acetate. The ethyl acetate layer was washed sequentially with ice-cold 10% sulfuric acid, saturated sodium bicarbonate solution, and saturated brine. The washed ethyl acetate layer was dried with anhydrous sodium sulfate, filtered, and concentrated to obtain a pale yellow oily substance (1.65 g). A mixture of the resulting oily substance, N,N-dimethyltrimethylenediamine (1.03 mL, 8.27 mmol) and dimethyl sulfoxide (30 mL) was stirred at 50 °C for 2 h. The reaction solution was poured into ice water (50 mL) and extracted with a mixture of diethyl ether and hexane (3/1). The organic layer was washed with saturated brine, dried over anhydrous sodium sulfate, filtered, and concentrated. The residue was purified by column chromatography (CHCl₃/MeOH) to afford the title compound (948 mg, 1.25 mmol, 94%) as a pale yellow solid.

The NMR spectrum is shown below.

Pale yellow solid: IR (KBr): 3387, 2974, 1746, 1651, 1618, 1481, 1290, 1173, 1144 cm⁻¹. ¹H NMR (800 MHz, DMSO-d₆, 60 °C) δ: 0.81/1.05/1.11/1.16 (each 9H, s, COCH(CH₃)₃), 3.99-4.04 (2H, br-m, H-5' andH-6'a), 4.16 (1H, d, J = 12.0 Hz, H-6'b), 5.10 (1H, d, J = 10.4 Hz, H-1'), 5.17 (1H, t, J = 9.6 Hz, H-4'), 5.33 (1H, t, J = 9.6 Hz, H-3'), 5.85 (1H, br, H-2'), 6.35 (1H, s, H-4), 6.84 (1H, s, H-5), 7.38 (1H, s, H-8). ¹³C NMR (200 MHz, DMSO-d₆, 60 °C) δ: 26.9/27.2/27.32/27.36 [COCH(CH₃)₃], 38.5/38.70/38.79/38.9 (COCH(CH₃)₃), 62.0 (C-6'), 68.2 (C-4'), 69.5 (C-2'), 71.1 (C-1'), 74.7 (C-3'), 75.9 (C-5'), 94.2 (C-4), 103.3 (C-5), 104.8 (C-9a), 108.8 (C-8), 112.3 (C-2), 114.5 (C-8a), 144.5 (C-7), 151.4 (C-6), 153.6 (C-1), 154.8 (C-8b), 157.4 (C-4a), 162.5 (C-3), 176.2/176.3/176.9/177.5 [COCH(CH₃)₃], HRMS (ESI) m/z: [M-H]⁻ Calcd for C₃₉H₄₉O₁₅ 757.3066; Found 757.3037.

### < Synthesis of (10) formula (M16) >

2',3'-di-O-butylmangiferin (540 mg, 1.01 mmol), butyric anhydride (2.48 mL, 15.2 mmol), 4- dimethylaminopyridine (12 mg, 0.10 mmol) and dry pyridine (20 mL) were stirred at 80 °C for 15 h. The reaction solution was poured into ice water and extracted with ethyl acetate. The ethyl acetate layer was washed sequentially with ice-cold 10% sulfuric acid, saturated sodium bicarbonate solution, and saturated brine. The washed ethyl acetate layer was dried with anhydrous sodium sulfate, filtered, and concentrated to yield a pale yellow liquid (1.09 g). The resulting pale yellow liquid, N,N-dimethyltrimethylenediamine (0.63 mL, 5.05 mmol) and dimethyl sulfoxide (10 mL) were stirred at 50 °C for 3 h. The reaction solution was poured into ice water (50 mL) and extracted with a mixture of diethyl ether and hexane (3/1). The organic layer was washed with saturated brine, dried over anhydrous sodium sulfate, filtered, and concentrated. The residue was purified using column chromatography (CHCl₃/MeOH) to afford the title compound (589 mg, 0.87 mmol, 86%) as a pale yellow solid.

The NMR spectrum is shown below.

Pale yellow solid: IR (KBr): 3354, 2960, 2934, 2874, 1748, 1616, 1476, 1293, 1188, 1084 cm⁻¹. ¹H NMR (800 MHz, CDCl₃) δ: 0.57/0.95 (each 3H, t, J = 7.6 Hz, COCH₂CH₂CH₃), 0.87/0.98 (each 3H, t, J = 7.3 Hz, OCH₂CH₂CH₂CH₃), 1.10/1.18/1.19/1.26 (each 1H, sext, J = 7.3 Hz, OCH₂CH₂CH₂CH₃), 1.27-1.34/2.14-1.53 (each 2H, m, OCH₂CH₂CH₂CH₃), 1.68 (4H, sext, J = 7.6 Hz, COCH₂CH₂CH₃×2), 2.34/2.37 (each 2H, t, J = 7.6 Hz, COCH₂CH₂CH₃), 3.05/3.46/3.58/3.80 (each 1H, td, J = 6.1, 9.2 Hz, OCH₂CH₂CH₂CH₃), 3.56 (1H, t, J = 9.2 Hz, H-2'), 3.62 (1H, br, H-4'), 3.82 (1H, td, J= 9.2, 4.0 Hz, H-5'), 4.23/4.26 (each 1H, dd, J = 12.8, 4.0 Hz, H-6'), 5.09 (1H, d, J = 9.2 Hz, H-1'), 5.16 (1H, t, J = 9.2 Hz, H-3'), 6.39 (1H, s, H -4), 6.81 (1H, s, H-5), 7.59 (1H, s, H-8), 7.28/7.94/8.38/13.4 (each 1H, s, OH). ¹³C NMR (200 MHz, CDCl₃) δ: 13.56/13.61 cm⁻¹; (COCH₂CH₂CH₃), 13.67/13.89 (OCH₂CH₂CH₂CH₃), 18.2/18.3 (COCH₂CH₂CH₃), 18.8/19.2 (OCH₂CH₂CH₂CH₃), 31.9/32.4 (OCH₂CH₂CH₂CH₃), 35.9/36.1 (COCH₂CH₂CH₃), 61.9 (C-6'), 69.2 (C-3'), 73.1/73.5 (OCH₂CH₂CH₂CH₃), 74.3 (C-1'), 76.6 (C-5'), 81.1 (C-4'), 83.4 (C-2'), 95.5 (C-4), 102.2 (C-9a), 102.8 (C-5), 105.2 (C-2), 108.3 (C-8), 113.0 (C-8a), 141.8 (C-7), 152.2 (C-8b), 152.7 (C-6), 157.5 (C-4a), 160.5 (C-1), 163.3 (C-3), 172.3/173.7 (COCH₂CH₂CH₃), 179.9 (C-9). HRMS (ESI) m/z: [M+Na]⁺ Calcd for C₃₅H₄₆O₁₃Na 697.2831; Found 697.2835.

### < Synthesis of (11) formula (M18) >

A mixture of 1-(2-hydroxyethoxy)-3,6,7-tris(methoxymethoxy)-9H-xanthen-9-one (100 mg, 0.23 mmol), 10% hydrochloric acid (0.5 mL) and methanol (5.0 mL) was heated at 80°C for 5 h. The precipitate in the reaction solution was filtered and washed with methanol to afford the title compound (60.7 mg, 0.20 mmol, 87%) as a pale yellow solid.

The NMR spectrum is shown below.

Pale yellow solid: IR (KBr): 3470, 3205, 3066, 2978, 2878, 1777, 1697, 1562, 1404, 1354, 1298, 1238, 1122 cm⁻¹. ¹H NMR (800 MHz, DMSO-d₆) δ: 3.75 (2H, t, J = 5.2 Hz, OCH₂CH₂OH), 4.05 (2H, t, J = 5.2 Hz, OCH₂CH₂OH), 6.35 (1H, d, 2.2 Hz, H-2), 6.38 (1H, d, 2.2 Hz, H-4), 6.75 (1H, s, H-5), 7.34 (1H, s, H-8), 9.50/10.26/10.65 (each 1H, s, phenolic OH). ¹³C NMR (200 MHz, DMSO-d₆) δ: 59.8 (OCH₂CH₂OH), 71.5 (OCH₂CH₂OH), 95.7 (C-4), 97.9 (C-2), 102.5 (C-5), 105.8 (C-9a), 109.6 (C-8), 115.2 (C-8a), 143.6 (C-7), 149.7 (C-8b), 152.7 (C-6), 159.3 (C-4a), 161.4 (C-1), 163.1 (C-3), 173.5 (C-9). HRMS (ESI) m/z: [M-H]⁻ Calcd for C₁₅H₁₂O₇ 304.0583; Found 304.0579.

### < Synthesis of (12) formula (M19) >

1-Hydroxy-3,6,7-tris(methoxymethoxy)-9H-xanthen-9-one (70 mg 0.18 mmol), allyl bromide (46 µL, 0.54 mmol), cesium carbonate (175 mg, 0.54 mmol) and N,N-dimethylformamide (5.0 mL) were stirred for 1 h at room temperature. The reaction solution was poured into ice water (10 mL) and extracted with diethyl ether. The diethyl ether layer was washed with saturated brine and dried over anhydrous sodium sulfate. To the pale yellow solid (78 mg) obtained by concentrating the dried filtrate was added concentrated hydrochloric acid (0.05 mL) and methanol (1.5 mL), and the mixture was stirred at 50°C for 3 h. The reaction solution was poured into ice water (5 mL) and extracted with a mixture of dichloromethane/methanol (5/1). The organic layer was washed with saturated brine and dried with anhydrous sodium sulfate. The yellow solid obtained by concentrating the dried filtrate was recrystallized from hexane/ethyl acetate (20/1) to afford the title compound (30 mg, 0.10 mmol, 56%) as a pale yellow solid.

The NMR spectrum is shown below.

Pale yellow solid: Melting point 207-208°C; IR (KBr): 3410, 3275, 1608, 1562, 1493, 1464, 1282, 1149, 1092 cm⁻¹. ¹H NMR (800 MHz, CDsOD) δ: 4.67 (2H, td, J = 4.8, 1.8 Hz, OCH₂CH=CH₂), 5.30 (1H, ddd, J = 10.5, 3.2, 1.8 Hz, OCH₂CH=CH₂), 5.57 (1H, ddd, J = 17.2, 3.4, 1.8 Hz, OCH₂CH=CH₂), 6.12 (1H, tdd, J = 17.2, 10.5, 4.8 Hz, OCH₂CH=CH₂), 6.33 (1H, d, J = 2.1 Hz, H-2), 6.38 (1H, d, J = 2.1 Hz, H-4), 6.76 (1H, s), 7.47 (1H, s). ¹³C NMR (200 MHz, CDsOD) δ: 69.4 (OCH₂CH=CH₂), 95.0 (C-4), 96.3 (C-2), 101.7 (C-5), 105.4 (C-9a), 108.9 (C-8), 115.0 (C-8a), 116.5 (OCH₂CH=CH₂), 132.8 (OCH₂CH=CH₂), 143.2 (C-7), 150.6 (C-8b), 152.8 (C-6), 159.9 (C-4a), 161.0 (C-1), 163.5 (C-3), 175.7 (C-9). HRMS (ESI) m/z: [M-H]⁻ Calcd for C₁₆H₁₁O₆ 300.0634; Found 300.0641.

### < Synthesis of (a) formula (mangiferin 8a) >

Mangiferin 8a (M8) was synthesized as a comparative example. Mangiferin 8a was synthesized as follows: A mixture of 1,3,2',3',4',6,6',7-octa-O-propionylmangiferin (M7) (5.06 g, 5.68 mmol), ammonium acetate (6.7 g, 87.0 mmol), methanol (160 mL) and water (20 mL) was stirred at room temperature for 6 h. Methanol was removed from the reaction solution by reduced pressure concentration, and the residue was diluted with ethyl acetate (100 mL). The mixture was washed sequentially with water and saturated brine, dried over anhydrous sodium sulfate, filtered, and concentrated. The residue was purified by column chromatography [CHCl₃/CH₃OH (20:1)] to afford the title compound (3.89 g, 95%) as a pale yellow solid. Mangiferin 8a is represented by formula (a).

The NMR spectrum is shown below.

IR (KBr): 3406, 1751, 1620, 1462, 1354, 1284, 1192, 1083 cm⁻¹. ¹H NMR (800 MHz, DMSO-d₆, 25 °C) δ: 0.74/0.94 (each 3H, t-like, J= 7.6, COCH₂CH₃), 0.97-1.02 (6H, m, COCH₂CH₃), 1.20/1.23 (each 1.5H, t-like) J = 7.6, COCH2CH3), 0.97-1.02 (6H, m, COCH2CH3), 1.20/1.23 (each 1.5H, t-like, J = 7.6, COCH₂CH₃), 1.92-2.02 (2H, m, COCH₂CH₃), 2.15-2.33 (6H, m, COCH₂CH₃), 2.61-2.86 (2H, m, COCH₂CH₃), 3.85 (0.5H, dd-like, J = ca. 12.5, 1.9, H-6a'), 4.05 (0.5H, br d-like, J = ca. 12.5, H-6a'), 4.07 (0.5H, ddd, J = 9.8, 4.7, 1.9, H-5'), 4.09-4.14 (0.5H, m, H-6b'), 4.11 (0.5H, br d-like, J = ca. 9.5, H-5'), 4.24 (0.5H, dd, J = 12.5, 4.7, H-6b'), 4.96 (0.5H, d, J = 9.9, H-1'), 5.00 (0.5H, dd, J = 9.5, 9.5, H-4'), 5.02 (0.5H, dd, J = 9.8, 9.8, H-4'), 5.16 (0.5H, d, J = 10.0, H-1'), 5.35 (0.5H, dd, J = 9.5, 9.5, H-3'), 5.40 (0.5H, dd, J = 9.8, 9.5, H-3'), 5.49 (0.5H, dd, J = 10.0, 9.5, H-2'), 5.84 (0.5H, dd, J = 9.9, 9.5, H-2'), 6.72/6.75 (each 0.5H, s, H-4), 6.80/6.81 (each 0.5H, s, H-5), 7.29/7.30 (each 0.5H, s, H-8), 9.62/10.5 (each 1H, br s, OH), 11.2/11.4 (each 0.5H, br s, OH). ¹³C NMR (200 MHz, DMSO-d₆, 25 °C) δ: 8.81/8.86/8.96/9.02/9.06/9.11/9.12/9.20 (COCH₂CH₃), 26.6/26.89/26.91/26.94/27.00/27.04/27.1/27.4 (COCH₂CH₃), 62.1/62.3 (C-6'), 68.2/68.3 (C-4'), 68.7/70.4 (C-2'), 71.0/71.1 (C-1'), 73.8/74.1 (C-3'), 74.6/75.2 (C-5'), 99.5/100.8 (C-4), 102.5 (C-5), 106.6/107.8 (C-9a), 109.1 (C-8), 113.3 (C-2), 114.0/114.1 (C-8a), 143.8 (C-7), 149.77/149.82 (C-6, C-1), 151.3 (C-1), 153.3 (C-8b), 157.5/157.7 (C-4a), 160.7/162.2 (C-3), 171.1/171.9/172.2/172.5/172.8/173.1/173.4/173.6 (COCH₂CH₃), 172.68/172.71 (C-9).HRMS (ESI) m/z: [M-H]⁻ Calcd for C₃₄H₃₇O₁₆ 701.2076; Found 701.2070.

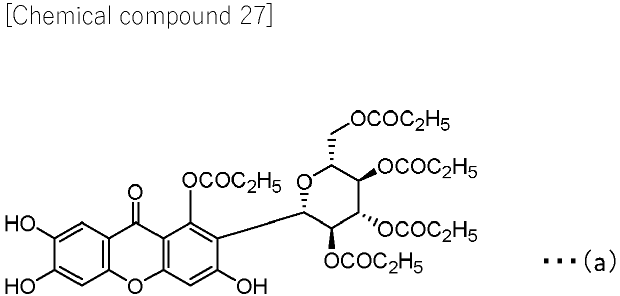

### < Synthesis of (n) formula (norathyriol) >

Norathyriol was prepared as another comparative example.

Norathyriol was synthesized according to the method of Non-Patent Literature 48. Norathyriol is a known substance represented by CAS number 3542-72-1 and is commercially available and may be purchased.

Norathyriol was synthesized as follows. Namely, 2,4,5-trimethoxybenzoic acid (Compound II) was treated with thionyl chloride to obtain 2,4,5-trimethoxybenzoic acid chloride (Compound III) according to the method described in the literature (Non-Patent Literature 48). Next, the obtained compound (Compound III) was reacted with 1,3,5-trimethoxybenzene (Compound IV) by Friedel-Craft reaction to give 2-hydrodoxy-2',4,4',5,6'-pentamethoxybenzophenone (Compound V) was obtained. The compound (V) was further treated with tetrabutylammonium hydroxide to afford 1,3,6,7-tetramethoxyxanthone (compound VI), followed by demethylation to give norathyriol (compound I) in 39% yield.

The following is a detailed description of the synthetic route of this example. (1) Synthesis of 2,4,5-trimethoxybenzoic acid chloride (Compound III) 2,4,5-trimethoxybenzoic acid (Compound II, 8.49 g, 0.040 mol) was dissolved by gradually adding thionyl chloride (5 mL) at room temperature under an argon atmosphere, and then heated to reflux for 6 h. After completion of the reaction, the reaction mixture was distilled off under reduced pressure to obtain 2,4,5-trimethoxybenzoic acid chloride (compound III, 8.30 g, 90%). The obtained compound (III) was immediately used for the next reaction.

### (2) Synthesis of 2-hydrodoxy-2',4,4',5,6'-pentamethoxybenzophenone (Compound V)

To a mixed suspension of 2,4,5-trimethoxybenzoic acid chloride (Compound III, 8.07 g, 0.035 mol) obtained as described above, 1,3,5-trimethoxybenzene (Compound IV, 6.48 g, 0.0385 mol), and anhydrous diethyl ether (500 mL) was added aluminum chloride (16 g) gradually at room temperature under argon atmosphere, and the reaction mixture was stirred at room temperature for 48 h. After removing the solvent from the reaction mixture under reduced pressure, water was added to the residue and extracted with ethyl acetate. The extract was washed with saturated brine, dried over anhydrous sodium sulfate, filtered off the drying agent using folded filter paper, and the filtrate was removed under reduced pressure to give the crude product. The crude product was purified by silica gel column chromatography (n-hexane:ethyl acetate = 1:1, v/v) to give 2-hydrodoxy-2',4,4',5,6'-pentamethoxybenzophenone (Compound V, 8.43 g, 69%).

### (3) Synthesis of 1,3,6,7-tetramethoxyxanthone (Compound VI)

2-hydrodoxy-2',4,4',5,6'-pentamethoxybenzophenone (Compound V, 6.97 g, 0.020 mol) obtained as described above was dissolved in a mixture of pyridine (10 mL) and water (10 mL), and 40% tetrabutylammonium hydroxide solution (5 mL) was added and heated to reflux for 6 h. The resulting reaction mixture was poured into 5% hydrochloric acid and extracted with ethyl acetate. The extract was washed with saturated brine, dried over anhydrous sodium sulfate, filtered off the drying agent using folded filter paper, and the filtrate was removed under reduced pressure. The crude product was purified by silica gel column chromatography (n-hexane: ethyl acetate = 1:1, v/v) to afford 1,3,6,7-tetramethoxyxanthone (compound VI, 5.82 g, 92%).

### (4) Synthesis of norathyriol (Compound I)

A mixture of 1,3,6,7-tetramethoxyxanthone (Compound VI, 4.74 g, 0.015 mol) and pyridine hydrochloride (5.00 g) obtained as described above was heated and stirred at 200 °C for 6 h. The resulting reaction mixture was allowed to cool to room temperature, poured into 5% hydrochloric acid, and extracted with ethyl acetate. The extract was washed with saturated brine, dried over anhydrous sodium sulfate, filtered off the drying agent using folded filter paper, and the filtrate was removed under reduced pressure to obtain the crude product. The crude product was purified by silica gel column chromatography (chloroform:methanol = 7:1, v/v) to afford norathyriol of formula (n) (Compound I, 2.65 g, 68%). The norathyriol structure is represented by formula (n).

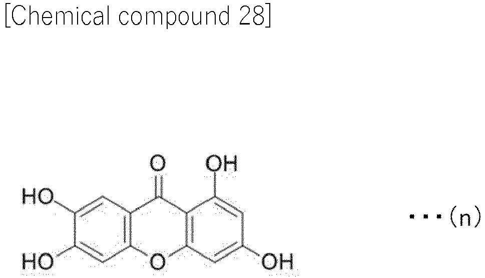

The following are the results of our investigation of the cell death inducing effects of the compounds of the present invention.

### < Example 1: Investigation of cell death inducing effects of each compound on KMS-28BM cells >

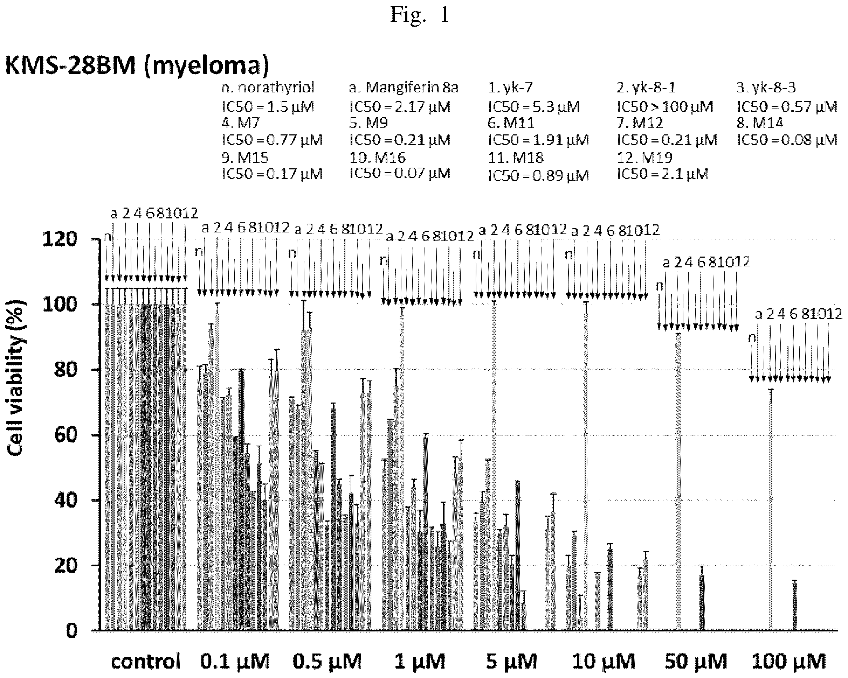

KMS-28BM cells (myeloma cell line) were cultured under 5% CO₂ at 37°C. The culture medium used RPMI-1640 medium with 100 µg/mL penicillin, 100 U/mL streptomycin and fetal bovine serum. KMS-28BM cells were seeded into 96-well plates and incubated with each concentration of the compounds was added, and cell viability was determined by the trypan blue staining. The results are shown in Figure 1.

Referring to Figure 1, the horizontal axis shows the concentration of each compound and the vertical axis shows the cell viability (%). For controls, only 0.5% DMSO (diluted in PBS) used to dissolve the compounds was added. For each concentration, each bar line is indicated by an arrow with the sign "n, a, 1, 2, 3, 4, 5, 6, 7, 8, 9, 10, 11, 12" from left to right. The "1, 3, 5, 7, 9, 11" are omitted from the graph, but the sign "1" shall be indicated by an arrow between the signs "a" and "2", the sign "3" between the signs "2" and "4", and other odd numbers shall likewise be indicated by arrows between even numbers before and after.

The sign "n" is the norathyriol (formula (n)) administration group, the sign "a" is the mangiferin 8a (formula (a)) administration group, the sign "1" is the yk-7 (formula (1)) administration group, the sign "2" is the yk-8-1 (formula (2)) administration group, the sign "3" is the yk-8-3 (formula (3)) administration group, the sign "4" is the M7 (formula (4)) administration group, the sign "5" is the M9 (formula (5)) administration group, the sign "6" is the M11 (formula (6)) administration group, the sign "7" is the M12 (formula (7)) administration group, the sign "8" is the M14 (formula (8)) administration group, the sign "9" is the M15 (formula (9)) administration group, the sign "10" is the M16 (formula (10)) administration group, the sign "11" is the M18 (formula (11)) administration group, the sign "12" is the M19 (formula (12)) administration group, respectively. The IC50 values for each compound is also listed in the upper right corner of the figure.

A concentration-dependent decrease in cell viability was observed for all compounds. Table 1 shows the results of IC50 (half inhibitory concentration; same hereafter) values calculated.

Although the IC50 value of the yk-8-1 (formula (2)) administration group was high, the yk-7 (formula (1)) administration group, yk-8-3 (formula (3)) administration group, M7 (formula (4)) administration group, M9 (formula (5)) administration group, M11 (formula (6)) administration group, M12 (formula (7)) administration group, M14 (formula (8)) administration group, M15 (formula (9)) administration group, M16 (formula (10)) administration group, M18 (formula (11)) administration group, and M19 (formula (12)) administration group were induced cell death in myeloma cell line KMS-28BM cells at concentrations lower than or equivalent to norathyriol (formula (n)) administration group and mangiferin 8a (formula (a)) administration group.

**[Table 1]**

| Cell lines | KMS-28BM(myeloma) | | | | | | |
|---|---|---|---|---|---|---|---|
| Compounds | norathyriol formula (n) | 8a formula (a) | yk-7 formula (1) | yk-8-1 formula (2) | yk-8-3 formula (3) | M7 formula (4) | M9 formula (5) |
| IC50 values | 1.5 µM | 2.17 µM | 5.3 µM | > 100 µM | 0.57 µM | 0.77 µM | 0.21 µM |
| | | | | | | | |

| Cell lines | KMS-28BM(myeloma) | | | | | | |
|---|---|---|---|---|---|---|---|
| Compounds | M11 formula (6) | M12 formula (7) | M14 formula (8) | M15 formula (9) | M16 formula (10) | M18 formula (11) | M19 formula (12) |
| IC50 values | 1.91 µM | 0.21 µM | 0.08 µM | 0.17 µM | 0.07 µM | 0.89 µM | 2.1 µM |

### < Example 2: Investigation of cell death inducing effects of each compound on L363 cells >

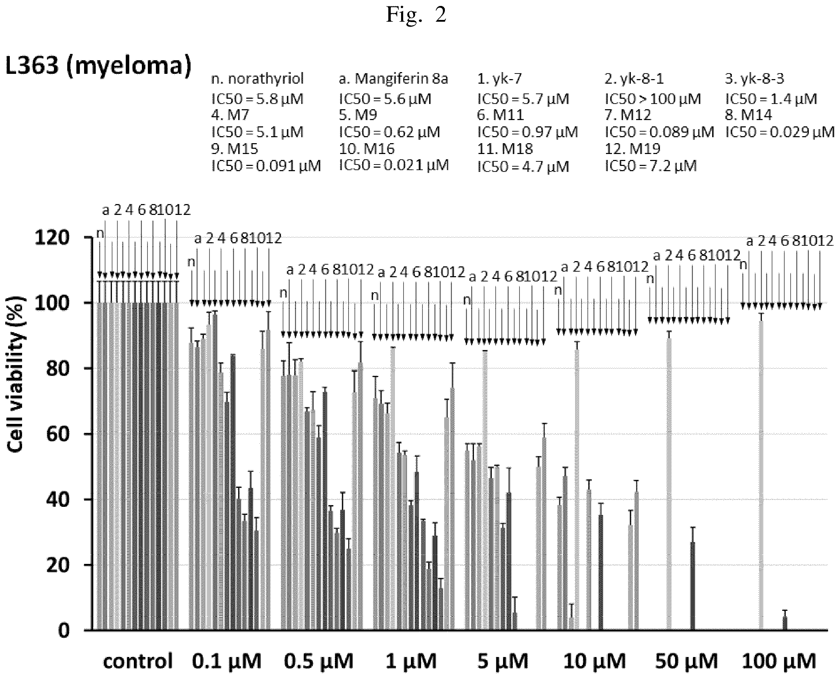

L363 cells (myeloma cell line) were cultured under 5% CO₂ at 37°C. The culture medium used RPMI-1640 medium with 100 µg/mL penicillin, 100 U/mL streptomycin and fetal bovine serum. L363 cells were seeded into 96-well plates and incubated with each concentration of the compounds was added, and cell viability was determined by the trypan blue staining. The results are shown in Figure 2.

Referring to Figure 2, the horizontal axis shows the concentration of each comnound and the vertical axis shows the cell viability (%). For controls, only 0.5% DMSO (diluted in PBS) used to dissolve the compounds was added. For each concentration, each bar line is indicated by an arrow with the sign "n, a, 1, 2, 3, 4, 5, 6, 7, 8, 9, 10, 11, 12" from left to right. The "1, 3, 5, 7, 9, 11" are omitted from the graph, but the sign "1" shall be indicated by an arrow between the signs "a" and "2", the sign "3" between the signs "2" and "4", and other odd numbers shall likewise be indicated by arrows between even numbers before and after.

The sign "n" is the norathyriol (formula (n)) administration group, the sign "a" is the mangiferin 8a (formula (a)) administration group, the sign "1" is the yk-7 (formula (1)) administration group, the sign "2" is the yk-8-1 (formula (2)) administration group, the sign "3" is the yk-8-3 (formula (3)) administration group, the sign "4" is the M7 (formula (4)) administration group, the sign "5" is the M9 (formula (5)) administration group, the sign "6" is the M11 (formula (6)) administration group, the sign "7" is the M12 (formula (7)) administration group, the sign "8" is the M14 (formula (8)) administration group, the sign "9" is the M15 (formula (9)) administration group, the sign "10" is the M16 (formula (10)) administration group, the sign "11" is the M18 (formula (11)) administration group, the sign "12" is the M19 (formula (12)) administration group, respectively. The IC50 values for each compound is also listed in the upper right corner of the figure.

A concentration-dependent decrease in cell viability was observed for each compound except for the yk-8-1 (formula (2)) administration group. The IC50 values were calculated in Table 2.

Although the IC50 value of the yk-8-1 (formula (2)) administration group was high, the yk-7 (formula (1)) administration group, yk-8-3 (formula (3)) administration group, M7 (formula (4)) administration group, M9 (formula (5)) administration group, M11 (formula (6)) administration group, M12 (formula (7)) administration group, M14 (formula (8)) administration group, M15 (formula (9)) administration group, M16 (formula (10)) administration group, M18 (formula (11)) administration group, and M19 (formula (12)) administration group were induced cell death in myeloma cell line L363 cells at concentrations lower than or equivalent to norathyriol (formula (n)) administration group and mangiferin 8a (formula (a)) administration group.

**[Table 2]**

| Cell lines | L363 (myeloma) | | | | | | |
|---|---|---|---|---|---|---|---|
| Compounds | norathyriol formula (n) | 8a formula (a) | yk-7 formula (1) | yk-8-1 formula (2) | yk-8-3 formula (3) | M7 formula (4) | M9 formula (5) |
| IC50 values | 5.8 µM | 5.6 µM | 5.7 µM | > 100 µM | 1.4 µM | 5.1 µM | 0.62 µM |
| | | | | | | | |

| Cell lines | L363 (myeloma) | | | | | | |
|---|---|---|---|---|---|---|---|
| Compounds | M11 formula (6) | M12 formula (7) | M14 formula (8) | M15 formula (9) | M16 formula (10) | M18 formula (11) | M19 formula (12) |
| IC50 values | 0.97 µM | 0.089 µM | 0.029 µM | 0.091 µM | 0.021 µM | 4.7 µM | 7.2 µM |

### < Example 3: Investigation of cell death inducing effects of each compound on RPMI8226 cells >

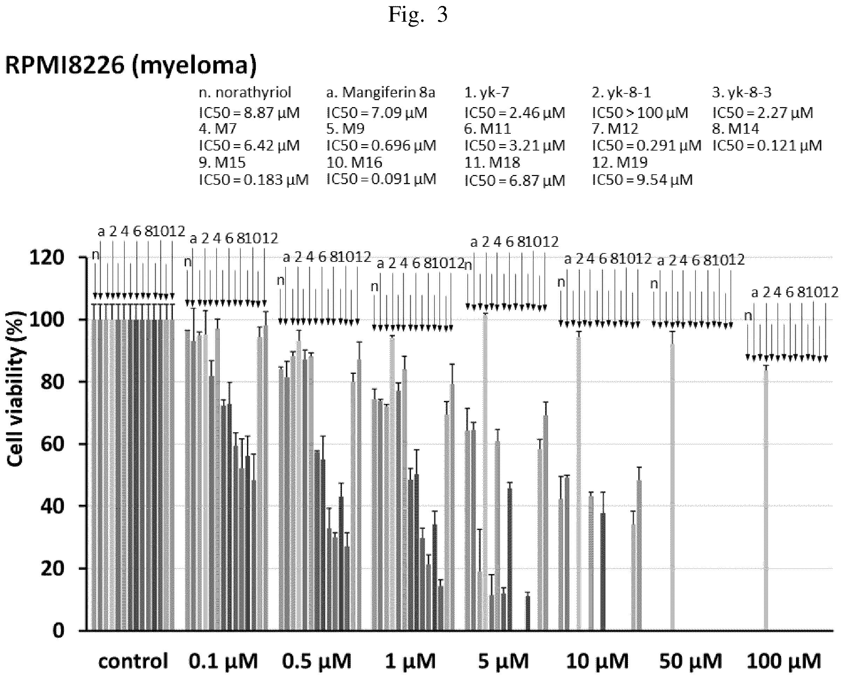

RPMI8226 cells (myeloma cell line) were cultured under 5% CO₂ at 37°C. The culture medium used RPMI-1640 medium with 100 µg/mL penicillin, 100 U/mL streptomycin and fetal bovine serum. RPMI8226 cells were seeded into 96-well plates and incubated with each concentration of the compounds was added, and cell viability was determined by the trypan blue staining. The results are shown in Figure 3.

Referring to Figure 3, the horizontal axis shows the concentration of each compound and the vertical axis shows the cell viability (%). For controls, only 0.5% DMSO (diluted in PBS) used to dissolve the compounds was added. For each concentration, each bar line is indicated by an arrow with the sign "n, a, 1, 2, 3, 4, 5, 6, 7, 8, 9, 10, 11, 12" from left to right. The "1, 3, 5, 7, 9, 11" are omitted from the graph, but the sign "1" shall be indicated by an arrow between the signs "a" and "2", the sign "3" between the signs "2" and "4", and other odd numbers shall likewise be indicated by arrows between even numbers before and after.

The sign "n" is the norathyriol (formula (n)) administration group, the sign "a" is the mangiferin 8a (formula (a)) administration group, the sign "1" is the yk-7 (formula (1)) administration group, the sign "2" is the yk-8-1 (formula (2)) administration group, the sign "3" is the yk-8-3 (formula (3)) administration group, the sign "4" is the M7 (formula (4)) administration group, the sign "5" is the M9 (formula (5)) administration group, the sign "6" is the M11 (formula (6)) administration group, the sign "7" is the M12 (formula (7)) administration group, the sign "8" is the M14 (formula (8)) administration group, the sign "9" is the M15 (formula (9)) administration group, the sign "10" is the M16 (formula (10)) administration group, the sign "11" is the M18 (formula (11)) administration group, the sign "12" is the M19 (formula (12)) administration group, respectively. The IC50 values for each compound is also listed in the upper right corner of the figure.

A concentration-dependent decrease in cell viability was observed for all compounds. Table 3 shows the results of IC50 values calculated.

Although the IC50 value of the yk-8-1 (formula (2)) administration group was high, the yk-7 (formula (1)) administration group, yk-8-3 (formula (3)) administration group, M7 (formula (4)) administration group, M9 (formula (5)) administration group, M11 (formula (6)) administration group, M12 (formula (7)) administration group, M14 (formula (8)) administration group, M15 (formula (9)) administration group, M16 (formula (10)) administration group, M18 (formula (11)) administration group, and M19 (formula (12)) administration group were induced cell death in myeloma cell line RPMI8226 cells at concentrations lower than or equivalent to norathyriol (formula (n)) administration group and mangiferin 8a (formula (a)) administration group.

**[Table 3]**

| Cell lines | RPMI8226(myeloma) | | | | | | |
|---|---|---|---|---|---|---|---|
| Compounds | norathyriol formula (n) | 8a formula (a) | yk-7 formula (1) | yk-8-1 formula (2) | yk-8-3 formula (3) | M7 formula (4) | M9 formula (5) |
| IC50 values | 8.87 µM | 7.09 µM | 2.46 µM | > 100 µM | 2.27 µM | 6.42 µM | 0.696 µM |
| | | | | | | | |

| Cell lines | RPMI8226(myeloma) | | | | | | |
|---|---|---|---|---|---|---|---|
| Compounds | M11 formula (6) | M12 formula (7) | M14 formula (8) | M15 formula (9) | M16 formula (10) | M18 formula (11) | M19 formula (12) |
| IC50 values | 3.21 µM | 0.291 µM | 0.121 µM | 0.183 µM | 0.091 µM | 6.87 µM | 9.54 µM |

### < Example 4: Investigation of cell death inducing effects of each compound on RPMI8226/B cells >

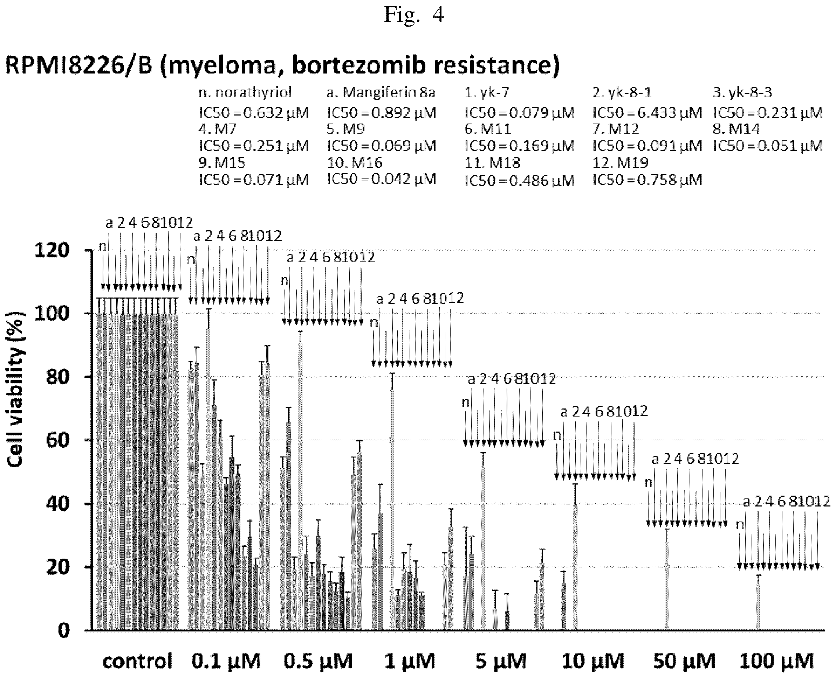

RPMI8226/B cells (bortezomib-resistant myeloma cell line) were cultured under 5% CO₂ at 37°C. The culture medium used RPMI-1640 medium with 100 µg/mL penicillin, 100 U/mL streptomycin and fetal bovine serum. RPMI8226/B cells were seeded into 96-well plates and incubated with each concentration of the compounds was added, and cell viability was determined by the trypan blue staining. The results are shown in Figure 4.

Referring to Figure 4, the horizontal axis shows the concentration of each compound and the vertical axis shows the cell viability (%). For controls, only 0.5% DMSO (diluted in PBS) used to dissolve the compounds was added. For each concentration, each bar line is indicated by an arrow with the sign "n, a, 1, 2, 3, 4, 5, 6, 7, 8, 9, 10, 11, 12" from left to right. The "1, 3, 5, 7, 9, 11" are omitted from the graph, but the sign "1" shall be indicated by an arrow between the signs "a" and "2", the sign "3" between the signs "2" and "4", and other odd numbers shall likewise be indicated by arrows between even numbers before and after.

The sign "n" is the norathyriol (formula (n)) administration group, the sign "a" is the mangiferin 8a (formula (a)) administration group, the sign "1" is the yk-7 (formula (1)) administration group, the sign "2" is the yk-8-1 (formula (2)) administration group, the sign "3" is the yk-8-3 (formula (3)) administration group, the sign "4" is the M7 (formula (4)) administration group, the sign "5" is the M9 (formula (5)) administration group, the sign "6" is the M11 (formula (6)) administration group, the sign "7" is the M12 (formula (7)) administration group, the sign "8" is the M14 (formula (8)) administration group, the sign "9" is the M15 (formula (9)) administration group, the sign "10" is the M16 (formula (10)) administration group, the sign "11" is the M18 (formula (11)) administration group, the sign "12" is the M19 (formula (12)) administration group, respectively. The IC50 values for each compound is also listed in the upper right corner of the figure.

A concentration-dependent decrease in cell viability was observed for all compounds. Table 4 shows the results of IC50 values calculated.

Although the IC50 value of the yk-8-1 (formula (2)) administration group was high, the yk-7 (formula (1)) administration group, yk-8-3 (formula (3)) administration group, M7 (formula (4)) administration group, M9 (formula (5)) administration group, M11 (formula (6)) administration group, M12 (formula (7)) administration group, M14 (formula (8)) administration group, M15 (formula (9)) administration group, M16 (formula (10)) administration group, M18 (formula (11)) administration group, and M19 (formula (12)) administration group were induced cell death in bortezomib-resistant myeloma cell line RPMI8226/B cells at concentrations lower than or equivalent to norathyriol (formula (n)) administration group and mangiferin 8a (formula (a)) administration group.

**[Table 4]**

| Cell lines | RPMI8226/B (myeloma; bortezomib resistance) | | | | | | |
|---|---|---|---|---|---|---|---|
| Compounds | norathyriol formula (n) | 8a formula (a) | yk-7 formula (1) | yk-8-1 formula (2) | yk-8-3 formula (3) | M7 formula (4) | M9 formula (5) |
| IC50 values | 0.632 µM | 0.892 µM | 0.079 µM | 6.433 µM | 0.231 µM | 0.251 µM | 0.069 µM |
| | | | | | | | |

| Cell lines | RPMI8226/B (myeloma; bortezomib resistance) | | | | | | |
|---|---|---|---|---|---|---|---|
| Compounds | M11 formula (6) | M12 formula (7) | M14 formula (8) | M15 formula (9) | M16 formula (10) | M18 formula (11) | M19 formula (12) |
| IC50 values | 0.169 µM | 0.091 µM | 0.051 µM | 0.071 µM | 0.042 µM | 0.486 µM | 0.758 µM |

### < Example 5: Investigation of cell death inducing effects of each compound on Rec-l cells >

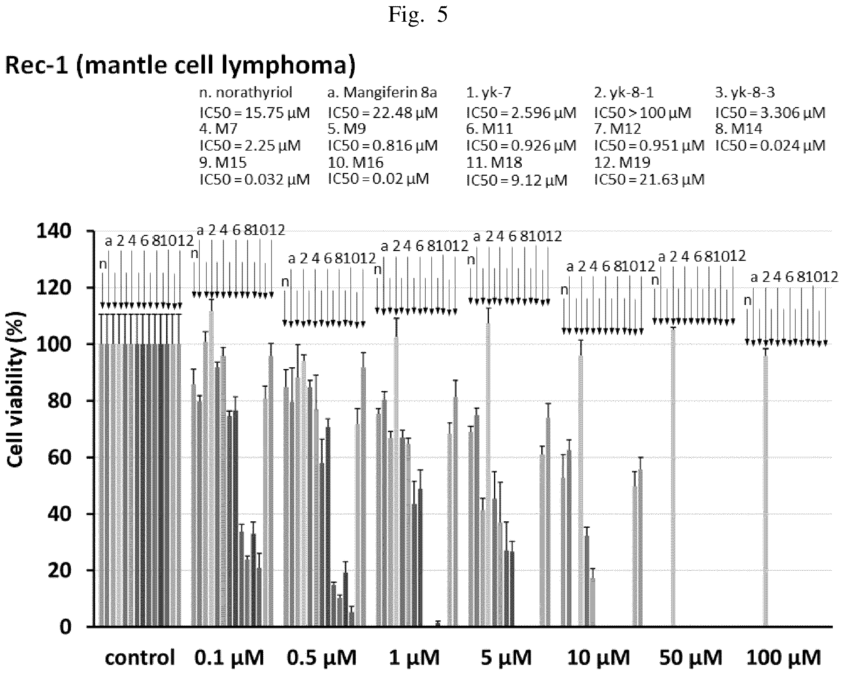

Rec-1 cells (mantle cell lymphoma cell line) were cultured under 5% CO₂ at 37°C. The culture medium used RPMI-1640 medium with 100 µg/mL penicillin, 100 U/mL streptomycin and fetal bovine serum. Rec-1 cells were seeded into 96-well plates and incubated with each concentration of the compounds was added, and cell viability was determined by the trypan blue staining. The results are shown in Figure 5.

Referring to Figure 5, the horizontal axis shows the concentration of each compound and the vertical axis shows the cell viability (%). For controls, only 0.5% DMSO (diluted in PBS) used to dissolve the compounds was added. For each concentration, each bar line is indicated by an arrow with the sign "n, a, 1, 2, 3, 4, 5, 6, 7, 8, 9, 10, 11, 12" from left to right. The "1, 3, 5, 7, 9, 11" are omitted from the graph, but the sign "1" shall be indicated by an arrow between the signs "a" and "2", the sign "3" between the signs "2" and "4", and other odd numbers shall likewise be indicated by arrows between even numbers before and after.

The sign "n" is the norathyriol (formula (n)) administration group, the sign "a" is the mangiferin 8a (formula (a)) administration group, the sign "1" is the yk-7 (formula (1)) administration group, the sign "2" is the yk-8-1 (formula (2)) administration group, the sign "3" is the yk-8-3 (formula (3)) administration group, the sign "4" is the M7 (formula (4)) administration group, the sign "5" is the M9 (formula (5)) administration group, the sign "6" is the M11 (formula (6)) administration group, the sign "7" is the M12 (formula (7)) administration group, the sign "8" is the M14 (formula (8)) administration group, the sign "9" is the M15 (formula (9)) administration group, the sign "10" is the M16 (formula (10)) administration group, the sign "11" is the M18 (formula (11)) administration group, the sign "12" is the M19 (formula (12)) administration group, respectively. The IC50 values for each compound is also listed in the upper right corner of the figure.

A concentration-dependent decrease in cell viability was observed for each compound except for the yk-8-1 (formula (2)) administration group. The IC50 values were calculated in Table 5.

Although the IC50 value of the yk-8-1 (formula (2)) administration group was high, the yk-7 (formula (1)) administration group, yk-8-3 (formula (3)) administration group, M7 (formula (4)) administration group, M9 (formula (5)) administration group, M11 (formula (6)) administration group, M12 (formula (7)) administration group, M14 (formula (8)) administration group, M15 (formula (9)) administration group, M16 (formula (10)) administration group, M18 (formula (11)) administration group, and M19 (formula (12)) administration group were induced cell death in mantle cell lymphoma cell line Rec-1 cells at concentrations lower than or equivalent to norathyriol (formula (n)) administration group and mangiferin 8a (formula (a)) administration group.

**[Table 5]**

| Cell lines | Rec-1 (mantle cell lymphoma) | | | | | | |
|---|---|---|---|---|---|---|---|
| Compounds | norathyriol formula (n) | 8a formula (a) | yk-7 formula (1) | yk-8-1 formula (2) | yk-8-3 formula (3) | M7 formula (4) | M9 formula (5) |
| IC50 values | 1575 µM | 22.48 µM | 2.596 µM | > 100 µM | 3.306 µM | 2.250 µM | 0.816 µM |
| | | | | | | | |

| Cell lines | Rec-1 (mantle cell lymphomal | | | | | | |
|---|---|---|---|---|---|---|---|
| Compounds | M11 formula (6) | M12 formula (7) | M14 formula (8) | M15 formula (9) | M16 formula (10) | M18 formula (11) | M19 formula (12) |
| IC50 values | 0.926 µM | 0.951 µM | 0.024 µM | 0.032 µM | 0.020 µM | 9.12 µM | 21.63 µM |

### < Example 6: Investigation of cell death inducing effects of each compound on Raji cells >

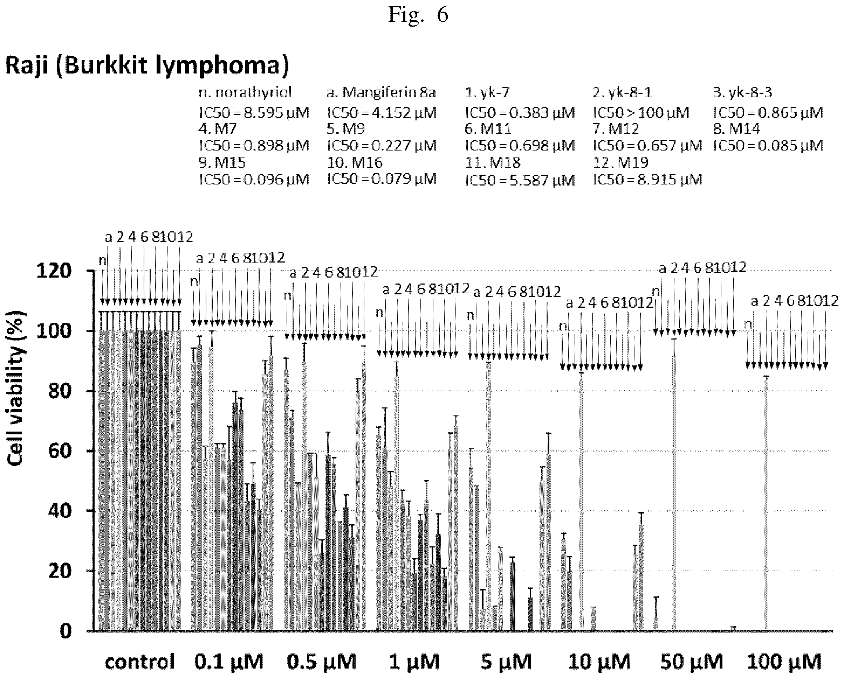

Raji cells (burkitt lymphoma cell line) were cultured under 5% CO₂ at 37°C. The culture medium used RPMI-1640 medium with 100 µg/mL penicillin, 100 U/mL streptomycin and fetal bovine serum. Raji cells were seeded into 96-well plates and incubated with each concentration of the compounds was added, and cell viability was determined by the trypan blue staining. The results are shown in Figure 6.

Referring to Figure 6, the horizontal axis shows the concentration of each compound and the vertical axis shows the cell viability (%). For controls, only 0.5% DMSO (diluted in PBS) used to dissolve the compounds was added. For each concentration, each bar line is indicated by an arrow with the sign "n, a, 1, 2, 3, 4, 5, 6, 7, 8, 9, 10, 11, 12" from left to right. The "1, 3, 5, 7, 9, 11" are omitted from the graph, but the sign "1" shall be indicated by an arrow between the signs "a" and "2", the sign "3" between the signs "2" and "4", and other odd numbers shall likewise be indicated by arrows between even numbers before and after.

The sign "n" is the norathyriol (formula (n)) administration group, the sign "a" is the mangiferin 8a (formula (a)) administration group, the sign "1" is the yk-7 (formula (1)) administration group, the sign "2" is the yk-8-1 (formula (2)) administration group, the sign "3" is the yk-8-3 (formula (3)) administration group, the sign "4" is the M7 (formula (4)) administration group, the sign "5" is the M9 (formula (5)) administration group, the sign "6" is the M11 (formula (6)) administration group, the sign "7" is the M12 (formula (7)) administration group, the sign "8" is the M14 (formula (8)) administration group, the sign "9" is the M15 (formula (9)) administration group, the sign "10" is the M16 (formula (10)) administration group, the sign "11" is the M18 (formula (11)) administration group, the sign "12" is the M19 (formula (12)) administration group, respectively. The IC50 values for each compound is also listed in the upper right corner of the figure.

A concentration-dependent decrease in cell viability was observed for each compound except for the yk-8-1 (formula (2)) administration group. The IC50 values were calculated in Table 6.

Although the IC50 value of the yk-8-1 (formula (2)) administration group was high, the yk-7 (formula (1)) administration group, yk-8-3 (formula (3)) administration group, M7 (formula (4)) administration group, M9 (formula (5)) administration group, M11 (formula (6)) administration group, M12 (formula (7)) administration group, M14 (formula (8)) administration group, M15 (formula (9)) administration group, M16 (formula (10)) administration group, M18 (formula (11)) administration group, and M19 (formula (12)) administration group were induced cell death in burkitt lymphoma cell line Raji cells at concentrations lower than or equivalent to norathyriol (formula (n)) administration group and mangiferin 8a (formula (a)) administration group.

**[Table 6]**

| Cell lines | Raji(Burkkit lymphoma) | | | | | | |
|---|---|---|---|---|---|---|---|
| Compounds | norathyriol formula (n) | 8a formula (a) | yk-7 formula (1) | yk-8-1 formula (2) | yk-8-3 formula (3) | M7 formula (4) | M9 formula (5) |
| IC50 values | 8.595 µM | 4.152 µM | 0.383 µM | > 100 µM | 0.865 µM | 0.898 µM | 0.227 µM |
| | | | | | | | |

| Cell lines | Raji(Burkkit lymphoma) | | | | | | |
|---|---|---|---|---|---|---|---|
| Compounds | M11 formula (6) | M12 formula (7) | M14 formula (8) | M15 formula (9) | M16 formula (10) | M18 formula (11) | M19 formula (12) |
| IC50 values | 0.698 µM | 0.657 µM | 0.085 µM | 0.096 µM | 0.079 µM | 5.587 µM | 8.915 µM |

### < Example 7: Investigation of cell death inducing effects of each compound on RR1 cells >

Raji cells, a burkitt lymphoma cell line, were modified to be rituximab-resistant and named RR1 cells.

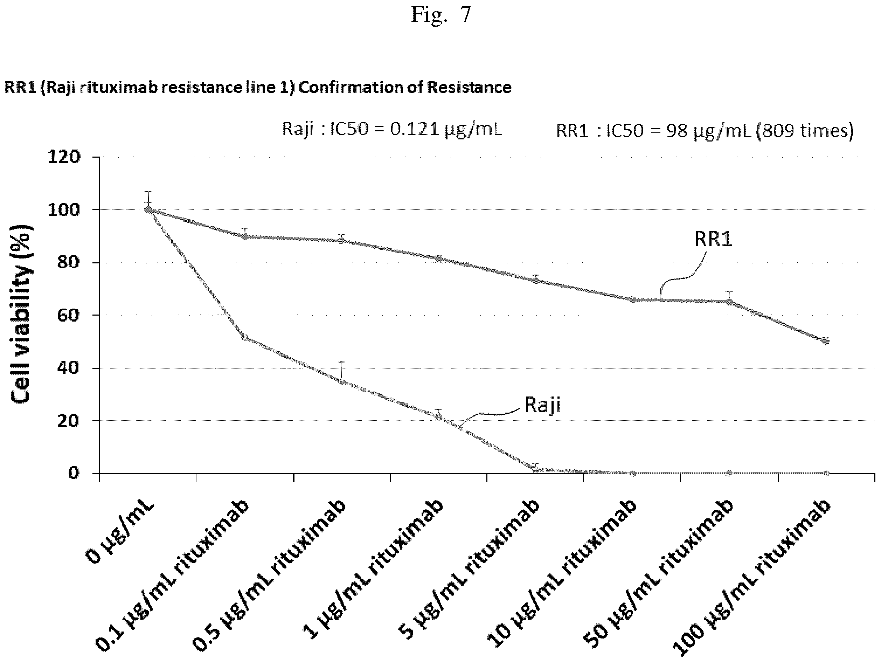

Figure 7 shows the rituximab resistance of RR1 cells. Referring to Figure 7, the horizontal axis is the rituximab concentration (µg/mL) and the vertical axis is the cell viability (%). Both Raji and RR1 cells showed a decrease in cell viability as the rituximab concentration increased. However, the cell viability was clearly higher in RR1 cells, confirming that they are resistant to rituximab.

The IC50 of Raji cells to rituximab was 0.121 pg/mL, whereas the IC50 of RR1 cells to rituximab was 98 µg/mL, approximately 809 times more resistant than Raji cells.

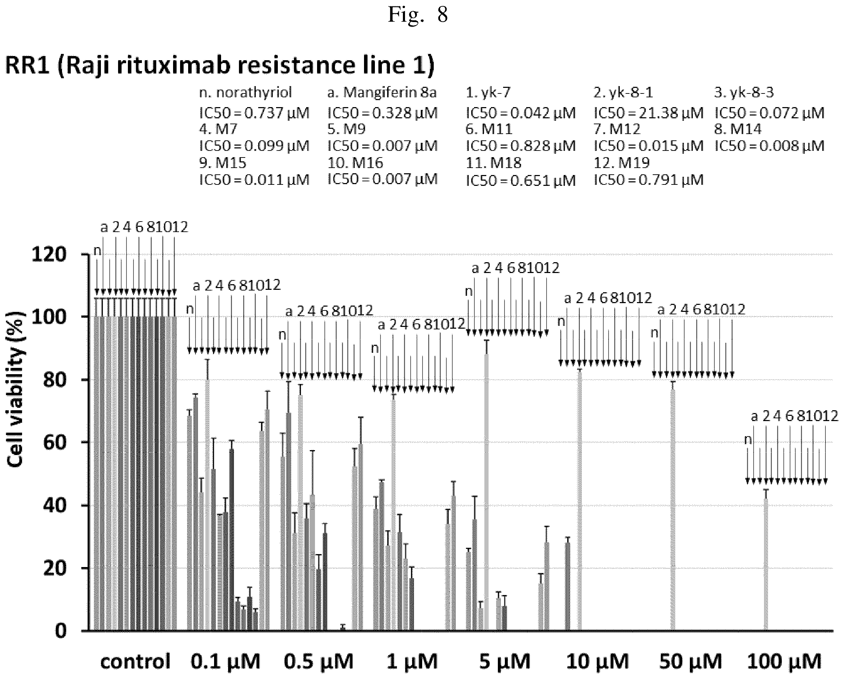

RR1 cells (rituximab-resistant burkitt lymphoma cell line) were cultured under 5% CO₂ at 37°C. The culture medium used RPMI-1640 medium with 100 µg/mL penicillin, 100 U/mL streptomycin and fetal bovine serum. RR-1 cells were seeded into 96-well plates and incubated with each concentration of the compounds was added, and cell viability was determined by the trypan blue staining. The results are shown in Figure 8.

Referring to Figure 8, the horizontal axis shows the concentration of each compound and the vertical axis shows the cell viability (%). For controls, only 0.5% DMSO (diluted in PBS) used to dissolve the compounds was added. For each concentration, each bar line is indicated by an arrow with the sign "n, a, 1, 2, 3, 4, 5, 6, 7, 8, 9, 10, 11, 12" from left to right. The "1, 3, 5, 7, 9, 11" are omitted from the graph, but the sign "1" shall be indicated by an arrow between the signs "a" and "2", the sign "3" between the signs "2" and "4", and other odd numbers shall likewise be indicated by arrows between even numbers before and after.

The sign "n" is the norathyriol (formula (n)) administration group, the sign "a" is the mangiferin 8a (formula (a)) administration group, the sign "1" is the yk-7 (formula (1)) administration group, the sign "2" is the yk-8-1 (formula (2)) administration group, the sign "3" is the yk-8-3 (formula (3)) administration group, the sign "4" is the M7 (formula (4)) administration group, the sign "5" is the M9 (formula (5)) administration group, the sign "6" is the M11 (formula (6)) administration group, the sign "7" is the M12 (formula (7)) administration group, the sign "8" is the M14 (formula (8)) administration group, the sign "9" is the M15 (formula (9)) administration group, the sign "10" is the M16 (formula (10)) administration group, the sign "11" is the M18 (formula (11)) administration group, the sign "12" is the M19 (formula (12)) administration group, respectively. The IC50 values for each compound is also listed in the upper right corner of the figure.

A concentration-dependent decrease in cell viability was observed for all compounds. Table 7 shows the results of IC50 values calculated.

Although the IC50 value of the yk-8-1 (formula (2)) administration group was high, the yk-7 (formula (1)) administration group, yk-8-3 (formula (3)) administration group, M7 (formula (4)) administration group, M9 (formula (5)) administration group, M11 (formula (6)) administration group, M12 (formula (7)) administration group, M14 (formula (8)) administration group, M15 (formula (9)) administration group, M16 (formula (10)) administration group, M18 (formula (11)) administration group, and M19 (formula (12)) administration group were induced cell death in rituximab-resistant burkitt lymphoma cell line RR1 cells at concentrations lower than or equivalent to norathyriol (formula (n)) administration group and mangiferin 8a (formula (a)) administration group.

In particular, these compounds with a xanthone skeleton were able to induce cell death against RR1 cells, which are rituximab-resistant burkitt lymphoma cells.

**[Table 7]**

| Cell lines | RR1 (Burkkit lymphoma; rituximab resistance) | | | | | | |
|---|---|---|---|---|---|---|---|
| Compounds | norathyriol formula (n) | 8a formula (a) | yk-7 formula (1) | yk-8-1 formula (2) | yk-8-3 formula (3) | M7 formula (4) | M9 formula (5) |
| IC50 values | 0.737 µM | 0.328 µM | 0.042 µM | 21.38 µM | 0.072 µM | 0.099 µM | 0.007 µM |
| | | | | | | | |

| Cell lines | RR1 (Burkkit lymphoma; rituximab resistance) | | | | | | |
|---|---|---|---|---|---|---|---|
| Compounds | M11 formula (6) | M12 formula (7) | M14 formula (8) | M15 formula (9) | M16 formula (10) | M18 formula (11) | M19 formula (12) |
| IC50 values | 0.828 µM | 0.015 µM | 0.008 µM | 0.011 µM | 0.007 µM | 0.651 µM | 0.791 µM |

### < Example 8: Investigation of cell death inducing effects of each compound on SU-DHL-4 cells>

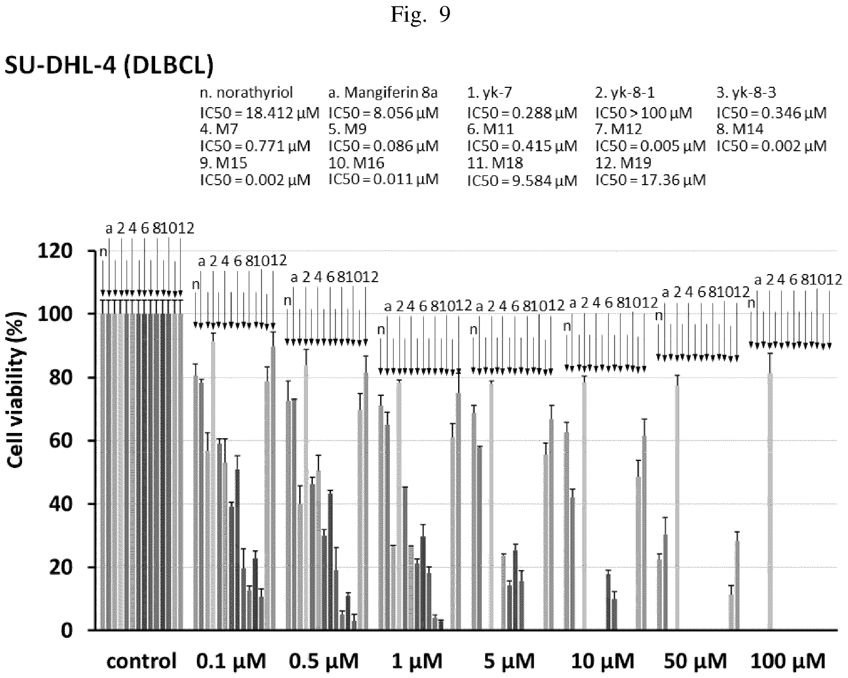

SU-DHL-4 cells (diffuse large B-cell lymphoma) were cultured under 5% CO₂ at 37°C. The culture medium used RPMI-1640 medium with 100 µg/mL penicillin, 100 U/mL streptomycin and fetal bovine serum. SU-DHL-4 cells were seeded into 96-well plates and incubated with each concentration of the compounds was added, and cell viability was determined by the trypan blue staining. The results are shown in Figure 9.

Referring to Figure 9, the horizontal axis shows the concentration of each compound and the vertical axis shows the cell viability (%). For controls, only 0.5% DMSO (diluted in PBS) used to dissolve the compounds was added. For each concentration, each bar line is indicated by an arrow with the sign "n, a, 1, 2, 3, 4, 5, 6, 7, 8, 9, 10, 11, 12" from left to right. The "1, 3, 5, 7, 9, 11" are omitted from the graph, but the sign "1" shall be indicated by an arrow between the signs "a" and "2", the sign "3" between the signs "2" and "4", and other odd numbers shall likewise be indicated by arrows between even numbers before and after.

The sign "n" is the norathyriol (formula (n)) administration group, the sign "a" is the mangiferin 8a (formula (a)) administration group, the sign "1" is the yk-7 (formula (1)) administration group, the sign "2" is the yk-8-1 (formula (2)) administration group, the sign "3" is the yk-8-3 (formula (3)) administration group, the sign "4" is the M7 (formula (4)) administration group, the sign "5" is the M9 (formula (5)) administration group, the sign "6" is the M11 (formula (6)) administration group, the sign "7" is the M12 (formula (7)) administration group, the sign "8" is the M14 (formula (8)) administration group, the sign "9" is the M15 (formula (9)) administration group, the sign "10" is the M16 (formula (10)) administration group, the sign "11" is the M18 (formula (11)) administration group, the sign "12" is the M19 (formula (12)) administration group, respectively. The IC50 values for each compound is also listed in the upper right corner of the figure.

A concentration-dependent decrease in cell viability was observed for each compound except for the yk-8-1 (formula (2)) administration group. The IC50 values were calculated in Table 8.

Although the IC50 value of the yk-8-1 (formula (2)) administration group was high, the yk-7 (formula (1)) administration group, yk-8-3 (formula (3)) administration group, M7 (formula (4)) administration group, M9 (formula (5)) administration group, M11 (formula (6)) administration group, M12 (formula (7)) administration group, M14 (formula (8)) administration group, M15 (formula (9)) administration group, M16 (formula (10)) administration group, M18 (formula (11)) administration group, and M19 (formula (12)) administration group were induced cell death in diffuse large B-cell lymphoma cell line SU-DHL-4 cells at concentrations lower than or equivalent to norathyriol (formula (n)) administration group and mangiferin 8a (formula (a)) administration group.

**[Table 8]**

| Cell lines | SU-DHL-4 (DLBCL) | | | | | | |
|---|---|---|---|---|---|---|---|
| Compounds | norathyriol formula (n) | 8a formula (a) | yk-7 formula (1) | yk-8-1 formula (2) | yk-8-3 formula (3) | M7 formula (4) | M9 formula (5) |
| IC50 values | 18.412 µM | 8.056 µM | 0.288 µM | > 100 µM | 0.346 µM | 0.771 µM | 0.086 µM |
| | | | | | | | |

| Cell lines | SU-DHL-4 (DLBCL) | | | | | | |
|---|---|---|---|---|---|---|---|
| Compounds | M11 formula (6) | M12 formula (7) | M14 formula (8) | M15 formula (9) | M16 formula (10) | M18 formula (11) | M19 formula (12) |
| IC50 values | 0.415 µM | 0.005 µM | 0.002 µM | 0.002 µM | 0.011 µM | 9.584 µM | 17.36 µM |

### < Example 9: Investigation of cell death inducing effects of each compound on CCRF-SB (acute lymphocytic leukemia) cells >

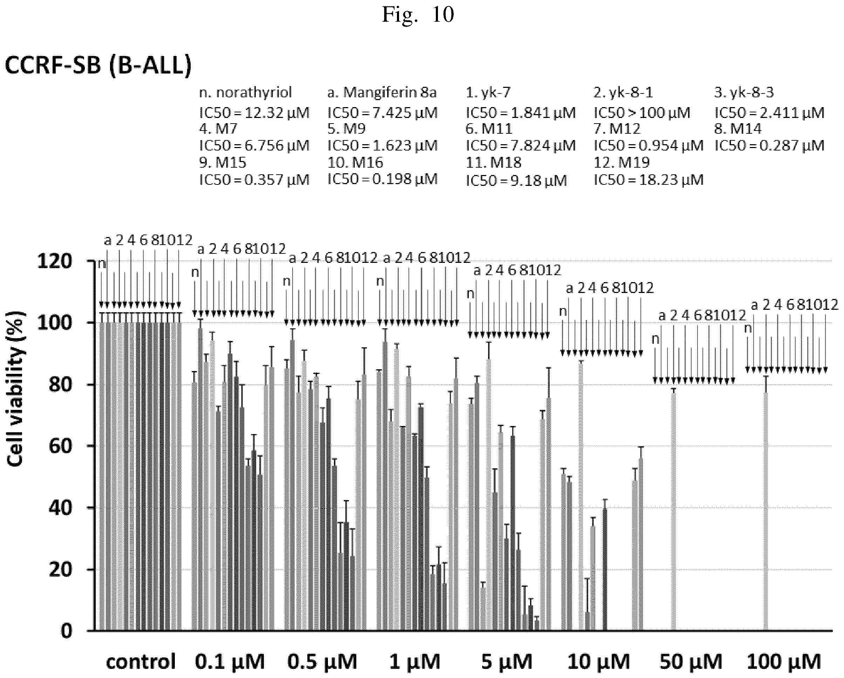

CCRF-SB cells (acute lymphocytic leukemia cell line) were cultured under 5% CO₂ at 37°C. The culture medium used RPMI-1640 medium with 100 µg/mL penicillin, 100 U/mL streptomycin and fetal bovine serum. CCRF-SB cells were seeded into 96-well plates and incubated with each concentration of the compounds was added, and cell viability was determined by the trypan blue staining. The results are shown in Figure 10.

Referring to Figure 10, the horizontal axis shows the concentration of each compound and the vertical axis shows the cell viability (%). For controls, only 0.5% DMSO (diluted in PBS) used to dissolve the compounds was added. For each concentration, each bar line is indicated by an arrow with the sign "n, a, 1, 2, 3, 4, 5, 6, 7, 8, 9, 10, 11, 12" from left to right. The "1, 3, 5, 7, 9, 11" are omitted from the graph, but the sign "1" shall be indicated by an arrow between the signs "a" and "2", the sign "3" between the signs "2" and "4", and other odd numbers shall likewise be indicated by arrows between even numbers before and after.

The sign "n" is the norathyriol (formula (n)) administration group, the sign "a" is the mangiferin 8a (formula (a)) administration group, the sign "1" is the yk-7 (formula (1)) administration group, the sign "2" is the yk-8-1 (formula (2)) administration group, the sign "3" is the yk-8-3 (formula (3)) administration group, the sign "4" is the M7 (formula (4)) administration group, the sign "5" is the M9 (formula (5)) administration group, the sign "6" is the M11 (formula (6)) administration group, the sign "7" is the M12 (formula (7)) administration group, the sign "8" is the M14 (formula (8)) administration group, the sign "9" is the M15 (formula (9)) administration group, the sign "10" is the M16 (formula (10)) administration group, the sign "11" is the M18 (formula (11)) administration group, the sign "12" is the M19 (formula (12)) administration group, respectively. The IC50 values for each compound is also listed in the upper right corner of the figure.

A concentration-dependent decrease in cell viability was observed for each compound. Table 9 shows the results of IC50 values calculated.

Although the IC50 value of the yk-8-1 (formula (2)) administration group was high, the yk-7 (formula (1)) administration group, yk-8-3 (formula (3)) administration group, M7 (formula (4)) administration group, M9 (formula (5)) administration group, M11 (formula (6)) administration group, M12 (formula (7)) administration group, M14 (formula (8)) administration group, M15 (formula (9)) administration group, M16 (formula (10)) administration group, M18 (formula (11)) administration group, and M19 (formula (12)) administration group were induced cell death in acute lymphocytic leukemia cell line CCRF-SB cells at concentrations lower than or equivalent to norathyriol (formula (n)) administration group and mangiferin 8a (formula (a)) administration group.

**[Table 9]**

| Cell lines | CCRF-SB (acute lymphoblastic leukemia) | | | | | | |
|---|---|---|---|---|---|---|---|
| Compounds | norathyriol formula (n) | 8a formula (a) | yk-7 formula (1) | yk-8-1 formula (2) | yk-8-3 formula (3) | M7 formula (4) | M9 formula (5) |
| IC50 values | 12.32 µM | 7.425 µM | 1.841 µM | > 100 µM | 2.411 µM | 6.756 µM | 1.623 µM |
| | | | | | | | |

| Cell lines | CCRF-SB (acute lymphoblastic leukemia) | | | | | | |
|---|---|---|---|---|---|---|---|
| Compounds | M11 formula (6) | M12 formula (7) | M14 formula (8) | M15 formula (9) | M16 formula (10) | M18 formula (11) | M19 formula (12) |
| IC50 values | 7.824 µM | 0.954 µM | 0.287 µM | 0.357 µM | 0.198 µM | 9.18 µM | 18.23 µM |

### < Example 10: Investigation of cell death inducing effects of each compound on MEC-1 (chronic lymphocytic leukemia) cells >

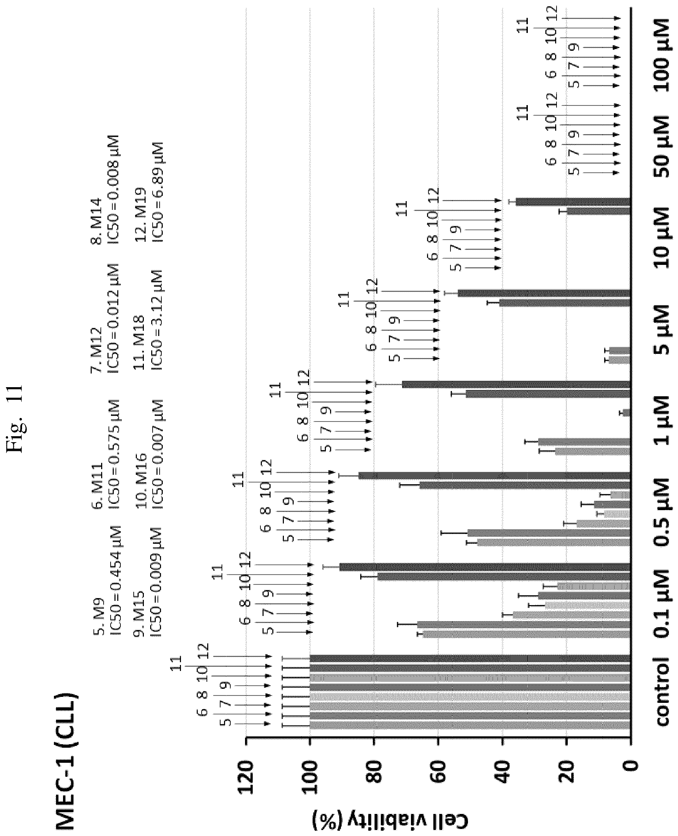

MEC-1 cells (chronic lymphocytic leukemia cell line) were cultured under 5% CO₂ at 37°C. The culture medium used RPMI-1640 medium with 100 µg/mL penicillin, 100 U/mL streptomycin and fetal bovine serum. MEC-1 cells were seeded into 96-well plates and incubated with each concentration of the compounds was added, and cell viability was determined by the trypan blue staining. The results are shown in Figure 11.

Referring to Figure 11, the horizontal axis shows the concentration of each compound and the vertical axis shows the cell viability (%). For controls, only 0.5% DMSO (diluted in PBS) used to dissolve the compounds was added. For each concentration, the codes "5, 6, 7, 8, 9, 10, 11, 12" and arrows are shown from left to right. The sign "5" is the M9 (formula (5)) administration group, the sign "6" is the M11 (formula (6)) administration group, the sign "7" is the M12 (formula (7)) administration group, the sign "8" is the M14 (formula (8)) administration group, the sign "9" is the M15 (formula (9)) administration group, the sign "10" is the M16 (formula (10)) administration group, the sign "11" is the M18 (formula (11)) administration group, the sign "12" is the M19 (formula (12)) administration group, respectively. The IC50 values for each compound is also listed in the upper right corner of the figure.

A concentration-dependent decrease in cell viability was observed for each compound. Table 10 shows the results of IC50 values calculated.

M9 (formula (5)) administration group, M11 (formula (6)) administration group, M12 (formula (7)) administration group, M14 (formula (8)) administration group, M15 (formula (9)) administration group, M16 (formula (10)) administration group, M18 (formula (11)) administration group, and M19 (formula (12)) administration group were induced cell death in chronic lymphocytic leukemia cell line MEC-1 cells at concentrations lower.

**[Table 10]**

| Cell lines | MEC-1(CLL) | | | | | | | |
|---|---|---|---|---|---|---|---|---|
| Compounds | M9 formula (5) | M11 formula (6) | M12 formula (7) | M14 formula (8) | M15 formula (9) | M16 formula (10) | M18 formula (11) | M19 formula (12) |
| IC50 values | 0.454 µM | 0.575 µM | 0.012 µM | 0.008 µM | 0.009 µM | 0.007 µM | 3.12 µM | 6.89 µM |

### < Example 11: Investigation of cell death inducing effects of each compound on HUT-78 (peripheral T lymphoma) cells >

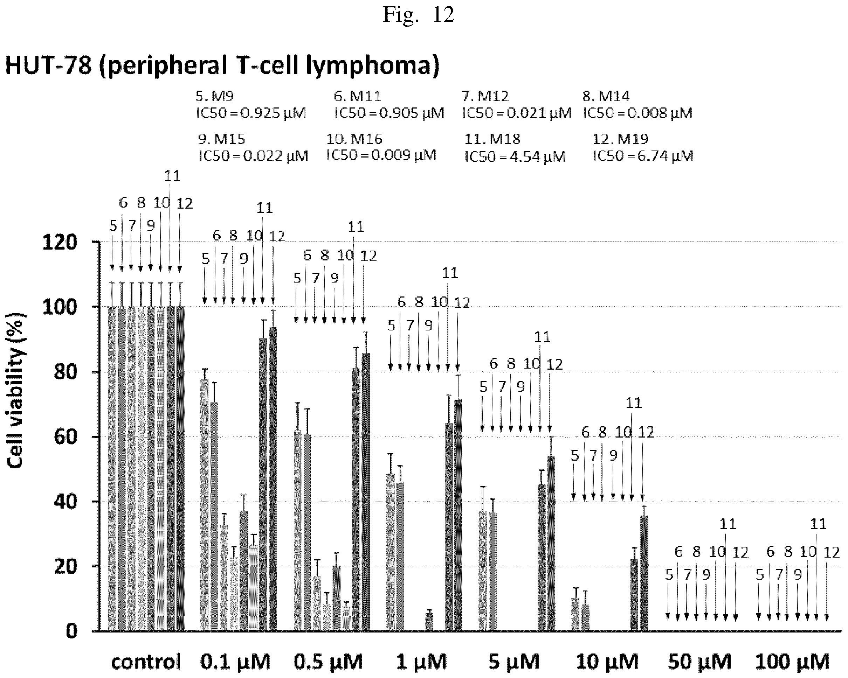

HUT-78 cells (peripheral T lymphoma cell line) were cultured under 5% CO₂ at 37°C. The culture medium used RPMI-1640 medium with 100 µg/mL penicillin, 100 U/mL streptomycin and fetal bovine serum. HUT-78 cells were seeded into 96-well plates and incubated with each concentration of the compounds was added, and cell viability was determined by the trypan blue staining. The results are shown in Figure 12.

Referring to Figure 12, the horizontal axis shows the concentration of each compound and the vertical axis shows the cell viability (%). For controls, only 0.5% DMSO (diluted in PBS) used to dissolve the compounds was added. For each concentration, the codes "5, 6, 7, 8, 9, 10, 11, 12" and arrows are shown from left to right. The sign "5" is the M9 (formula (5)) administration group, the sign "6" is the M11 (formula (6)) administration group, the sign "7" is the M12 (formula (7)) administration group, the sign "8" is the M14 (formula (8)) administration group, the sign "9" is the M15 (formula (9)) administration group, the sign "10" is the M16 (formula (10)) administration group, the sign "11" is the M18 (formula (11)) administration group, the sign "12" is the M19 (formula (12)) administration group, respectively. The IC50 values for each compound is also listed in the upper right corner of the figure.

A concentration-dependent decrease in cell viability was observed for each compound. Table 11 shows the results of IC50 values calculated.

M9 (formula (5)) administration group, M11 (formula (6)) administration group, M12 (formula (7)) administration group, M14 (formula (8)) administration group, M15 (formula (9)) administration group, M16 (formula (10)) administration group, M18 (formula (11)) administration group, and M19 (formula (12)) administration group were induced cell death in chronic lymphocytic leukemia cell line MEC-1 cells at concentrations lower.

**[Table 11]**

| Cell lines | HUT-78 (peripheral T-cell lymphoma) | | | | | | | |
|---|---|---|---|---|---|---|---|---|
| Compounds | M9 formula (5) | M11 formula (6) | M12 formula (7) | M14 formula (8) | M15 formula (9) | M16 formula (10) | M18 formula (11) | M19 formula (12) |
| IC50 values | 0.925 µM | 0.905 µM | 0.021 µM | 0.008 µM | 0.022 µM | 0.009 µM | 454 µM | 6.74 µM |

### < Example 12: Investigation of cell death inducing effects of each compound on ATN-1 (adult T cell leukemia) cells >

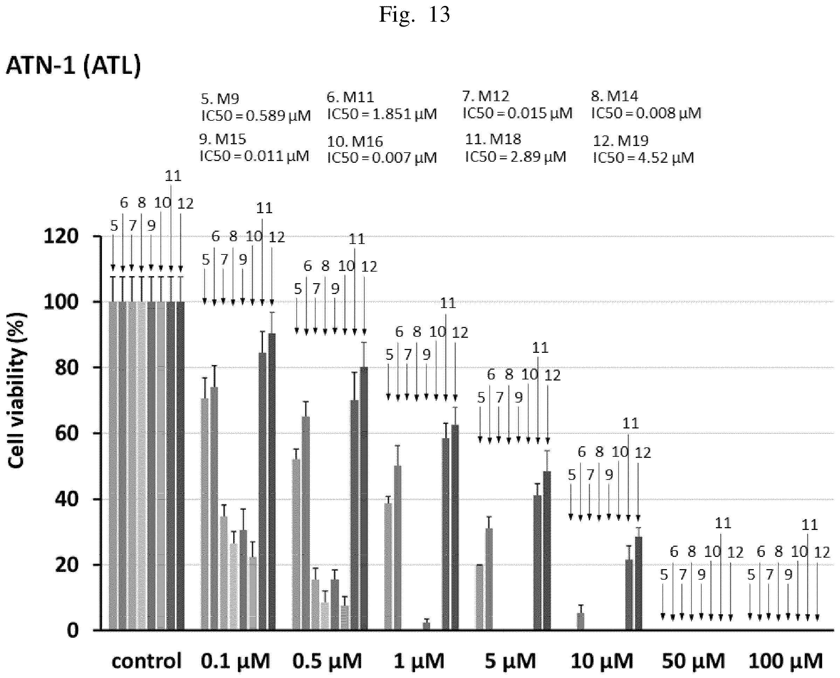

ATN-1 cells (adult T cell leukemia cell line) were cultured under 5% CO₂ at 37°C. The culture medium used RPMI-1640 medium with 100 µg/mL penicillin, 100 U/mL streptomycin and fetal bovine serum. ATN-1 cells were seeded into 96-well plates and incubated with each concentration of the compounds was added, and cell viability was determined by the trypan blue staining. The results are shown in Figure 13.

Referring to Figure 13, the horizontal axis shows the concentration of each compound and the vertical axis shows the cell viability (%). For controls, only 0.5% DMSO (diluted in PBS) used to dissolve the compounds was added. For each concentration, the codes "5, 6, 7, 8, 9, 10, 11, 12" and arrows are shown from left to right. The sign "5" is the M9 (formula (5)) administration group, the sign "6" is the M11 (formula (6)) administration group, the sign "7" is the M12 (formula (7)) administration group, the sign "8" is the M14 (formula (8)) administration group, the sign "9" is the M15 (formula (9)) administration group, the sign "10" is the M16 (formula (10)) administration group, the sign "11" is the M18 (formula (11)) administration group, the sign "12" is the M19 (formula (12)) administration group, respectively. The IC50 values for each compound is also listed in the upper right corner of the figure.

A concentration-dependent decrease in cell viability was observed for each compound. Table 12 shows the results of IC50 values calculated.

M9 (formula (5)) administration group, M11 (formula (6)) administration group, M12 (formula (7)) administration group, M14 (formula (8)) administration group, M15 (formula (9)) administration group, M16 (formula (10)) administration group, M18 (formula (11)) administration group, and M19 (formula (12)) administration group were induced cell death in adult T cell leukemia cell line ATN-1 cells at concentrations lower.

**[Table 12]**

| Cell lines | ATN-1 (ATL) | | | | | | | |
|---|---|---|---|---|---|---|---|---|
| Compounds | M9 formula (5) | M11 formula (6) | M12 formula (7) | M14 formula (8) | M15 formula (9) | M16 formula (10) | M18 formula (11) | M19 formula (12) |
| IC50 values | 0.589 µM | 1.851 µM | 0.015 µM | 0.008 µM | 0.011 µM | 0.007 µM | 2.89 µM | 4.52 µM |

### < Example 13: Investigation of cell death inducing effects of each compound on RPMI1788 Cells >

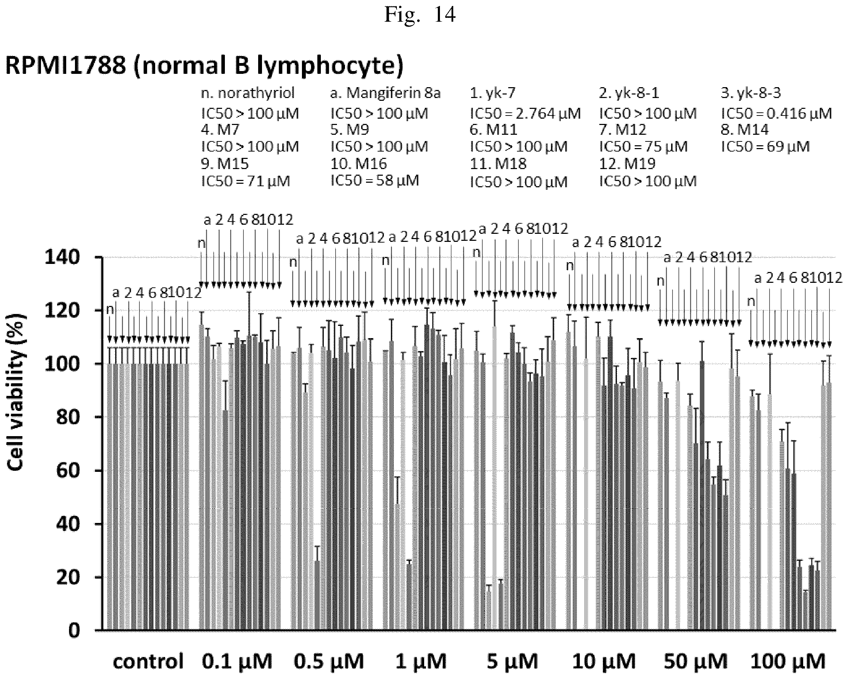

RPMI1788 cells (human normal B cell line) were cultured under 5% CO₂ at 37°C. The culture medium used RPMI-1640 medium with 100 µg/mL penicillin, 100 U/mL streptomycin and fetal bovine serum. RPMI1788 cells were seeded into 96-well plates and incubated with each concentration of the compounds was added, and cell viability was determined by the trypan blue staining. The results are shown in Figure 14.

Referring to Figure 14, the horizontal axis shows the concentration of each compound and the vertical axis shows the cell viability (%). For controls, only 0.5% DMSO (diluted in PBS) used to dissolve the compounds was added. For each concentration, each bar line is indicated by an arrow with the sign "n, a, 1, 2, 3, 4, 5, 6, 7, 8, 9, 10, 11, 12" from left to right. The "1, 3, 5, 7, 9, 11" are omitted from the graph, but the sign "1" shall be indicated by an arrow between the signs "a" and "2", the sign "3" between the signs "2" and "4", and other odd numbers shall likewise be indicated by arrows between even numbers before and after.

The sign "n" is the norathyriol (formula (n)) administration group, the sign "a" is the mangiferin 8a (formula (a)) administration group, the sign "1" is the yk-7 (formula (1)) administration group, the sign "2" is the yk-8-1 (formula (2)) administration group, the sign "3" is the yk-8-3 (formula (3)) administration group, the sign "4" is the M7 (formula (4)) administration group, the sign "5" is the M9 (formula (5)) administration group, the sign "6" is the M11 (formula (6)) administration group, the sign "7" is the M12 (formula (7)) administration group, the sign "8" is the M14 (formula (8)) administration group, the sign "9" is the M15 (formula (9)) administration group, the sign "10" is the M16 (formula (10)) administration group, the sign "11" is the M18 (formula (11)) administration group, the sign "12" is the M19 (formula (12)) administration group, respectively. The IC50 values for each compound is also listed in the upper right corner of the figure.

A concentration-dependent decrease in cell viability was observed for each compound. Table 13 shows the results of IC50 values calculated.

The yk-7 (formula (1)) administration group and yk-8-3 (formula (3)) administration group had lower IC50 values, but the other compounds administration group, including the norathyriol (formula (n)) administration group and mangiferin 8a (formula (a)) administration group shown as comparative examples, showed no decrease in cell viability in normal B cells even at higher concentrations. In other words, it was recognized that the compounds of the present invention could be a therapeutic agent with fewer side effects for lymphoma and myeloma.

**[Table 13]**

| Cell lines | RPMI1788(normal B lymphocyte) | | | | | | |
|---|---|---|---|---|---|---|---|
| Compounds | norathyriol formula (n) | 8a formula (a) | yk-7 formula (1) | yk-8-1 formula (2) | yk-8-3 formula (3) | M7 formula (4) | M9 formula (5) |
| IC50 values | > 100µM | > 100 µM | 2.764 µM | > 100 µM | 0.416 µM | > 100 µM | > 100 µM |
| | | | | | | | |

| Cell lines | RPMI1788(normal B lymphocyte) | | | | | | |
|---|---|---|---|---|---|---|---|
| Compounds | M11 formula (6) | M12 formula (7) | M14 formula (8) | M15 formula (9) | M16 formula (10) | M18 formula (11) | M19 formula (12) |
| IC50 values | > 100 µM | 75 µM | 69 µM | 71 µM | 58 µM | > 100 µM | > 100 µM |

### < Example 14: Investigation of the effect of each compound administration on the signaling system NIK activation inhibition >

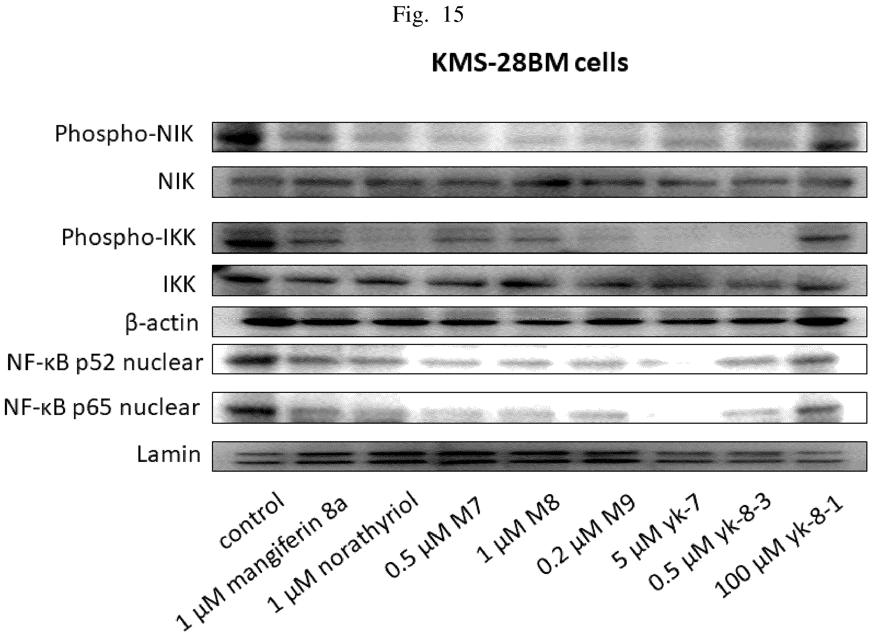

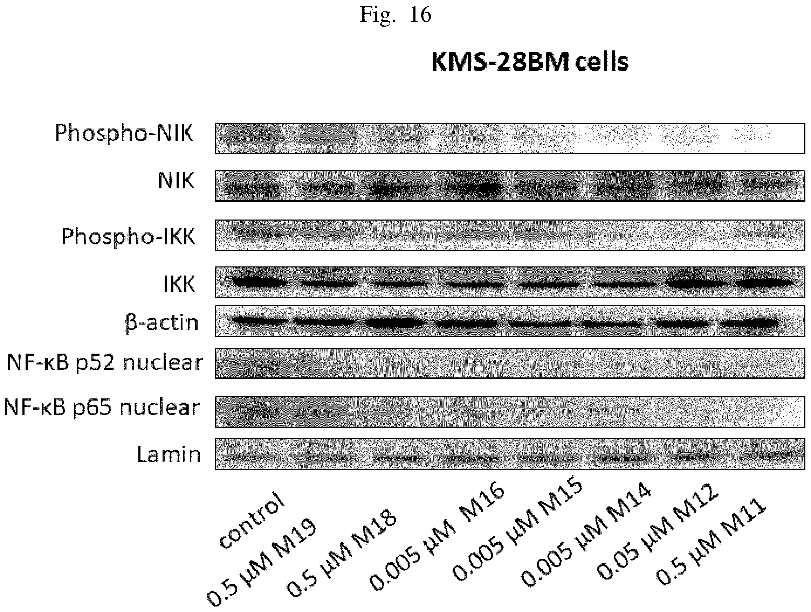

Immunoblotting was performed on KMS-28BM cells to investigate the inhibitory effect of each compound administration on NIK inhibition and the activation of IKK, NF-κB p52, and NF-κB p65, which are downstream signals of NIK. As a result, inhibition of NIK activation, inhibition of IKK activity, and inhibition of nuclear translocation of NF-κB p52 and NF-κB p65 were observed (Figure 15 and Figure 16).

As a control, compounds untreated KMS-28BM cells were seeded in 150 cm² flasks and incubated for 72 h at 37 °C and 5% CO₂. KMS-28BM cells were seeded into 150 cm² flasks and pre-cultured for 24 h. After incubation, KMS-28BM cells were treated with 1 µM mangiferin 8a, 1 µM norathyriol, 0.5 µM M7 (formula (4)), 1 µM M8 (mangiferin 8a: listed in duplicate), 0.2 µM M9 (formula (5)), 5 µM yk-7 (formula (1)), 0.5 µM yk-8 -3 (formula (3)) and 100 µM yk-8-1 (formula (2)) at final concentrations, and incubated for 72 h at 37 °C and 5% CO₂. In addition, KMS-28BM cells were also treated with 0.5 µM M19 (formula (12)), 0.5 µM M18 (formula (11)), 0.005 µM M16 (formula (10)), 0.005 µM M15 (formula (9)), 0.005 µM M14 (formula (8)), 0.05 µM M12 (formula (7)) and 0.5 µM M11 (formula (6)) at the final concentrations, and incubated for 72 h at 37 °C and 5% CO₂. Proteins were extracted from these cell suspensions with cell lysate and used as samples.

The nucleus and cytoplasmic substrate were separated from the cells using the ProteoExtract (registered trademark) Subcellular Proteome Extraction Kit from Merck Ltd. to extract the cytoplasmic and nuclear fractions.

After SDS-PAGE, each sample was transferred to a PVDF membrane and examined using anti-phospho-NIK, anti-NIK, anti-phospho-IKK, anti-IKK, anti-β-actin, anti-NF-κB p52 and anti-NF-κB p65 antibody, and anti-Lamin antibody.

Immunoblotting results are shown in Figures 15 and 16. In Figure 15, the horizontal direction of the picture shows control, administration of 1 µM mangiferin 8a, administration of 1 µM norathyriol, administration of 0.5 µM M7 (formula (4)), administration of 1 µM M8 (mangiferin 8a: duplicate listing), administration of 0.2 µM M9 (formula (5)), administration of 5 µM yk-7 (formula (1)), administration of 0.5 µM yk -8-3 (formula (3)), and administration of 100 µM yk-8-1 (formula (2)) were lined up. In Figure 16, the horizontal direction of the picture shows control, administration of 0.5 µM M19 (formula (12)), administration of 0.5 µM M18 (formula (11)), administration of 0.005 µM M16 (formula (10)), administration of 0.005 µM M15 (formula (9)), administration of 0.005 µM M14 (formula (8)), administration of 0.05 µM M12 (formula (7)), and administration of 0.5 µM M11 (formula (6)) were lined up.

The antibody species is indicated in the vertical direction. Specifically, for anti-phospho-NIK antibody, "Phospho-NIK" was used, for anti-NIK antibody, "NIK" was used, for anti-phospho-IKK antibody, "Phospho-IKK" was used, for anti-IKK antibody, "IKK" was used, for anti-β-actin antibody, " *β* -actin" was used, for anti-NF-κB p52 antibody, "NF-κB p52 nuclear" was used, for anti-NF-κB p65 antibody, "NF-κB p65 nuclear" was used, and for anti-Lamin antibody, "Lamin" was used.

No decrease in the density of the NIK bands was observed in the anti-NIK antibody pictures compared to the control. On the other hand, in the case of anti-phospho-NIK antibody, administrations of mangiferin 8a, norathyriol, M7 (formula (4)), M8 (mangiferin 8a: duplicate listing), M9 (formula (5)), yk-7 (formula (1)), yk-8 -3 (formula (3)), yk-8-1 (formula (2)), M19 (formula (12)), M18 (formula (11)), M16 (formula (10)), M15 (formula (9)), M14 (formula (8)), M12 (formula (7)), and M11 (formula (6)) results showed a decrease in band density.

No decrease in the density of the IKK bands was observed in the anti-IKK antibody pictures compared to the control. On the other hand, in the case of anti-phospho-IKK antibody, administrations of mangiferin 8a, norathyriol, M7 (formula (4)), M8 (mangiferin 8a: duplicate listing), M9 (formula (5)), yk-7 (formula (1)), yk-8 -3 (formula (3)), yk-8-1 (formula (2)), M19 (formula (12)), M18 (formula (11)), M16 (formula (10)), M15 (formula (9)), M14 (formula (8)), M12 (formula (7)), and M11 (formula (6)) results showed a decrease in band density.

Next, anti-NF-κB p52 antibody (described as "NF-κB p52 nuclear"), anti-NF-κB p65 antibody (described as "NF-κB p65 nuclear") and anti-Lamin antibody (described as "Lamin") against cell nuclear substance. Lamin is present in all compounds. However, NF-κB p52 nuclear is decreased in administrations of mangiferin 8a, norathyriol, M7 (formula (4)), M8 (mangiferin 8a: duplicate listing), M9 (formula (5)), yk-7 (formula (1)), yk -8-3 (formula (3)), yk-8-1 (formula (2)), M19 (formula (12)), M18 (formula (11)), M16 (formula (10)), M15 (formula (9)), M14 (formula (8)), M12 (formula (7)), and M11 (formula (8)). NF-κB p65 nuclear was also reduced (shaded) in administrations of all compounds.

Lamin is a fibrous protein that maintains structure and regulates transcription in the cell nucleus. Therefore, with all samples detecting nuclear material, the addition of each compound indicates that NF-κB p65 nuclear and NF-κB p52 nuclear are not present or, if present, very low.

### < Example 15: Inhibition of CD138 expression by administration of each compound in KMS-28BM cells >

Flow cytometry was used to examine the inhibitory effect of each compound on CD138 expression in KMS-28BM cells, and the results showed that CD138 expression was inhibited.

As a control, compounds untreated KMS-28BM cells were seeded in 75 cm² flasks and incubated for 10 days at 37 °C and 5% CO₂. KMS-28BM cells were seeded into 75 cm² flasks and pre-cultured for 4 h. After incubation, KMS-28BM cells were treated with 0.05 µM M7, 0.05 µM M9, 0.1 µM yk-7, 0.1 µM yk-8-3, 50 µM yk-8-1, 0.5 µM M11, 0.05 µM M12, 0.005 µM M14, 0.005 µM M15, 0.005 µM M16, 0.5 µM M18, and 0.5 µM M19 at final concentrations, and incubated for 10 days at 37 °C and 5% CO₂. This added amount is a concentration lower than the IC50 of the above compounds for KMS-28BM cells. After 10 days of incubation, cells were stained with anti-CD138 antibody, a marker of multiple myeloma grade, and CD138 expression was measured using BD LSRFortessa.

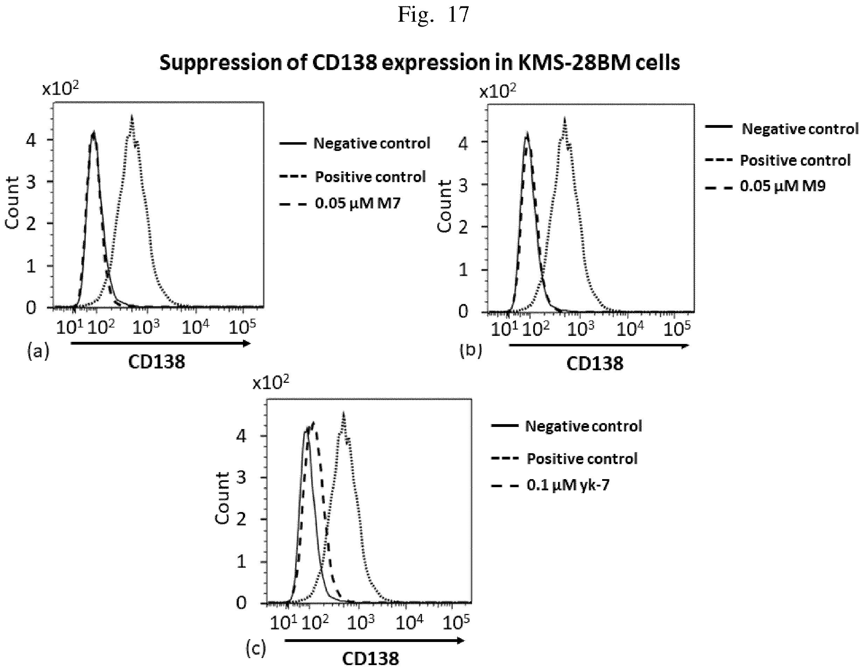

Results are shown in Figure 17, Figure 18, Figure 19 and Figure 20. The horizontal axis represents the expression level of CD138 and the vertical axis represents the number of cells. The solid line in the panel shows the negative control treated with isotype control antibody without compounds, the dotted line shows the positive control treated with anti-CD138 antibody without compounds, and the dashed line shows those treated with compounds and anti-CD138 antibody. Compared to the negative control group, which was not treated with the compounds and treated with isotype control antibody, CD138 expression was significantly increased in the positive control.

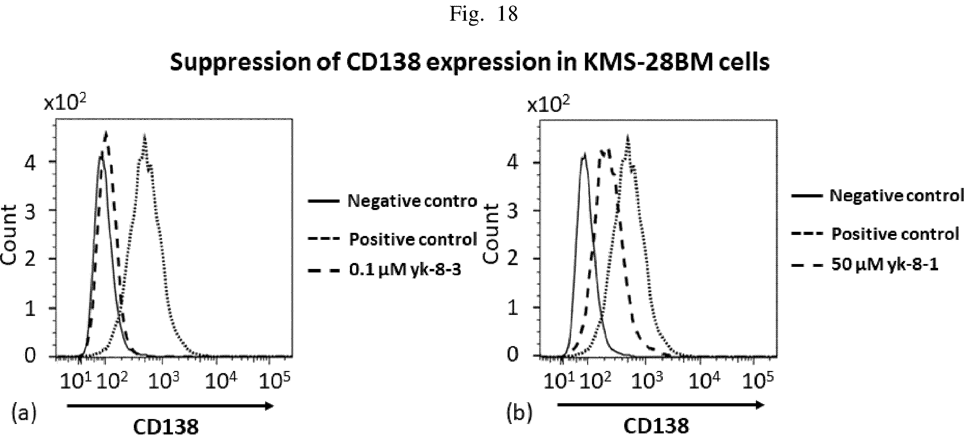

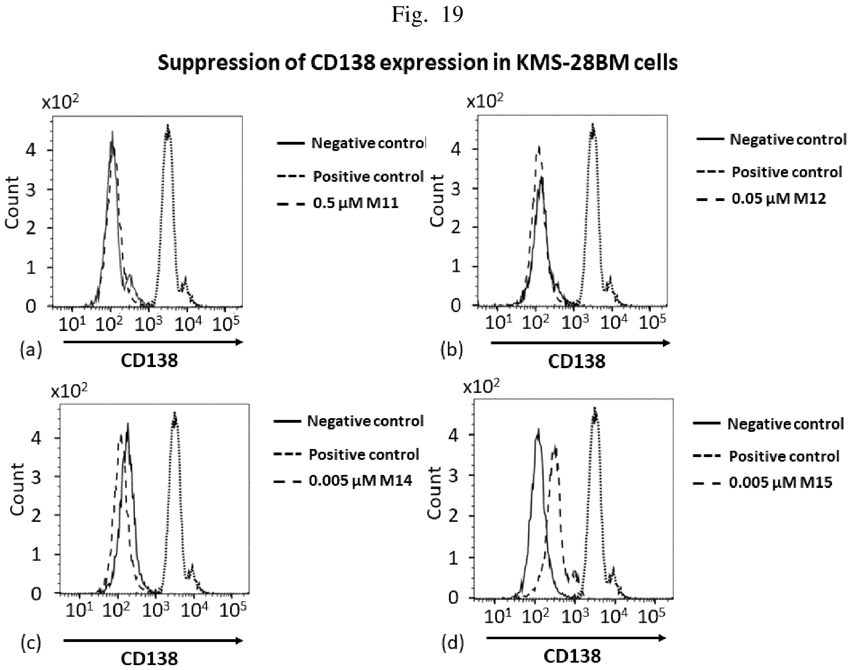

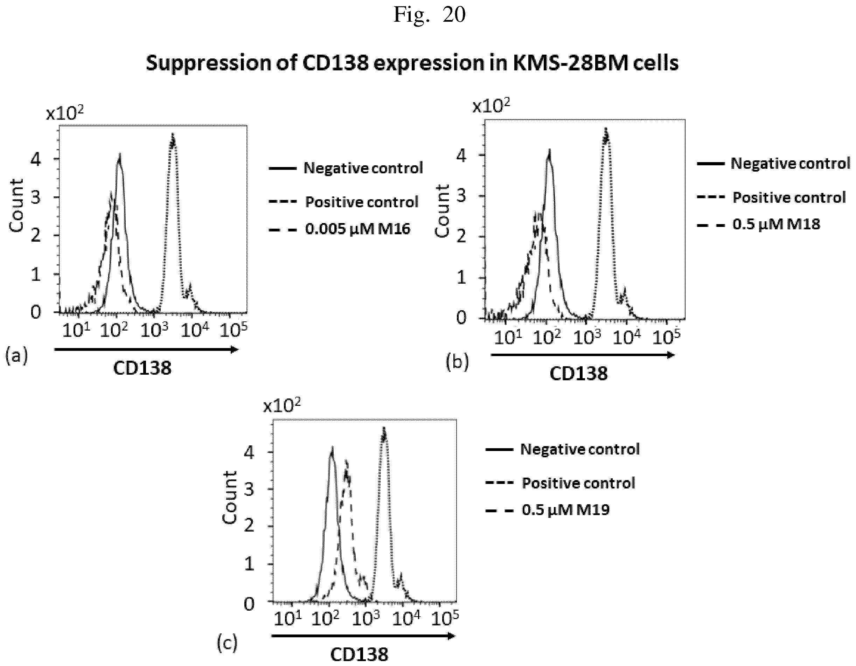

Compared to the positive control, the CD138 expression levels in the M7 administration group (Figure 17(a)), M9 administration group (Figure 17(b)), yk-7 administration group (Figure 17(c)), yk-8-3 administration group (Figure 18(a)), yk-8-1 administration group (Figure 18(b)), M11 administration group (Figure 19(a)), M12 administration group (Figure 19(b)), M14 administration group (Figure 19(c)), M15 administration group (Figure 19(d)), M16 administration group (Figure 20(a)), M18 administration group (Figure 20(b)), and M19 administration group (Figure 20(c)) were markedly reduced and almost the same as in the negative control. In other words, M7, M9, yk-7, yk-8-3, yk-8-1, M11, M12, M14, M15, M16, M18, and M19 were found to suppress CD138 expression, a marker of multiple myeloma grade.

### < Example 16: Increased CD20 expression by administration of each compound in KMS-28BM cells >

Flow cytometry was used to examine the effects of each compound administration with CD20 expression in KMS-28BM cells, which showed an increase in CD20 expression.

As a control, compounds untreated KMS-28BM cells were seeded in 75 cm² flasks and incubated for 10 days at 37 °C and 5% CO₂. KMS-28BM cells were seeded into 75 cm² flasks and pre-cultured for 4 h. After incubation, KMS-28BM cells were treated with 0.05 µM M7, 0.05 µM M9, 0.1 µM yk-7, 0.1 µM yk-8-3, 50 µM yk-8-1, 0.5 µM M11, 0.05 µM M12, 0.005 µM M14, 0.005 µM M15, 0.005 µM M16, 0.5 µM M18, and 0.5 µM M19 at final concentrations, and incubated for 10 days at 37 °C and 5% CO₂. This added amount is a concentration lower than the IC50 of the above compounds for KMS-28BM cells. After 10 days of incubation, cells were stained with anti-CD20 antibody, a B cell marker, and CD20 expression was measured using BD LSRFortessa. CCRF-SB cells were used as CD20-positive controls.

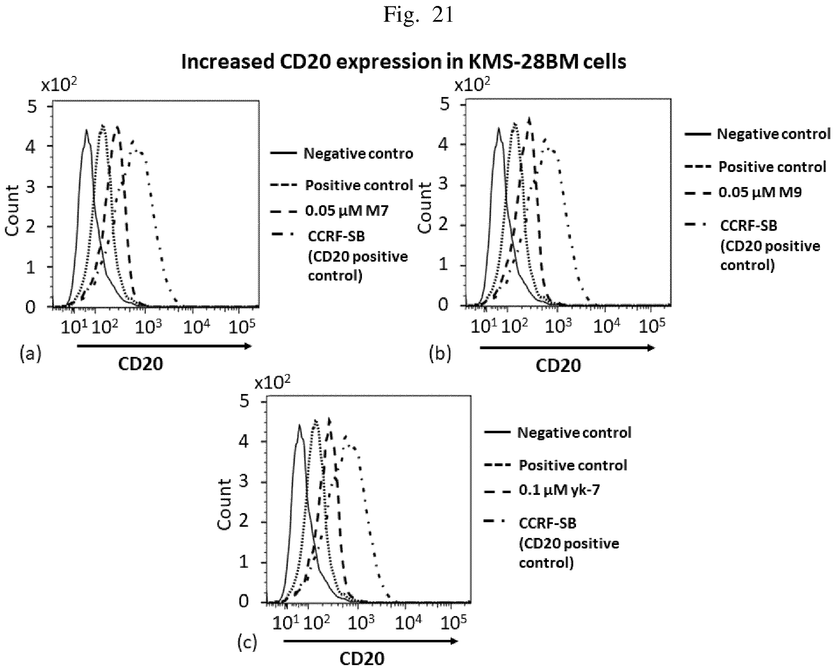

Results are shown in Figure 21, Figure 22, Figure 23, and Figure 24. The horizontal axis represents the expression level of CD20 and the vertical axis represents the number of cells. The solid line in the panel shows the negative control treated with isotype control antibody without compounds, the dotted line shows the positive control treated with anti-CD20 antibody without compounds, and the dashed line shows those treated with compounds and anti-CD20 antibody. Single-dotted line shows CCRF-SB cells (leukemia, acute B-lymphocytic leukemia cells) treated with anti-CD20 antibody.

Compared to the negative control group treated with isotype control, CD20 expression was hardly increased in the positive control and was comparable to the negative control. This means that KMS-28BM cells do not express CD20.

On the other hand, CD20 expression was significantly increased in CCRF-SB cells used as CD20-positive controls compared to the negative and positive controls of KMS-28BM cells. This indicates that CCRF-SB cells express CD20.

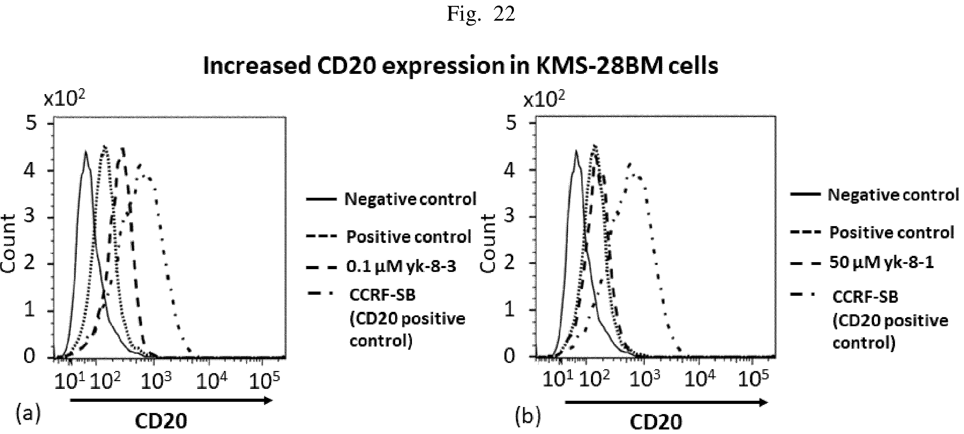

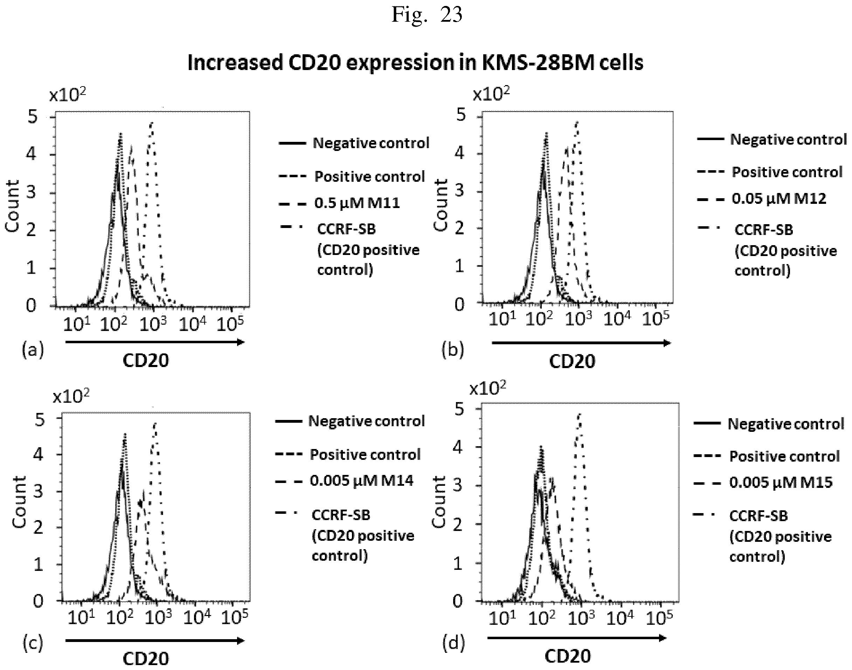

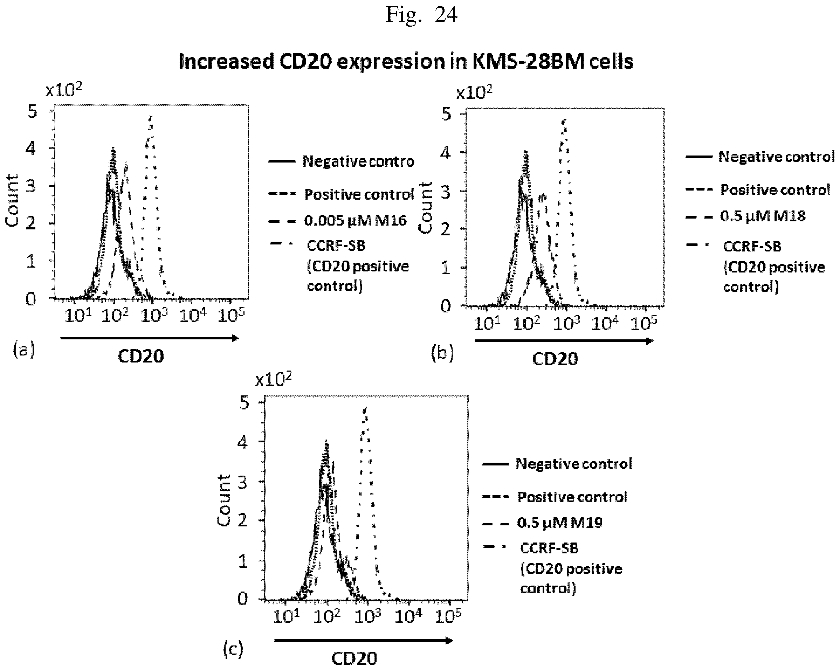

In addition, the CD20 expression level was significantly increased in the M7 administration group (Figure 21(a)), M9 administration group (Figure 21(b)), yk-7 administration group (Figure 21(c)), yk-8-3 administration group (Figure 22(a)), yk-8 -1 administration group (Figure 22(b)), M11 administration group (Figure 23(a)), M12 administration group (Figure 23(b)), M14 administration group (Figure 23(c)), M15 administration group (Figure 23(d)), M16 administration group (Figure 24(a)), M18 administration group (Figure 24(b)), and M19 administration group (Figure 24(c)), as compared to the positive control. In other words, M7, M9, yk-7, yk-8-3, yk-8-1, M11, M12, M14, M15, M16, M18, and M19 were found to increase CD20 expression, a B cell marker, and transform multiple myeloma cells to B cell-like.

### < Example 17: Inhibition of CD138 expression by administration of each compound in L363 cells >

Flow cytometry was used to examine the inhibitory effect of each compound on CD138 expression in L363 cells, and the results showed that CD138 expression was inhibited.

As a control, compounds untreated L363 cells were seeded in 75 cm² flasks and incubated for 10 days at 37 °C and 5% CO₂. L363 cells were seeded into 75 cm² flasks and pre-cultured for 4 h. After incubation, L363 cells were treated with 0.1 µM M7, 0.05 µM M9, 0.1 µM yk-7, 0.1 µM yk-8-3, 50 µM yk-8-1, 0.1 µM M11, 0.01 µM M12, 0.005 µM M14, 0.005 µM M15, 0.005 µM M16, 1 µM M18, and 1 µM M19 at final concentrations, and incubated for 10 days at 37 °C and 5% CO₂. This added amount is a concentration lower than the IC50 of the above compounds for L363 cells. After 10 days of incubation, cells were stained with anti-CD138 antibody, a marker of multiple myeloma grade, and CD138 expression was measured using BD LSRFortessa.

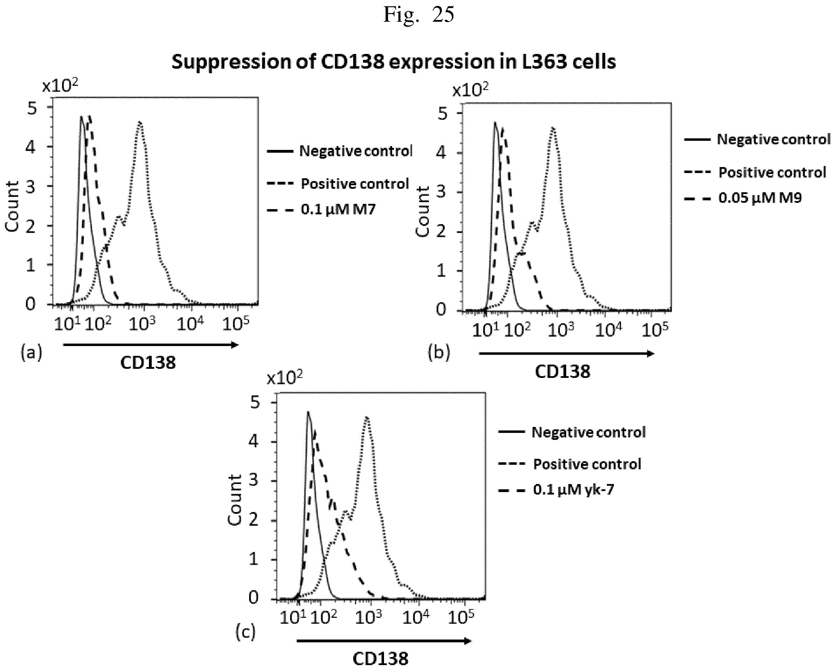

Results are shown in Figure 25, Figure 26, Figure 27 and Figure 28. The horizontal axis represents the expression level of CD138 and the vertical axis represents the number of cells. The solid line in the panel shows the negative control treated with isotype control antibody without compounds, the dotted line shows the positive control treated with anti-CD138 antibody without compounds, and the dashed line shows those treated with compounds and anti-CD138 antibody. Compared to the negative control group, which was not treated with the compounds and treated with isotype control antibody, CD138 expression was significantly increased in the positive control.

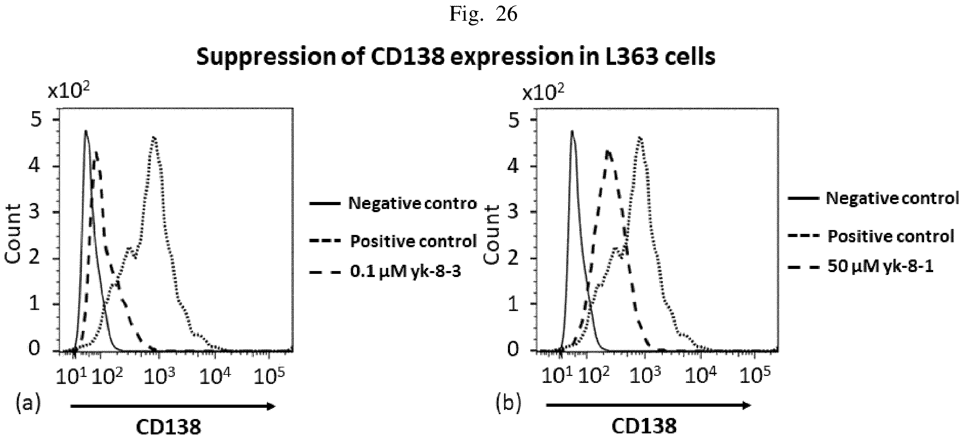

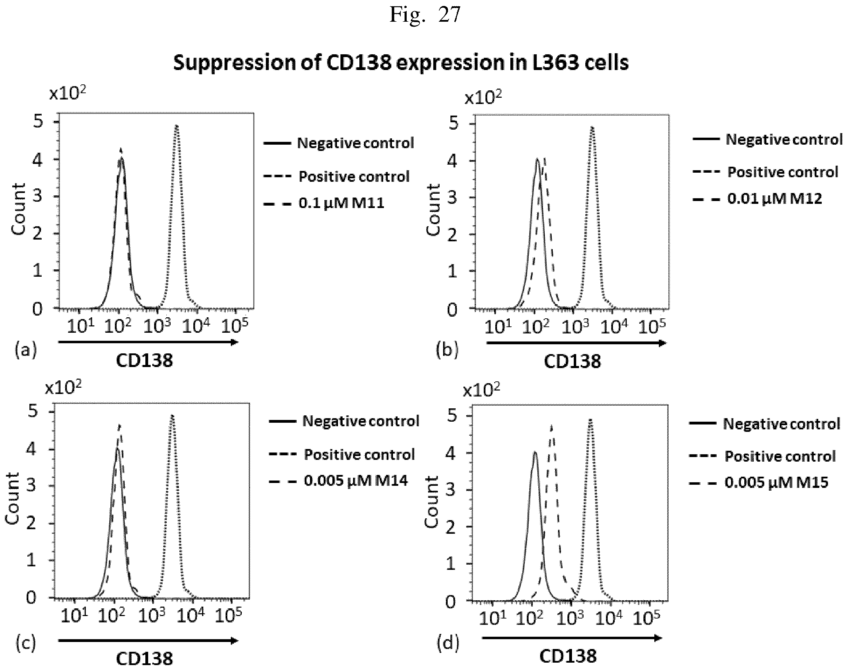

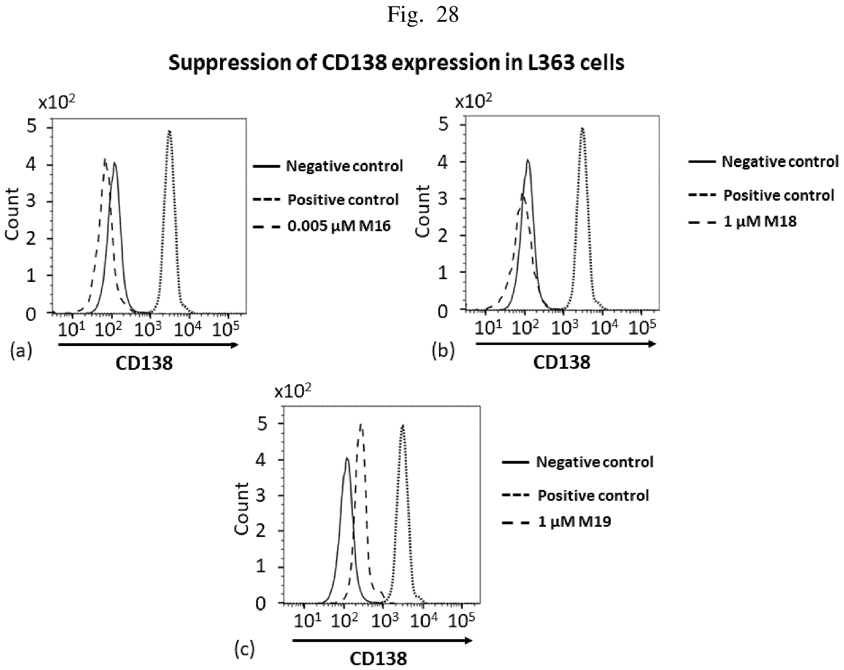

Compared to the positive control, the CD138 expression levels in the M7 administration group (Figure 25(a)), M9 administration group (Figure 25(b)), yk-7 administration group (Figure 25(c)), yk-8-3 administration group (Figure 26(a)), yk-8-1 administration group (Figure 26(b)), M11 administration group (Figure 27(a)), M12 administration group (Figure 27(b)), M14 administration group (Figure 27(c)), M15 administration group (Figure 27(d)), M16 administration group (Figure 28(a)), M18 administration group (Figure 28(b)), and M19 administration group (Figure 28(c)) were markedly reduced and almost the same as in the negative control. In other words, M7, M9, yk-7, yk-8-3, yk-8-1, M11, M12, M14, M15, M16, M18, and M19 were found to suppress CD138 expression, a marker of multiple myeloma grade.

### < Example 18: Increased CD20 expression by administration of each compound in L363 cells >

Flow cytometry was used to examine the effects of each compound administration with CD20 expression in L363 cells, which showed an increase in CD20 expression.

As a control, compounds untreated L363 cells were seeded in 75 cm² flasks and incubated for 10 days at 37 °C and 5% CO₂. L363 cells were seeded into 75 cm² flasks and pre-cultured for 4 h. After incubation, L363 cells were treated with 0.1 µM M7, 0.05 µM M9, 0.1 µM yk-7, 0.1 µM yk-8-3, 50 µM yk-8-1, 0.1 µM M11, 0.01 µM M12, 0.005 µM M14, 0.005 µM M15, 0.005 µM M16, 1 µM M18, and 1 µM M19 at final concentrations, and incubated for 10 days at 37 °C and 5% CO₂. This added amount is a concentration lower than the IC50 of the above compounds for L363 cells. After 10 days of incubation, cells were stained with anti-CD20 antibody, a B cell marker, and CD20 expression was measured using BD LSRFortessa. CCRF-SB cells were used as CD20-positive controls.

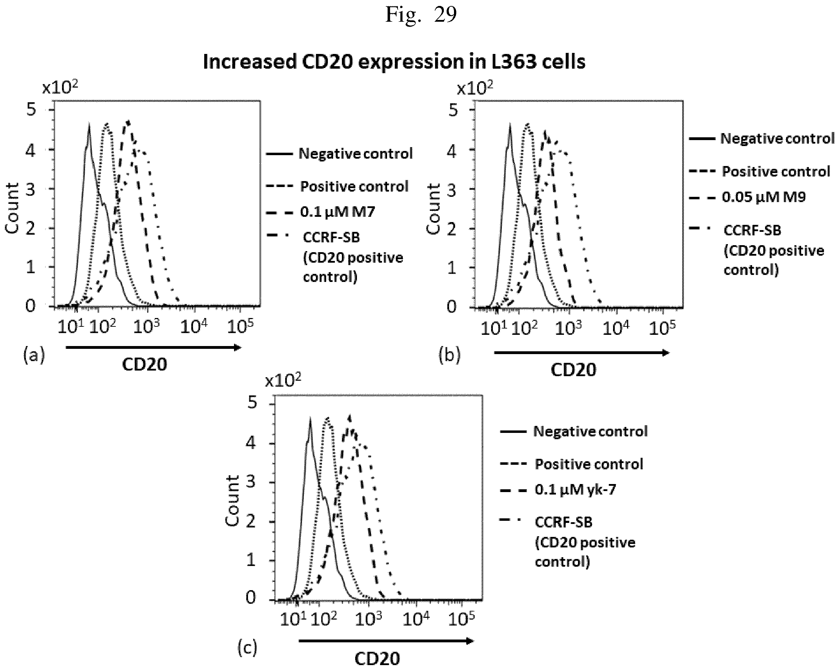

Results are shown in Figure 29, Figure 30, Figure 31, and Figure 32. The horizontal axis represents the expression level of CD20 and the vertical axis represents the number of cells. The solid line in the panel shows the negative control treated with isotype control antibody without compounds, the dotted line shows the positive control treated with anti-CD20 antibody without compounds, and the dashed line shows those treated with compounds and anti-CD20 antibody. Single-dotted line shows CCRF-SB cells (leukemia, acute B-lymphocytic leukemia cells) treated with anti-CD20 antibody.

Compared to the negative control group treated with isotype control, CD20 expression was hardly increased in the positive control and was comparable to the negative control. This means that L363 cells do not express CD20.

On the other hand, CD20 expression was significantly increased in CCRF-SB cells used as CD20-positive controls compared to the negative and positive controls of L363 cells.

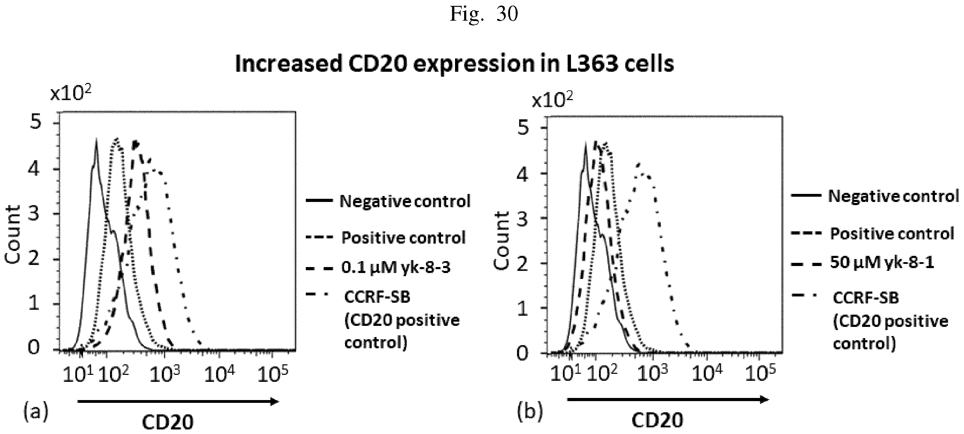

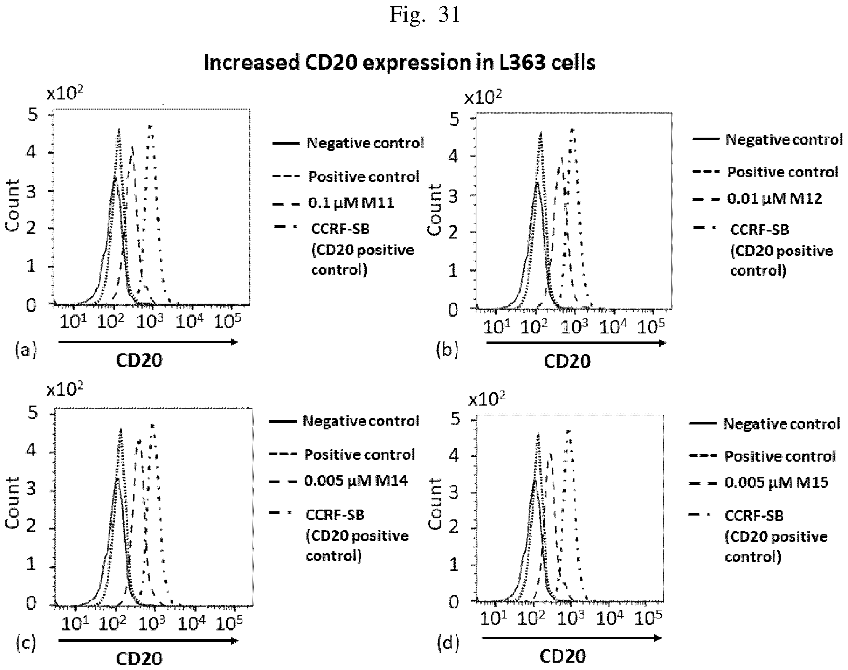

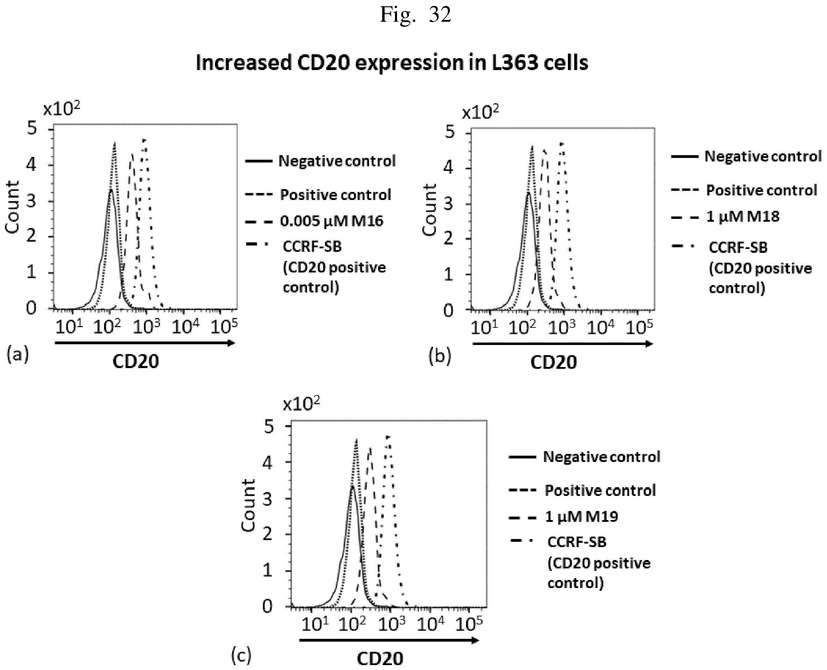

This indicates that CCRF-SB cells express CD20. In addition, the CD20 expression level was significantly increased in the M7 administration group (Figure 29(a)), M9 administration group (Figure 29(b)), yk-7 administration group (Figure 29(c)), yk-8-3 administration group (Figure 30(a)), yk-8 -1 administration group (Figure 30(b)), M11 administration group (Figure 31(a)), M12 administration group (Figure 31(b)), M14 administration group (Figure 31(c)), M15 administration group (Figure 31(d)), M16 administration group (Figure 32(a)), M18 administration group (Figure 32(b)), and M19 administration group (Figure 32(c)), as compared to the positive control. In other words, M7, M9, yk-7, yk-8-3, yk-8-1, M11, M12, M14, M15, M16, M18, and M19 were found to increase CD20 expression, a B cell marker, and transform multiple myeloma cells to B cell-like.

### < Example 19: Induction of cell death in KMS-28BM cells via induction of CD20 expression by administration with each compound in combination with rituximab >

After inducing increased CD20 expression in KMS-28BM cells with each compound, the cell death-inducing effect of each compound in combination with rituximab, an anti-CD20 monoclonal antibody, was examined, and the cell death-inducing effect was observed with each compound in combination with rituximab.

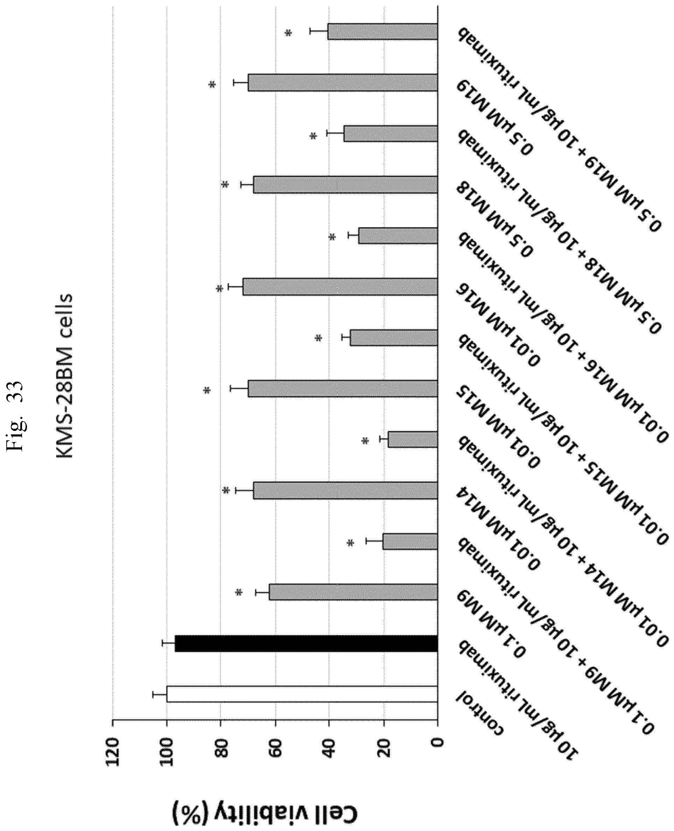

As a control, compounds untreated KMS-28BM cells were seeded in 75 cm² flasks and incubated for 10 days at 37 °C and 5% CO₂. KMS-28BM cells were seeded into 75 cm² flasks and pre-cultured for 24 h. After incubation, KMS-28BM cells were treated with 0.1 µM M9, 0.01 µM M14, 0.01 µM M15, 0.01 µM M16, 0.5 µM M18, and 0.5 µM M19 at final concentrations, and incubated for 10 days at 37 °C and 5% CO₂. This added amount is a concentration lower than the IC50 of the above compounds for KMS-28BM cells. After 10 days of incubation, cells were seeded into 96-well plates, human serum and 10 µg/mL rituximab were added, and cell viability was determined by the trypan blue staining. The results are shown in Figure 33.

Referring to Figure 33, the horizontal axis shows the concentration of each compound and the vertical axis shows the cell viability (%). For controls, only 0.5% DMSO (diluted in PBS) used to dissolve the compounds was added. Those that were significantly different (P<0.01) from the control in the target group are indicated with "*".

Compared to control, cell death was induced in the rituximab + M9 administration group, rituximab + M14 administration group, rituximab + M15 administration group, rituximab + M16 administration group, rituximab + M18 administration group, and rituximab + M19 administration group, but rituximab alone did not induce cell death. In addition, a significant decrease in cell viability was observed in the group treated with each compound in combination with rituximab compared to the rituximab alone and each compound alone.

### < Example 20: Induction of cell death in L363 cells via induction of CD20 expression by administration with each compound in combination with rituximab >

After inducing increased CD20 expression in L363 cells with each compound, the cell death-inducing effect of each compound in combination with rituximab, an anti-CD20 monoclonal antibody, was examined, and the cell death-inducing effect was observed with each compound in combination with rituximab.

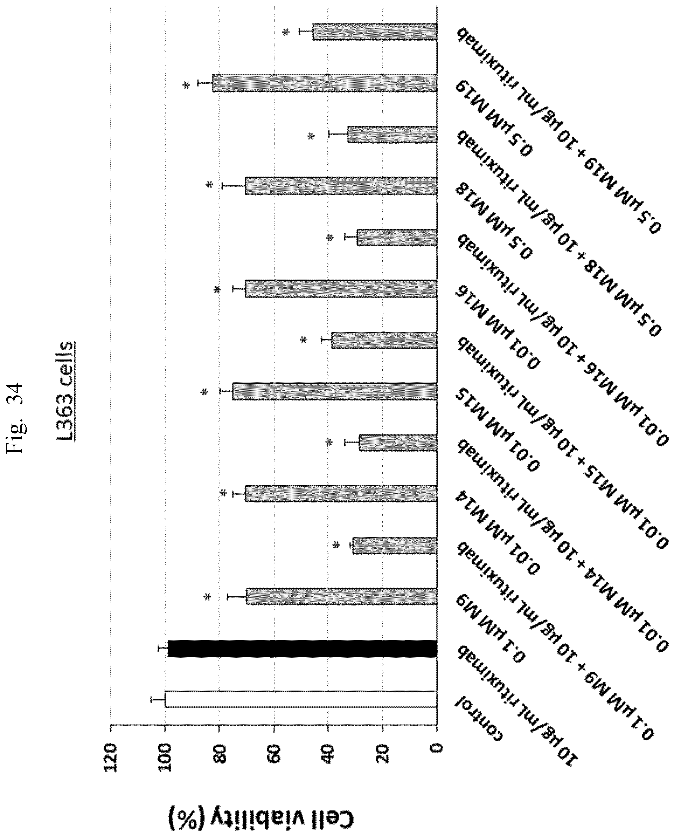

As a control, compounds untreated L363 cells were seeded in 75 cm² flasks and incubated for 10 days at 37 °C and 5% CO₂. L363 cells were seeded into 75 cm² flasks and pre-cultured for 24 h. After incubation, L363 cells were treated with 0.1 µM M9, 0.01 µM M14, 0.01 µM M15, 0.01 µM M16, 0.5 µM M18, and 0.5 µM M19 at final concentrations, and incubated for 10 days at 37 °C and 5% CO₂. This added amount is a concentration lower than the IC50 of the above compounds for L363 cells. After 10 days of incubation, cells were seeded into 96-well plates, human serum and 10 µg/mL rituximab were added, and cell viability was determined by the trypan blue staining. The results are shown in Figure 34.

Referring to Figure 34, the horizontal axis shows the concentration of each compound and the vertical axis shows the cell viability (%). For controls, only 0.5% DMSO (diluted in PBS) used to dissolve the compounds was added. Those that were significantly different (P<0.01) from the control in the target group are indicated with "*".

Compared to Control, cell death was induced in the rituximab + M9 administration group, rituximab + M14 administration group, rituximab + M15 administration group, rituximab + M16 administration group, rituximab + M18 administration group, and rituximab + M19 administration group. In addition, a significant decrease in cell viability was observed in the group treated with each compound in combination with rituximab compared to the rituximab alone and each compound alone.

The above experiments suggest that for both myeloma cells, KMS-28BM and L363 cells, CD138 decreased and CD20 increased, resulting in a transform to a B-cell-like (in this case, lymphoma) morphology. It was also observed that cell death induction was obtained with the rituximab combination via increasing CD20 expression. As is well known, anti-human CD20 monoclonal antibody drugs such as rituximab, obinutuzumab, ofatumumab, and ibritumomab tiuxetan are known as specific agents for lymphomas that express CD20. This means that the compounds can be administered as a treatment for multiple myeloma when mixed with anti-human CD20 monoclonal antibody drugs such as rituximab, obinutuzumab, ofatumumab, and ibritumomab tiuxetan.

### < Example 21: Inhibitory effect of each compound on IgG secretion in KMS-28BM cells >

The inhibitory effect of each compound on IgG secretion was examined by enzyme-linked immunosorbent assay (ELISA) using KMS-28BM cells. The each compound of the present invention inhibited IgG secretion in KMS-28BM cells.

As a control, compounds untreated KMS-28BM cells were seeded in 75 cm² flasks and incubated for 10 days at 37 °C and 5% CO₂. KMS-28BM cells were seeded into 75 cm² flasks and pre-cultured for 4 h. After incubation, KMS-28BM cells were treated with 0.05 µM M7, 0.05 µM M9, 0.1 µM yk-7, 0.1 µM yk-8-3, 50 µM yk-8-1, 0.5 µM M11, 0.05 µM M12, 0.005 µM M14, 0.005 µM M15, 0.005 µM M16, 0.5 µM M18, and 0.5 µM M19 at final concentrations, and incubated for 10 days at 37 °C and 5% CO₂. After 10 days of incubation, the culture supernatant was collected and IgG secretion was measured by ELISA. This measurement was performed using a human anti-IgG antibody ELISA kit (Funakoshi).

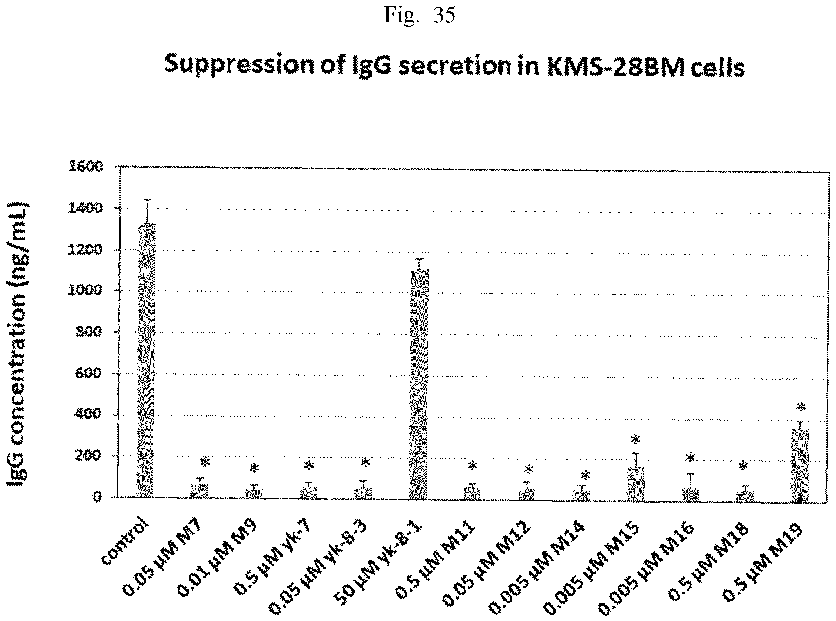

The results are shown in Figure 35. The horizontal axis represents the sample group and the vertical axis represents IgG secretion (ng/mL). In control, IgG is 1326 ng/mL, whereas in the M7 administration group, M9 administration group, yk-7 administration group, yk-8-3 administration group, yk-8-1 administration group, M11 administration group, M12 administration group, M14 administration group, M15 administration group, M16 administration group, M18 administration group, and M19 administration group, 67 ng/mL, 44 ng/mL, 56 ng/mL, 56 ng/mL, 1117 ng/mL, 61 ng/mL, 56 ng/mL, 50 ng/mL, 164 ng/mL, 67 ng/mL, 56 ng/mL, and 356 ng/mL, respectively.

As described above, M7 administration group, M9 administration group, yk-7 administration group, yk-8-3 administration group, yk-8-1 administration group, M11 administration group, M12 administration group, M14 administration group, M15 administration group, M16 administration group, M18 administration group, and M19 administration group significantly suppressed IgG secretion. In other words, the control group caused renal damage, amyloidosis, and hyperviscosity syndrome due to IgG production, while the M7 administration group, M9 administration group, yk-7 administration group, yk-8-3 administration group, yk-8-1 administration group, M11 administration group, M12 administration group, M14 administration group, M15 administration group, M16 administration group, M18 administration group, and M19 administration group suppressed renal damage, amyloidosis, and hyperviscosity syndrome.

### < Example 22: Inhibitory effect of each compound on immunoglobulin free λ light chain secretion in KMS-28BM cells >

The inhibitory effect of each compound on immunoglobulin free λ light chain secretion was examined by enzyme-linked immunosorbent assay (ELISA) using KMS-28BM cells. The each compound of the present invention inhibited immunoglobulin free λ light chain secretion in KMS-28BM cells.

As a control, compounds untreated KMS-28BM cells were seeded in 75 cm² flasks and incubated for 10 days at 37 °C and 5% CO₂. KMS-28BM cells were seeded into 75 cm² flasks and pre-cultured for 4 h. After incubation, KMS-28BM cells were treated with 0.05 µM M7, 0.05 µM M9, 0.1 µM yk-7, 0.1 µM yk-8-3, 50 µM yk-8-1, 0.5 µM M11, 0.05 µM M12, 0.005 µM M14, 0.005 µM M15, 0.005 µM M16, 0.5 µM M18, and 0.5 µM M19 at final concentrations, and incubated for 10 days at 37 °C and 5% CO₂. After 10 days of incubation, the culture supernatant was collected and immunoglobulin free λ light chain secretion was measured by ELISA. This measurement was performed using a human anti-λ chain antibody ELISA kit (Funakoshi).

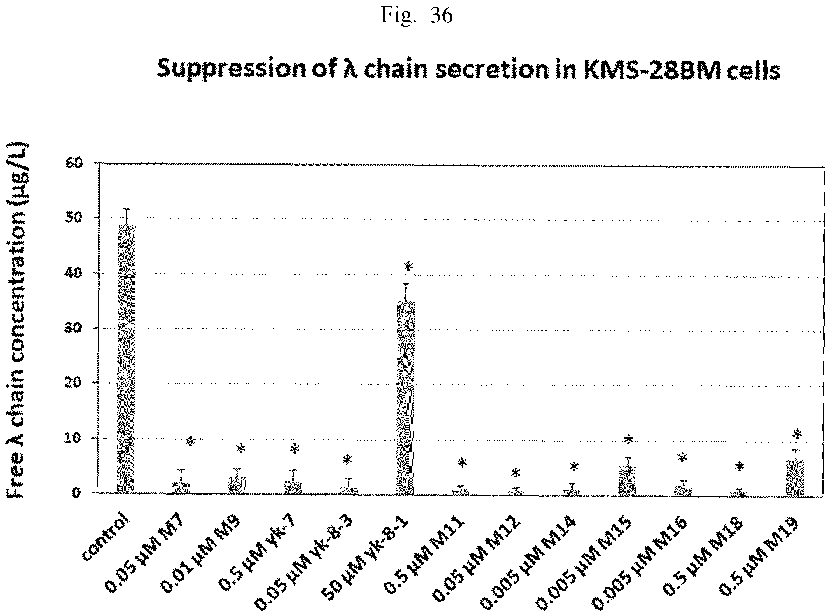

The results are shown in Figure 36. The horizontal axis represents the sample group and the vertical axis represents λ chain secretion (µg/mL). In control, λ chain is 48 µg/L, whereas in the M7 administration group, M9 administration group, yk-7 administration group, yk-8-3 administration group, yk-8-1 administration group, M11 administration group, M12 administration group, M14 administration group, M15 administration group, M16 administration group, M18 administration group, and M19 administration group, 2.1 µg/L, 3 µg/L, 2.3 µg/L, 1.3 µg/L, 35 µg/L, 1.1 µg/L, 0.6 µg/L, 1 µg/L, 5.4 µg/L, 1.8µg/L, 0.9 µg/L, and 6.6 µg/L, respectively.

As described above, M7 administration group, M9 administration group, yk-7 administration group, yk-8-3 administration group, yk-8-1 administration group, M11 administration group, M12 administration group, M14 administration group, M15 administration group, M16 administration group, M18 administration group, and M19 administration group significantly suppressed λ chain secretion. In other words, the control group caused renal damage, amyloidosis, and hyperviscosity syndrome due to λ chain production, while the M7 administration group, M9 administration group, yk-7 administration group, yk-8-3 administration group, yk-8-1 administration group, M11 administration group, M12 administration group, M14 administration group, M15 administration group, M16 administration group, M18 administration group, and M19 administration group suppressed renal damage, amyloidosis, and hyperviscosity syndrome.

### < Example 23: Inhibitory effect of each compound on IL-6 secretion in KMS-28BM cells >

The inhibitory effect of each compound on IL-6 secretion was examined using Luminex in KMS-28BM cells. The each compound of the present invention inhibited IL-6 secretion in KMS-28BM cells.

As a control, compounds untreated KMS-28BM cells were seeded in 75 cm² flasks and incubated for 10 days at 37 °C and 5% CO₂. KMS-28BM cells were seeded into 75 cm² flasks and pre-cultured for 4 h. After incubation, KMS-28BM cells were treated with 0.05 µM M7, 0.05 µM M9, 0.1 µM yk-7, 0.1 µM yk-8-3, 50 µM yk-8-1, 0.5 µM M11, 0.05 µM M12, 0.005 µM M14, 0.005 µM M15, 0.005 µM M16, 0.5 µM M18, and 0.5 µM M19 at final concentrations, and incubated for 10 days at 37 °C and 5% CO₂. After 10 days of incubation, the culture supernatant was collected and the secretion of IL-6, a bone destruction-related factor and a growth-promoting factor, was measured by Luminex. The human Magnetic Luminex Assay (R&D) was used for this measurement.

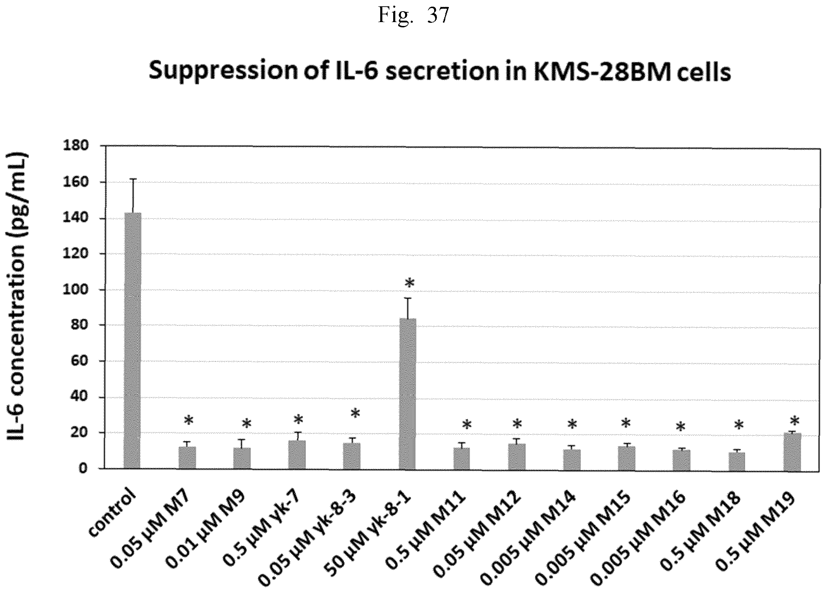

The results are shown in Figure 37. The horizontal axis represents the sample group and the vertical axis represents IL-6 secretion (pg/mL). In control, IL-6 is 143 pg/mL, whereas in the M7 administration group, M9 administration group, yk-7 administration group, yk-8-3 administration group, yk-8-1 administration group, M11 administration group, M12 administration group, M14 administration group, M15 administration group, M16 administration group, M18 administration group, and M19 administration group, 12 pg/mL, 11 pg/mL, 16 pg/mL, 14 pg/mL, 84 pg/mL, 12 pg/mL, 14 pg/mL, 12 pg/mL, 14 pg/mL, 12 pg/mL, 11 pg/mL, and 22 pg/mL, respectively.

As described above, M7 administration group, M9 administration group, yk-7 administration group, yk-8-3 administration group, yk-8-1 administration group, M11 administration group, M12 administration group, M14 administration group, M15 administration group, M16 administration group, M18 administration group, and M19 administration group significantly suppressed IL-6 secretion. In other words, the control group caused bone lesions (bone destruction), hypercalcemia associated with bone lesions, pathological fractures, spinal cord compression fractures, spinal cord compression symptoms associated with spinal cord compression fractures, and neurological symptoms associated with spinal cord compression symptoms due to IL-6 production, while the M7 administration group, M9 administration group, yk-7 administration group, yk-8-3 administration group, yk-8-1 administration group, M11 administration group, M12 administration group, M14 administration group, M15 administration group, M16 administration group, M18 administration group, and M19 administration group suppressed bone lesions (bone destruction), hypercalcemia associated with bone lesions, pathological fractures, spinal cord compression fractures, spinal cord compression symptoms associated with spinal cord compression fractures, and neurological symptoms associated with spinal cord compression symptoms. Furthermore, it can be said that the control group enhanced multiple myeloma cell growth due to autocrine associated with IL-6 production, while the M7 administration group, M9 administration group, yk-7 administration group, yk-8-3 administration group, yk-8-1 administration group, M11 administration group, M12 administration group, M14 administration group, M15 administration group, M16 administration group, M18 administration group, and M19 administration group inhibited multiple myeloma cell growth.

### < Example 24: Inhibitory effect of each compound on IgG secretion in L363 cells >

The inhibitory effect of each compound on IgG secretion was examined by enzyme-linked immunosorbent assay (ELISA) using L363 cells. The each compound of the present invention inhibited IgG secretion in L363 cells.

As a control, compounds untreated L363 cells were seeded in 75 cm² flasks and incubated for 10 days at 37 °C and 5% CO₂. L363 cells were seeded into 75 cm² flasks and pre-cultured for 4 h. After incubation, L363 cells were treated with 0.1 µM M7, 0.05 µM M9, 0.1 µM yk-7, 0.1 µM yk-8-3, 50 µM yk-8-1, 0.1 µM M11, 0.01 µM M12, 0.005 µM M14, 0.005 µM M15, 0.005 µM M16, 1 µM M18, and 1 µM M19 at final concentrations, and incubated for 10 days at 37 °C and 5% CO₂. After 10 days of incubation, the culture supernatant was collected and IgG secretion was measured by ELISA. This measurement was performed using a human anti-IgG antibody ELISA kit (Funakoshi).

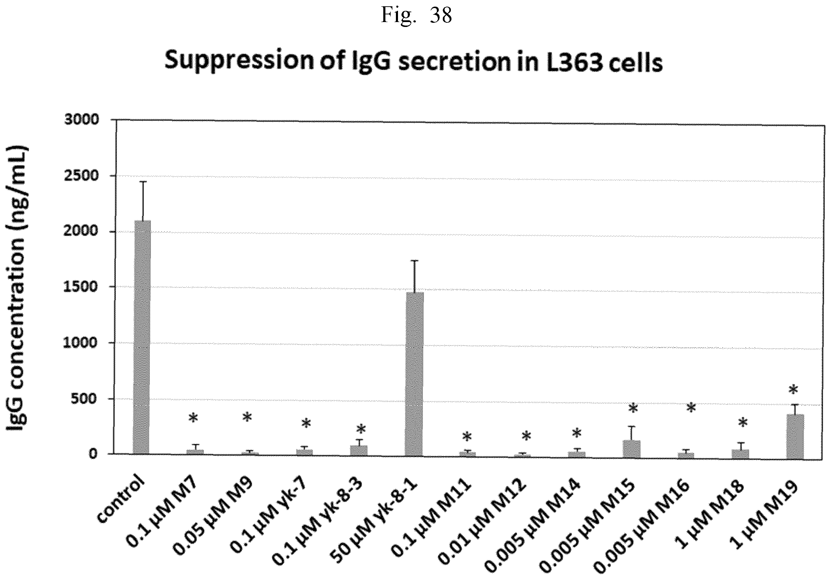

The results are shown in Figure 38. The horizontal axis represents the sample group and the vertical axis represents IgG secretion (ng/mL). In control, IgG is 2098 ng/mL, whereas in the M7 administration group, M9 administration group, yk-7 administration group, yk-8-3 administration group, yk-8-1 administration group, M11 administration group, M12 administration group, M14 administration group, M15 administration group, M16 administration group, M18 administration group, and M19 administration group, 50 ng/mL, 27 ng/mL, 61 ng/mL, 95 ng/mL, 1474 ng/mL, 44 ng/mL, 33 ng/mL, 61 ng/mL, 169 ng/mL, 56 ng/mL, 84 ng/mL, and 413ng/mL, respectively.

As described above, M7 administration group, M9 administration group, yk-7 administration group, yk-8-3 administration group, yk-8-1 administration group, M11 administration group, M12 administration group, M14 administration group, M15 administration group, M16 administration group, M18 administration group, and M19 administration group significantly suppressed IgG secretion. In other words, the control group caused renal damage, amyloidosis, and hyperviscosity syndrome due to IgG production, while the M7 administration group, M9 administration group, yk-7 administration group, yk-8-3 administration group, yk-8-1 administration group, M11 administration group, M12 administration group, M14 administration group, M15 administration group, M16 administration group, M18 administration group, and M19 administration group suppressed renal damage, amyloidosis, and hyperviscosity syndrome.

### < Example 25: Inhibitory effect of each compound on immunoglobulin free λ light chain secretion in L363 cells >

The inhibitory effect of each compound on immunoglobulin free λ light chain secretion was examined by enzyme-linked immunosorbent assay (ELISA) using L363 cells. The each compound of the present invention inhibited immunoglobulin free λ light chain secretion in L363 cells.

As a control, compounds untreated L363 cells were seeded in 75 cm² flasks and incubated for 10 days at 37 °C and 5% CO₂. L363 cells were seeded into 75 cm² flasks and pre-cultured for 4 h. After incubation, L363 cells were treated with 0.1 µM M7, 0.05 µM M9, 0.1 µM yk-7, 0.1 µM yk-8-3, 50 µM yk-8-1, 0.1 µM M11, 0.01 µM M12, 0.005 µM M14, 0.005 µM M15, 0.005 µM M16, 1 µM M18, and 1 µM M19 at final concentrations, and incubated for 10 days at 37 °C and 5% CO₂. After 10 days of incubation, the culture supernatant was collected and immunoglobulin free λ light chain secretion was measured by ELISA. This measurement was performed using a human anti-λ chain antibody ELISA kit (Funakoshi).

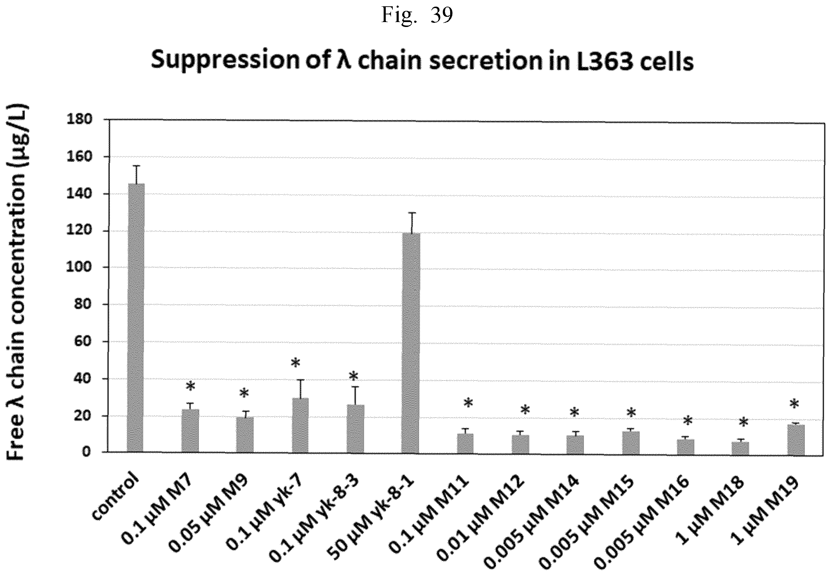

The results are shown in Figure 39. The horizontal axis represents the sample group and the vertical axis represents λ chain secretion (µg/mL). In control, λ chain is 145 µg/L, whereas in the M7 administration group, M9 administration group, yk-7 administration group, yk-8-3 administration group, yk-8-1 administration group, M11 administration group, M12 administration group, M14 administration group, M15 administration group, M16 administration group, M18 administration group, and M19 administration group, 23 µg/L, 19 µg/L, 30 µg/L, 26 µg/L, 119 µg/L, 11 µg/L, 10 µg/L, 10 µg/L, 13 µg/L, 9 µg/L, 8 µg/L, and 17 µg/L, respectively.

As described above, M7 administration group, M9 administration group, yk-7 administration group, yk-8-3 administration group, yk-8-1 administration group, M11 administration group, M12 administration group, M14 administration group, M15 administration group, M16 administration group, M18 administration group, and M19 administration group significantly suppressed λ chain secretion. In other words, the control group caused renal damage, amyloidosis, and hyperviscosity syndrome due to λ chain production, while the M7 administration group, M9 administration group, yk-7 administration group, yk-8-3 administration group, yk-8-1 administration group, M11 administration group, M12 administration group, M14 administration group, M15 administration group, M16 administration group, M18 administration group, and M19 administration group suppressed renal damage, amyloidosis, and hyperviscosity syndrome.

### < Example 26: Inhibitory effect of each compound on MIP-la secretion in L363 cells >

The inhibitory effect of each compound on MIP-la secretion was examined using Luminex in L363 cells. The each compound of the present invention inhibited MIP-la secretion in L363 cells.

As a control, compounds untreated L363 cells were seeded in 75 cm² flasks and incubated for 10 days at 37 °C and 5% CO₂. L363 cells were seeded into 75 cm² flasks and pre-cultured for 4 h. After incubation, L363 cells were treated with 0.1 µM M7, 0.05 µM M9, 0.1 µM yk-7, 0.1 µM yk-8-3, 50 µM yk-8-1, 0.1 µM M11, 0.01 µM M12, 0.005 µM M14, 0.005 µM M15, 0.005 µM M16, 1 µM M18, and 1 µM M19 at final concentrations, and incubated for 10 days at 37 °C and 5% CO₂. After 10 days of incubation, the culture supernatant was collected and the secretion of MIP-la, a bone destruction-related factor and a growth-promoting factor, was measured by Luminex. The human Magnetic Luminex Assay (R&D) was used for this measurement.

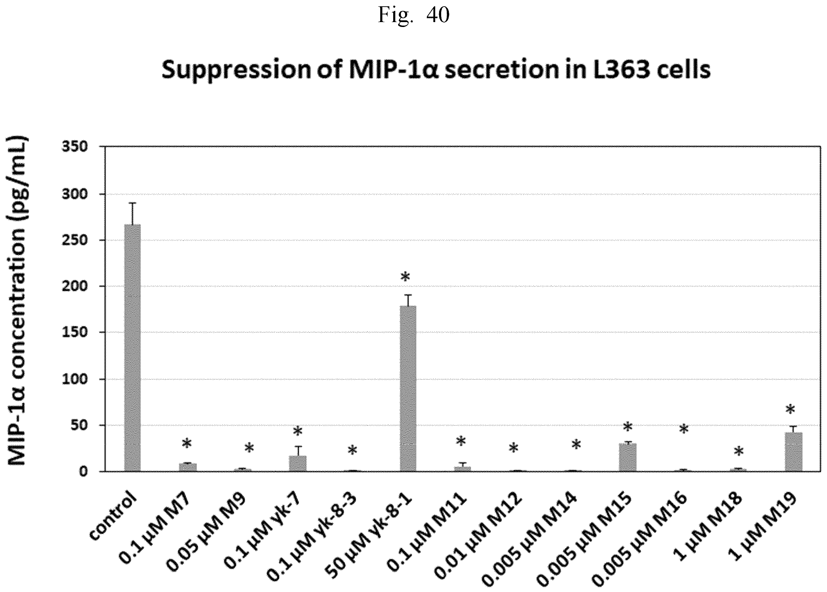

The results are shown in Figure 40. The horizontal axis represents the sample group and the vertical axis represents MIP-la secretion (pg/mL). In control, IL-6 is 266 pg/mL, whereas in the M7 administration group, M9 administration group, yk-7 administration group, yk-8-3 administration group, yk-8-1 administration group, M11 administration group, M12 administration group, M14 administration group, M15 administration group, M16 administration group, M18 administration group, and M19 administration group, 9 pg/mL, 3 pg/mL, 17 pg/mL, 2 pg/mL, 179 pg/mL, 6 pg/mL, 2 pg/mL, 2 pg/mL, 30 pg/mL, 2 pg/mL, 3 pg/mL, and 42 pg/mL, respectively.

As described above, M7 administration group, M9 administration group, yk-7 administration group, yk-8-3 administration group, yk-8-1 administration group, M11 administration group, M12 administration group, M14 administration group, M15 administration group, M16 administration group, M18 administration group, and M19 administration group significantly suppressed MIP-la secretion. In other words, the control group caused bone lesions (bone destruction), hypercalcemia associated with bone lesions, pathological fractures, spinal cord compression fractures, spinal cord compression symptoms associated with spinal cord compression fractures, and neurological symptoms associated with spinal cord compression symptoms due to MIP-1α production, while the M7 administration group, M9 administration group, yk-7 administration group, yk-8-3 administration group, yk-8-1 administration group, M11 administration group, M12 administration group, M14 administration group, M15 administration group, M16 administration group, M18 administration group, and M19 administration group suppressed bone lesions (bone destruction), hypercalcemia associated with bone lesions, pathological fractures, spinal cord compression fractures, spinal cord compression symptoms associated with spinal cord compression fractures, and neurological symptoms associated with spinal cord compression symptoms. Furthermore, it can be said that the control group enhanced multiple myeloma cell growth due to autocrine associated with MIP-la production, while the M7 administration group, M9 administration group, yk-7 administration group, yk-8-3 administration group, yk-8-1 administration group, M11 administration group, M12 administration group, M14 administration group, M15 administration group, M16 administration group, M18 administration group, and M19 administration group inhibited multiple myeloma cell growth.

### < Example 27: Inhibition of tumor growth by M9 administration in vivo in L363 cells >

L363 cells were transplanted into NOD/ShiJic-scidJcl mice, and the tumor growth inhibitory effect of oral administration of M9 was examined. As a result, a remarkable tumor growth inhibitory effect was observed.

L363 cells were transplanted into NOD/ShiJic-scidJcl mice, and M9 was orally administered to the mice at 100 mg/kg daily on day 0 when the average tumor volume exceeded 100 mm³, and tumor volume was measured on day 24.

The group that was only implanted with L363 cells is called the target group and the group that was treated with M9 is called the M9-treated group. Each group consisted of 5 animals.

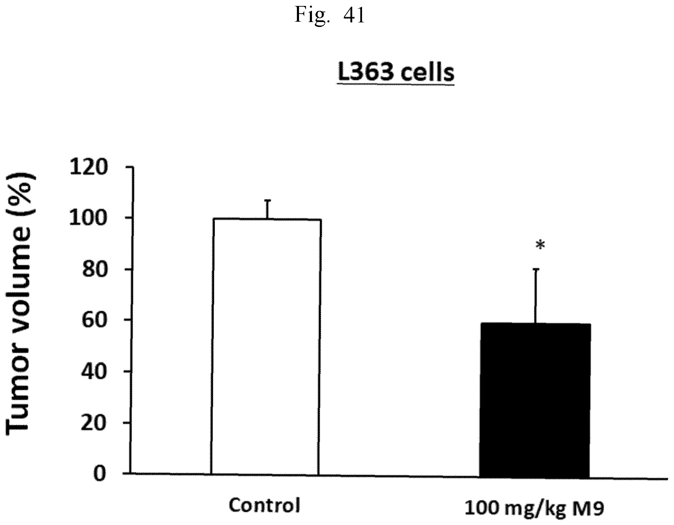

The results are shown in Figure 41. Referring to the figure, the horizontal axis indicates drug treatment, and the vertical axis indicates tumor volume (percent of the control group). White bars indicate the control group (labeled "Control"). Black bars indicate the M9 treatment group (labeled "100 mg/kg M9"). Those with significant differences (P<0.01) from the control group are marked with an asterisk (*).

Compared to the control group (white bars), the M9 group (black bars) showed significant inhibition of tumor growth. In other words, the M9 group significantly suppressed tumor growth more than the control group.

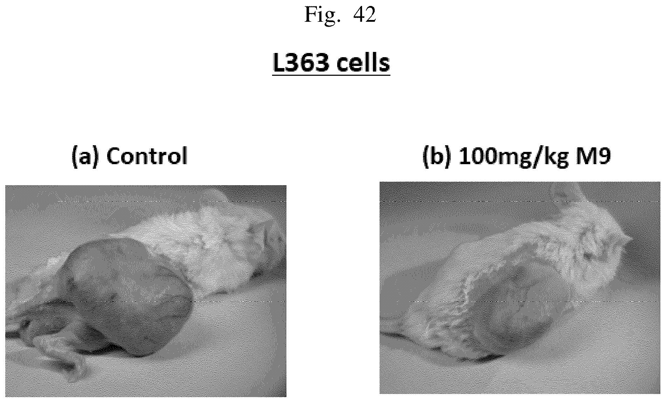

Figure 42 shows a photograph of a tumor on day 24 of dosing. Figure 42(a) shows the target group and Figure 42(b) shows a photograph of the tumor of one of the mice in the M9-treated group. In the control group in Figure 42(a), there is a marked increase in tumor size. On the other hand, in the M9-treated group in Figure 42(b), a significant decrease in volume was observed compared to the control group.

As described above, the M9-treated group was found to markedly inhibit tumor growth.

### < Example 28: Inhibition of tumor growth by M9 and M14 administration in vivo in Raji cells >

Raji cells were transplanted into NOD/ShiJic-scidJcl mice, and the tumor growth inhibitory effect of oral administration of M9 and M14 was examined. As a result, a remarkable tumor growth inhibitory effect was observed.

Raji cells were transplanted into NOD/ShiJic-scidJcl mice, and the mice were orally administered M9 at 50 mg/kg and M14 at 50 mg/kg daily on day 0 when the average tumor volume exceeded 100 mm³, and the tumor volume was measured on day 21.

The group that was only transplanted with Raji cells is called the control group, the group that was treated with M9 is called the M9-treated group, and the group that was treated with M14 is called the M14-treated group. Each group consisted of 5 animals.

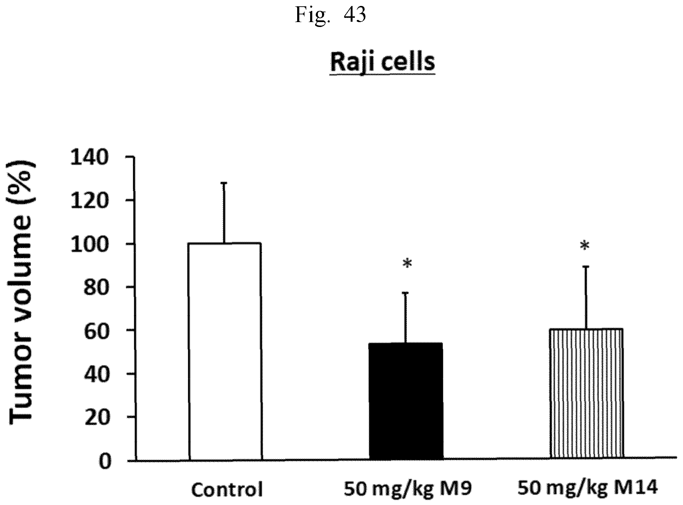

The results are shown in Figure 43. Referring to the figure, the horizontal axis indicates drug treatment, and the vertical axis indicates tumor volume (percent of the control group). White bars indicate the control group (labeled "Control"). Black bars indicate the M9 treatment group (labeled "50 mg/kg M9") and vertical bars indicate the M14 treatment group (labeled "50 mg/kg M14"). Those that were significantly different (P<0.01) from the control group are indicated with "*".

Compared to the control group (white bars), the M9 (black bars) and M14 (vertical bars) groups showed significant inhibition of tumor growth. In other words, the M9 and M14 groups significantly suppressed tumor growth more than the control group.

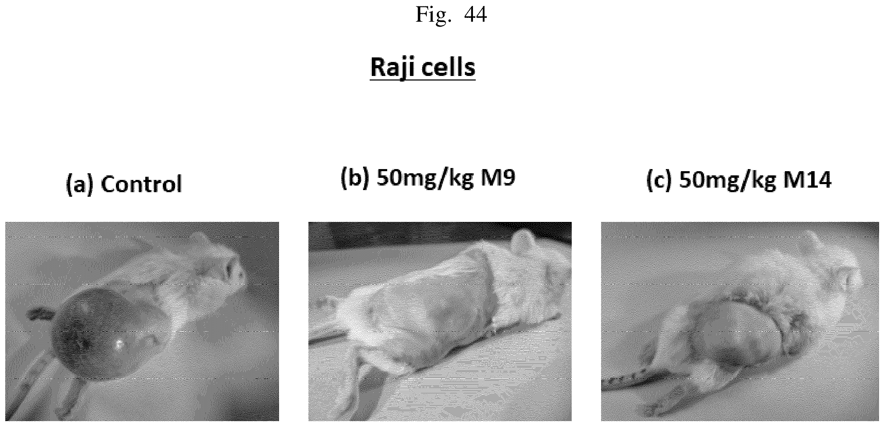

Figure 44 shows a photograph of a tumor on day 21 of dosing. Figure 44(a) shows a picture of a tumor of one mouse in the control group, Figure 44(b) in the M9-treated group, and Figure 44(c) in the M14-treated group. In the control group in Figure 44(a), there is a marked increase in tumor size. On the other hand, the M9-treated group in Figure 44(b) and the M14-treated group in Figure 44(c) show a marked decrease in volume compared to the control group. As described above, the M9- and M14-treated groups were found to significantly inhibit tumor growth.

### [Industrial applicability]

The compositions for amelioration of malignant tumor diseases of the present invention can provide pharmaceutical compositions or processed foods useful for multiple myeloma, lymphocytic leukemia, malignant lymphoma (MALT lymphoma, DLBCL, burkitt lymphoma, hodgkin lymphoma, adult T cell leukemia, peripheral T lymphoma, etc.), bortezomib-resistant multiple myeloma, rituximab-resistant malignant lymphoma CRAB (renal failure, amyloidosis, hyperviscosity syndrome, bone lesions (bone destruction), hypercalcemia associated with bone lesions, pathological fractures, spinal cord compression fractures, and hypercalcemia associated with bone lesions, (spinal cord compression symptoms associated with spinal cord compression fractures, neurological symptoms associated with spinal cord compression symptoms), B cell-like transformation in multiple myeloma with anti-CD20 monoclonal antibody available, and autocrine cell growth with IL-6 and MIP-la production in multiple myeloma.

## Claims

1. Compounds of any of formulas (1) to (12).

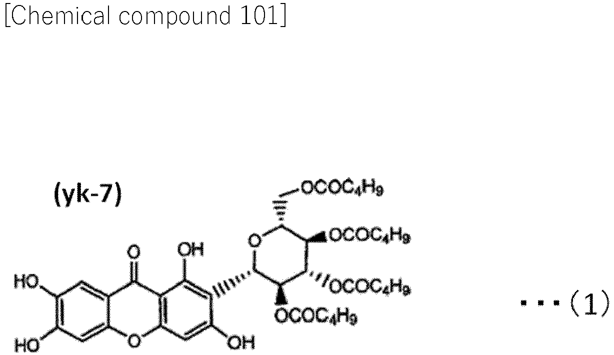

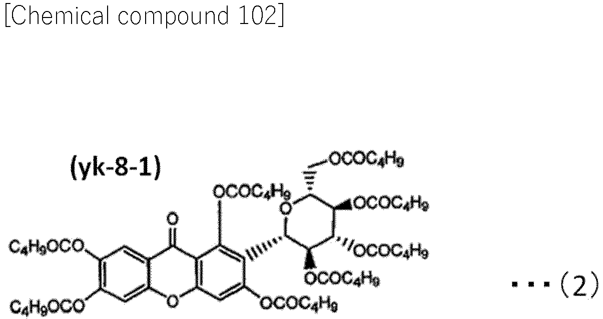

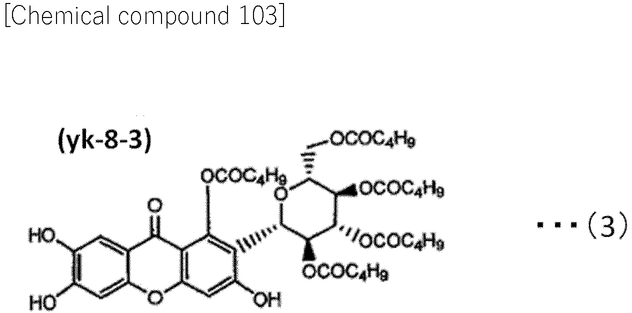

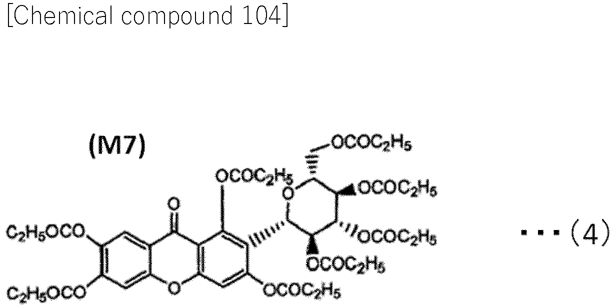

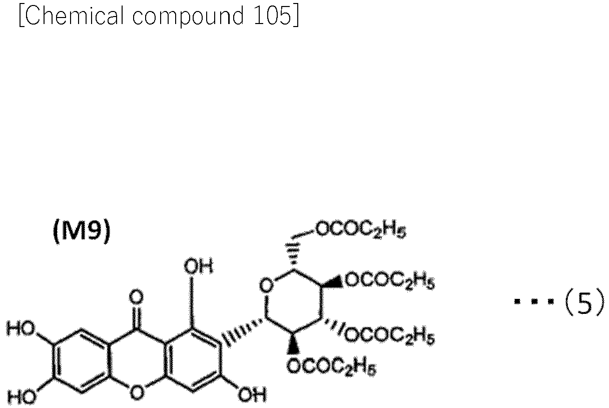

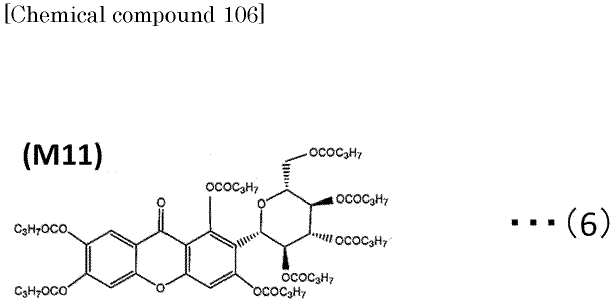

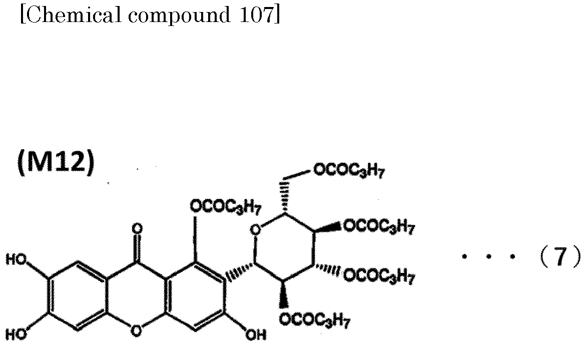

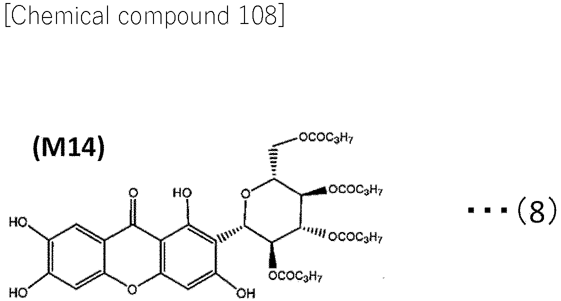

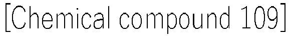

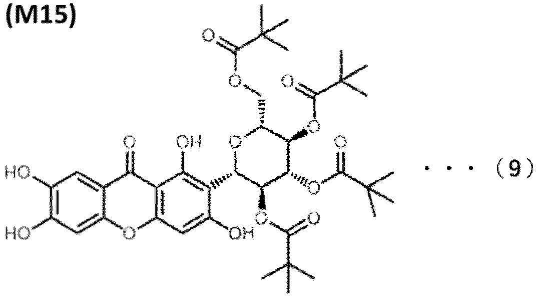

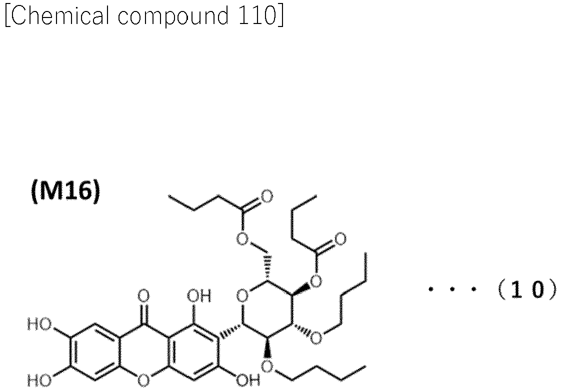

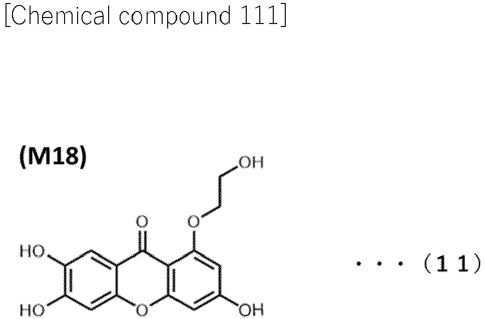

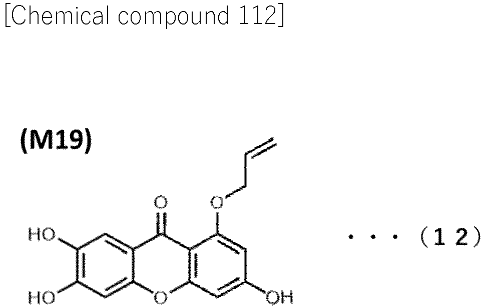

2. A pharmaceutical composition for ameliorating malignant tumor disease containing at least one compounds of formulas (1) to (12) as an effective ingredients as described in claim 1.

3. The pharmaceutical composition for ameliorating malignant tumor disease according to claim 2, **characterized in that** the malignant tumor disease is at least one of multiple myeloma, lymphoid leukemia, malignant lymphoma (MALT lymphoma, DLBCL, mantle cell lymphoma, burkitt lymphoma, hodgkin lymphoma, adult T cell leukemia, peripheral T lymphoma, etc.), pancreatic cancer, breast cancer, malignant melanoma, lung cancer, liver cancer, gastric cancer, colon cancer, head and neck tumor, glioma, renal cancer, ovarian cancer, and cervical cancer.

4. A pharmaceutical composition for ameliorating bortezomib-resistant multiple myeloma or rituximab-resistant malignant lymphoma, containing at least one compounds of formulas (1) to (12) as an effective ingredients as described in claim 1.

5. A pharmaceutical composition for improving CRAB, etc., an associated symptom of multiple myeloma, containing at least one compounds of formulas (1) to (12) described in claim 1 as an effective ingredients.

6. The pharmaceutical composition for improving CRAB, etc. according to claim 5, **characterized in that** the CRAB, etc. is at least one of hypercalcemia, renal disorder, anemia, bone lesions, amyloidosis, and hyperviscosity syndrome.

7. A pharmaceutical composition for ameliorating multiple myeloma, containing at least one compounds of formulas (1) to (12) as described in claim 1 and a CD20 monoclonal antibody drug as effective ingredients.

8. The pharmaceutical composition for ameliorating multiple myeloma according to claim 7, **characterized in that** the CD20 monoclonal antibody drug is at least one of rituximab, obinutuzumab, ofatumumab, and ibritumomab tiuxetan, etc.

9. A pharmaceutical composition for inducing transformation of multiple myeloma cells to B cell-like cells, containing at least one compounds of formulas (1) to (12) described in claim 1 as an effective ingredients.

10. A pharmaceutical composition for inhibiting cell proliferation by IL-6 and MIP-la autocrine containing at least one compounds of formulas (1) to (12) described in claim 1 as an effective ingredients.

11. A composition for inhibiting proliferation of malignant tumor cells containing at least one compounds of formulas (1) to (12) described in claim 1 as an effective ingredients.

12. The composition for growth inhibition according to claim 11, **characterized in that** the malignant tumor cells are at least one of multiple myeloma, lymphoid leukemia, malignant lymphoma (MALT lymphoma, DLBCL, mantle cell lymphoma, burkitt lymphoma, hodgkin lymphoma, adult T cell leukemia, peripheral T lymphoma, etc.), pancreatic cancer, breast cancer, malignant melanoma, lung cancer, liver cancer, gastric cancer, colon cancer, head and neck tumor, glioma, renal cancer, ovarian cancer, and cervical cancer.

13. A composition for growth inhibition of bortezomib-resistant multiple myeloma and rituximab-resistant malignant lymphoma containing at least one compounds of formulas (1) to (12) as an effective ingredients as described in claim 1.

14. A composition for inducing transformation of multiple myeloma cells to B cell-like cells, containing at least one compounds of formulas (1) to (12) described in claim 1 as an effective ingredients.

15. A composition for growth inhibition of cell proliferation by IL-6 and MIP-la autocrine in multiple myeloma containing at least one compounds of formulas (1) to (12) described in claim 1 as an effective ingredients.

16. Processed food product containing at least one compounds of formulas (1) to (12) as described in claim 1.

17. Processed food product containing at least one compounds of formulas (1) to (12) described in claim 1 as an effective ingredients and labeled as being for improving symptoms of malignant tumors such as multiple myeloma, lymphocytic leukemia, malignant lymphoma (MALT lymphoma, DLBCL, mantle cell lymphoma, burkitt lymphoma, hodgkin lymphoma, adult T-cell leukemia, peripheral T lymphoma, etc.), pancreatic cancer, breast cancer, malignant melanoma, lung cancer, liver cancer, gastric cancer, colon cancer, head and neck tumor, gliomas, renal cancer, ovarian cancer and uterine cancer, and bortezomib-resistant multiple myeloma, and rituximab-resistant malignant lymphoma.

18. Processed food product containing at least one compounds of formulas (1) to (12) described in claim 1 as an effective ingredients, and labeled to improve symptoms of CRAB, etc. that develop in association with multiple myeloma.

19. An agent containing at least one compounds of formulas (1) to (12) as described in claim 1.

20. An agent that contains at least one compounds of formulas (1) to (12) as an effective ingredients and that is labeled as being for symptomatic improvement of malignant tumors such as multiple myeloma, lymphocytic leukemia, malignant lymphoma (MALT lymphoma, DLBCL, mantle cell lymphoma, burkitt lymphoma, hodgkin lymphoma, adult T-cell leukemia, peripheral T lymphoma, etc.), pancreatic cancer, breast cancer, malignant melanoma, lung cancer, liver cancer, gastric cancer, colon cancer, head and neck tumor, gliomas, renal cancer, ovarian cancer and uterine cancer, and bortezomib-resistant multiple myeloma, and rituximab-resistant malignant lymphoma.

21. An agent that contains at least one compounds of formulas (1) to (12) as an effective ingredients and that is labeled for the improvement of symptoms of CRAB, etc. that develop in association with multiple myeloma.
